# EUROPEAN PATENT SPECIFICATION

(11) **EP 4 370 508 B1**
(45) Date of publication and mention of the grant of the patent: **10.09.2025**
(21) Application number: 22735923.9
(22) Date of filing: 04.07.2022
(51) Int. Cl.: C07D 249/08, C07D 401/12, A01N 43/653

(54) **TRIAZOLE COMPOUNDS FOR THE CONTROL OF INVERTEBRATE PESTS**
TRIAZOLVERBINDUNGEN ZUR BEKÄMPFUNG VON WIRBELLOSEN SCHÄDLINGEN
COMPOSÉS DE TRIAZOLE DESTINÉS À LA LUTTE CONTRE LES ORGANISMES NUISIBLES INVERTÉBRÉS

(30) Priority: 12.07.2021 EP 21185074; 12.07.2021 EP 21185079; 16.12.2021 EP 21215021
(43) Date of publication of application: 22.05.2024
(73) Proprietor: BASF SE, 67056 Ludwigshafen am Rhein (DE)
(72) Inventor: HUWYLER, Nikolas, 67056 Ludwigshafen (DE); KOERBER, Karsten, 67056 Ludwigshafen (DE); PEDRONI, Julia, 67056 Ludwigshafen (DE); GILBERG, Erik, 67056 Ludwigshafen (DE); POHLMAN, Matthias, 67056 Ludwigshafen (DE)
(74) Representative: BASF IP Association
(86) International application number: PCT/EP2022/068368
(87) International publication number: WO 2023/285175

(56) References cited:
- WO-A1-2020/053364
- WO-A2-2008/011557
- WO-A2-2020/053365

## Description

The invention relates to compounds of formula I 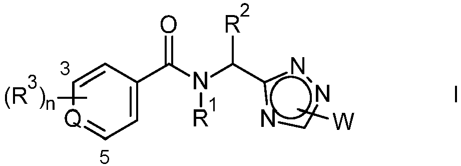 wherein
- R¹: is H, OH, C₁-C₆-alkyl, C₁-C₆-haloalkyl, C₃-C₆-cycloalkyl, C₃-C₆-halocycloalkyl, C₁-C₃-alkoxy, C₁-C₄-alkyl-C₃-C₆-cycloalkyl, C₁-C₄-alkyl-C₃-C₆-halocycloalkyl, which groups are unsubstituted, or partially or fully substituted with R¹¹;
or C(=N-R¹¹)R¹², G(O)R^{11a};
- R¹¹: is CN, NO₂, NR¹²R¹³, C(O)NH₂, C(S)NH₂, C(O)OH, OR¹⁴, OR¹⁵, Si(CH₃)₃; C₁-C₆-haloalkyl; C₂-C₆-alkenyl; C₂-C₆-haloalkenyl; C₂-C₆-alkynyl; C₂-C₆-haloalkynyl; C₃-C₄-cycloal kyl-C₁-C₂-alkyl, which ring is unsubstituted or substituted with 1 or 2 halogen; 3- to 6-membered heterocyclyl, 5- or 6-membered hetaryl, or phenyl, which rings are unsubstituted or substituted with halogen, C₁-C₃-haloalkyl, and/or CN;
R^{11a} is NR¹²R¹³, C(O)NH₂, C(S)NH₂, C(O)OH, OR¹⁴, OR¹⁵, Si(CH₃)₃; C₁-C₆-haloalkyl; C₂-C₆-alkenyl; C₂-C₆-haloalkenyl; C₂-C₆-alkynyl; C₂-C₆-haloalkynyl; C₃-C₄-cycloalkyl-C₁-C₂-alkyl, which ring is unsubstituted or substituted with 1 or 2 halogen; 3- to 6-membered heterocyclyl, which rings are unsubstituted or substituted with halogen, C₁-C₃-haloalkyl, and/or CN;
- R¹², R¹³: are independently from each other H, C₁-C₄-alkyl, C₁-C₄-haloalkyl, C₃-C₆-cycloalkyl, C(O)-C₁-C₄-alkyl, C(O)-C₁-C₄-haloalkyl, C(O)-C₃-C₄-cycloalkyl, C(O)-C₃-C₄-halocycloalkyl, S(O)ₘ-C₁-C₄-alkyl, S(O)ₘ-C₁-C₄-haloalyl, S(O)ₘ-C₃-C₄-cycloalkyl, S(O)ₘ₋C₃-C₄-halocycloalkyl; or
- R¹² and R¹³,: together with the nitrogen atom to which they are bound, form a 3-, 4-, 5-, 6- or 7-membered saturated, partially unsaturated or fully unsaturated heterocycle, which heterocycle may additionally contain 1 or 2 heteroatoms or heteroatom groups selected from N, O, and S(O)ₘ as ring members, which heterocycle is unsubstituted or substituted with one or more substituents selected from halogen, C₁-C₄-alkyl, C₁-C₄-haloalkyl, C₁-C₄-alkoxy, C₁-C₄-haloalkoxy, and oxo;
- m: is 0, 1, or 2;
- R¹⁴: is H, C₁-C₄-alkyl, C₁-C₄-haloalkyl, C₃-C₆-cycloalkyl, C₃-C₆-halo-cyclo-alkyl, C₃-C₄-cycloalkyl-C₁-C₂-alkyl, C₃-C₄-halocycloalkyl-C₁-C₂-alkyl, C(O)-C₁-C₄-alkyl, C(O)-C₁-C₄-haloalkyl, C(O)-C₃-C₄-cycloalkyl, C(O)-C₃-C₄-halo¬cyclo¬alkyl, NR¹²R¹³, or 5-, or 6-membered hetaryl or phenyl, which rings are unsubstituted or partially or fully substituted with R^{3a};
- R²: is H, CN, C₁₋C₃-alkyl, C₁-C₃-haloalkyl, C₂-C₃-alkynyl;
- Q: is CH, CR³, or N;
- R³: is halogen, CN, NO₂, C₁-C₄-alkyl, C₃-C₆-cycloalkyl, C₁-C₃-haloalkyl, C₁-C₆-halocycloalkyl, C₁-C₄-alkoxy-C₁-C₄-alkyl, NR¹²R¹³, OR¹⁴, S(O)ₘ-R¹⁴, phenyl, or 3-, 4-, 5-, or 6-membered saturated, or partially or fully unsaturated heterocycle, which heterocycle contains 1, 2, 3, or 4 heteroatoms selected from N, O, and S(O)ₘ as ring members; wherein R³ rings are bonded directly, or via C₁-C₂-alkylene, O, or S(O)ₘ spacer, and are unsubstituted or substituted with R^{3a};
R^{3a} halogen, CN, NO₂, C₁-C₄-alkyl, C₁-C₄-haloalkyl, C₁-C₄-alkoxy-C₁-C₄-alkyl, C₁-C₄-haloalkoxy, C₃-C₄-cycloalkyl, C₃-C₄-halocycloalkyl, S(O)ₘ₋C₁-C₄-alkyl, S(O)ₘ-C₁-C₄-haloalkyl, S(O)ₘ-C₃-C₄-cycloalkyl, S(O)ₘ-C₃-C₄-halocycloalkyl; two R^{3a} on the same carbon atom together may form a group oxo;
- two R³: on the same carbon atom together may form a group oxo;
- two R³: on two adjacent carbon atoms form together with the carbon atoms they are bonded to a 5-, 6-, or 7-membered saturated, partially unsaturated or fully unsaturated ring, wherein the ring may contain 1, 2, or 3 heteroatoms or heteroatom groups selected from N, O, and S(O)ₘ as ring members, and wherein the ring is optionally substituted with one or more groups halogen, CN, C₁-C₄-alkyl, C₁-C₄-haloalkyl, C₁-C₄-alkoxy, and/or C₁-C₄-haloalkoxy;
- n: is 0, 1, 2, or 3;
- W: is C(=X)R⁴, being bonded to a nitrogen atom of the triazole ring;
X is O, or NR⁵;
R⁴ is H, OR¹⁴, CN, C₁-C₄-alkyl, C₃-C₆-cycloalkyl, C₁-C₃-haloalkyl, C₁-C₆-halocycloalkyl, C₂-C₄-alkenyl, C₂-C₄-halo¬alkenyl, C₂-C₄-alkynyl, C₁-C₄-alkoxy, C₁-C₄-haloalkoxy, S(O)ₘ-C₁-C₄-alkyl, S(O)ₘ-C₁-C₄-haloalkyl, S(O)ₘ-C₃-C₄-cyclo¬alkyl, S(O)ₘ-C₃-C₄-halocyclo¬alkyl, NR¹²R¹³, C(O)NR¹²R¹³, C(O)OR¹⁴, 3- to 6-membered heterocyclyl, 5- or 6-membered hetaryl, or phenyl, which rings are unsubstituted or substituted with R^{3a};
R⁵ is H, OR¹⁴, OR¹⁵, NR¹²R¹³, or C₁-C₆-alkyl which is unsubstituted, or partially or fully substituted with R¹¹;
R¹⁵ is H, C₁-C₄-alkyl, or C₁-C₄-haloalkyl, C₃-C₆-cycloalkyl, C₁-C₃-halocycloalkyl, which carbon chains are unsubstituted or partially or fully substituted with R¹¹; or 3- to 6-membered heterocyclyl, 5- or 6-membered hetaryl, or phenyl, which rings are unsubstituted or substituted with R^{3a};
and the N-oxides, stereoisomers and agriculturally or veterinarily acceptable salts thereof.

The invention also provides agricultural compositions comprising at least one compound of formula I, a stereoisomer thereof and/or an agriculturally acceptable salt thereof and at least one liquid and/or solid carrier, especially at least one inert liquid and/or solid agriculturally acceptable carrier.

The invention also provides a veterinary composition comprising at least one compound of formula I, a stereoisomer thereof and/or a veterinarily acceptable salt thereof and at least one liquid and/or solid carrier, especially at least one inert veterinarily liquid and/or solid acceptable carrier.

The invention also relates to plant propagation material, in particular seed, comprising at least one compound of formula I and/or an agriculturally acceptable salt thereof.

The invention further relates to a compound of formula I or a veterinarily acceptable salt thereof for use in treating or protecting an animal from infestation or infection by parasites, the use comprising bringing the animal in contact with a parasiticidally effective amount of a compound of formula I or a veterinarily acceptable salt thereof. Bringing the animal in contact with the compound I, its salt or the veterinary composition of the invention means applying or administering it to the animal.

WO 2017/192385, WO 2019/197468, WO 2019/202077, WO 2019/201835, WO 2019/206799, WO 2020/002563, WO 2020/053364, WO 2020/053365, WO 2020/070049, and WO 2020/079198 describe structurally closely related active compounds. These compounds are mentioned to be useful for combating invertebrate pests.

Nevertheless, there remains a need for highly effective and versatile agents for combating invertebrate pests. It is therefore an object of the invention to provide compounds having a good pesticidal activity and showing a broad activity spectrum against a large number of different invertebrate pests, especially against difficult to control pests, such as insects.

It has been found that these objects can be achieved by compounds of formula I as depicted and defined below, and by their stereoisomers, salts, tautomers and N-oxides, in particular their agriculturally acceptable salts.

Compounds I can be obtained by reaction of a compound II with 1 to 1.5 equivalents of a suitable electrophile III in the presence of 1 to 2 equivalents of a base, such as Na₂CO₃, K₂CO₃, Cs₂CO₃, NaHCO₃, KHCO₃, NaH, KH, triethylamine, diisopropylethylamine, pyridine, 2,6-lutidine, 1,8-diazabicylo[5.4.0]undec-7-ene (DBU), 1,5-diazabicyclo[4.3.0]non-5-ene (DBN), or 1,4-diazabicyclo[2.2.2]octane (DABCO), or a salt, such as CsF, in a solvent such as dimethylformamide (DMF), ethyl acetate (EtOAc), tetrahydrofuran (THF), 1,4-dioxane, methanol, ethanol, 2-propanol, dichloromethane (DCM), dichloroethane (DCE), chloroform, benzene, toluene, xylenes, or mesitylene, at temperatures from 0°C to 200°C, preferably from 25°C to the boiling point of the solvent, as known from literature (cf. Chernikova et al, Russian Journal of Organic Chemistry 2019, 55, 325-329; Wang et al, Journal of Medicinal Chemistry 2011, 54, 8541-8554; Plenkiewicz et al, Bulletin des Societes Chimiques Belges 1987, 96, 675-709; Korff et al, Journal of Medicinal Chemistry 2020, 63, 13159-13186; J. R. Shroff et al, Journal of Medicinal Chemistry 1981, 24, 1521-1525).

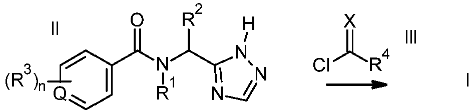

Further, compounds I, in which X is NOR¹⁵, can be obtained by alkylation of the corresponding compounds I, in which X is NOH, with alkylating reagents such as alkyl halides, alkyl tosylates, or alkyl mesylates, following procedures known from literature (cf. Kurbanli et al, Synthetic Communications 2004, 34, 1663-1675; Khomutov et al, Amino Acids 2010, 38, 509-517; Abele et al, Synthetic Communications 1998, 28, 2621-2633; Kocak et al, Synthetic Communications 2007, 37, 1155-1165; Faisal et al, Synthetic Communications 2010, 40, 3101-3108).

Compounds III with X being NOH or NOR¹⁵ can be obtained by reaction of the corresponding aldoxime(ether)s with 1 to 1.5 equivalents of a chlorinating agent such as N-chlorosuccinimide, optionally in the presence of 0.01 to 0.2 equivalents of a base such as pyridine, 2,6-lutidine, triethylamine, or diisopropylethylamine, or a chlorinating agent such as chlorine, optionally in the presence of 0.5 to 5 equivalents of hydrochloric acid, in a solvent such as DMF, ethyl acetate, chloroform, DCM, DCE, methanol, ethanol, ethylene glycol, 2-propanol, or water, at temperatures from -20°C to 100°C, preferably from 0°C to 60°C, as described in Jiang et al, Tetrahedron Letters 2016, 57, 712-714; Martsynkevich et al, Russian Chemical Bulletin, International Edition 2011, 60, 521-525, or G. Zinner, Chemische Berichte 1965, 98, 1353-1354.

Other compounds III can be obtained from the corresponding carboxylic acid (X=O) or secondary amide (X is NR⁵ with R⁵ other than O) following established literature procedures, e.g. Schaefer et al, Organic Syntheses 1929, 9, 32 and Detty et al, Journal of Organic Chemistry 1980, 45, 80-89, resp..

Alternatively, compounds II can be obtained from compounds IV by reaction with 1 to 1.5 equivalents of hydrazine hydrate in acetic acid (AcOH) as solvent, optionally using an alcohol as cosolvent such as methanol, ethanol, or 2-propanol, at temperatures from 25°C to 110°C as known from literature (cf. Lin et al, Journal of Organic Chemistry 1979, 44, 4160-4164; Wrobleski et al, Journal of Medicinal Chemistry 2019, 62, 8973-8995).

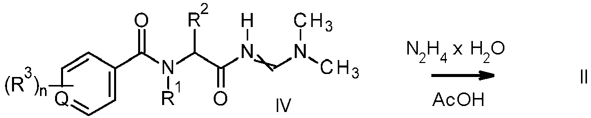

Compounds IV are known from WO 2020/094363 and can be obtained according to WO 2020/094363 from commercially available starting materials.

The reaction mixtures are worked up in a customary manner, for example by mixing with water, extracting with an appropriate organic solvent, separating the phases and, if appropriate, chromatographic purification of the crude products. Some of the intermediates and end products are obtained in the form of colourless or slightly brownish viscous oils which are purified or freed from volatile components under reduced pressure and at moderately elevated temperature. If the intermediates and end products are obtained as solids, purification can also be carried out by recrystallization or digestion.

If individual compounds I cannot be obtained by the routes described above, they can be prepared by derivatization of other compounds I.

However, if the synthesis yields mixtures of isomers, a separation is generally not necessarily required since in some cases the individual isomers can be interconverted during work-up for use or during application (for example under the action of light, acids or bases). Such conversions may also take place after use, for example in the treatment of plants in the treated plant, or in the pest to be controlled.

The organic moieties groups mentioned in the above definitions of the variables are - like the term halogen - collective terms for individual listings of the individual group members. The prefix Cₙ-Cₘ indicates in each case the possible number of carbon atoms in the group.

The term "partially or fully substituted" by a radical means that in general the group is substituted with same or different radicals.

The term "halogen" denotes in each case fluorine, bromine, chlorine, or iodine, in particular fluorine, chlorine, or bromine.

The term "alkyl" as used herein and in the alkyl moieties of alkylamino, alkylcarbonyl, alkylthio, alkylsulfinyl, alkylsulfonyl and alkoxyalkyl denotes in each case a straight-chain or branched alkyl group having usually from 1 to 10 carbon atoms, frequently from 1 to 6 carbon atoms, preferably 1 to 4 carbon atoms, more preferably from 1 to 3 carbon atoms. Examples of an alkyl group are methyl (Me), ethyl (Et), n-propyl (n-Pr), iso-propyl, n-butyl, 2-butyl, iso-butyl, tert-butyl, n-pentyl, 1-methylbutyl, 2-methylbutyl, 3-methylbutyl, 2,2-dimethylpropyl, 1-ethylpropyl, n-hexyl, 1,1-dimethylpropyl, 1,2-dimethylpropyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl, and 1-ethyl-2-methylpropyl.

The term "haloalkyl" as used herein and in the haloalkyl moieties of haloalkylcarbonyl, haloalkoxycarbonyl, haloalkylthio, haloalkylsulfonyl, haloalkylsulfinyl, haloalkoxy and haloalkoxyalkyl, denotes in each case a straight-chain or branched alkyl group having usually from 1 to 10 carbon atoms, frequently from 1 to 6 carbon atoms, preferably from 1 to 4 carbon atoms, wherein the hydrogen atoms of this group are partially or totally replaced with halogen atoms. Preferred haloalkyl moieties are selected from C₁-C₄-haloalkyl, more preferably from C₁-C₃-haloalkyl or C₁-C₂-haloalkyl, in particular from C₁-C₂-fluoroalkyl such as fluoromethyl, difluoromethyl, trifluoromethyl, 1-fluoroethyl, 2-fluoroethyl, 2,2-difluoroethyl, 2,2,2-trifluoroethyl, pentafluoroethyl, and the like.

The term "alkoxy" as used herein denotes in each case a straight-chain or branched alkyl group which is bonded via an oxygen atom and has usually from 1 to 10 carbon atoms, frequently from 1 to 6 carbon atoms, preferably 1 to 4 carbon atoms. Examples of an alkoxy group are methoxy, ethoxy, n-propoxy, iso-propoxy, n-butyloxy, 2-butyloxy, iso-butyloxy, tert.-butyloxy, and the like.

The term "alkoxyalkyl" as used herein refers to alkyl usually comprising 1 to 10, frequently 1 to 4, preferably 1 to 2 carbon atoms, wherein 1 carbon atom carries an alkoxy radical usually comprising 1 to 4, preferably 1 or 2 carbon atoms as defined above. Examples are CH₂OCH₃, CH₂-OC₂H₅, 2-(methoxy)ethyl, and 2-(ethoxy)ethyl.

The term "haloalkoxy" as used herein denotes in each case a straight-chain or branched alkoxy group having from 1 to 10 carbon atoms, frequently from 1 to 6 carbon atoms, preferably 1 to 4 carbon atoms, wherein the hydrogen atoms of this group are partially or totally replaced with halogen atoms, in particular fluorine atoms. Preferred haloalkoxy moieties include C₁-C₄-haloalkoxy, in particular C₁-C₂-fluoroalkoxy, such as fluoromethoxy, difluoromethoxy, trifluoro-methoxy, 1-fluoroethoxy, 2-fluoroethoxy, 2,2-difluoroethoxy, 2,2,2-trifluoroethoxy, 2-chloro-2-fluoroethoxy, 2-chloro-2,2-difluoro-ethoxy, 2,2dichloro-2-fluorethoxy, 2,2,2-trichloroethoxy, penta-fluoroethoxy and the like.

The term "alkylthio "(alkylsulfanyl: alkyl-S-)" as used herein refers to a straight-chain or branched saturated alkyl group having 1 to 10 carbon atoms, preferably 1 to 4 carbon atoms (= C₁-C₄-alkylthio), more preferably 1 to 3 carbon atoms, which is attached via a sulfur atom.

The term "haloalkylthio" as used herein refers to an alkylthio group as mentioned above wherein the hydrogen atoms are partially or fully substituted by fluorine, chlorine, bromine and/or iodine.

The term "alkylsulfinyl" (alkylsulfoxyl: C₁-C₃-alkyl-S(=O)-), as used herein refers to a straight-chain or branched saturated alkyl group (as mentioned above) having 1 to 10 carbon atoms, preferably 1 to 4 carbon atoms (= C₁-C₄-alkylsulfinyl), more preferably 1 to 3 carbon atoms bonded through the sulfur atom of the sulfinyl group at any position in the alkyl group.

The term "haloalkylsulfinyl" as used herein refers to an alkylsulfinyl group as mentioned above wherein the hydrogen atoms are partially or fully substituted by fluorine, chlorine, bromine and/or iodine.

The term "alkylsulfonyl" (alkyl-S(=O)₂-) as used herein refers to a straight-chain or branched saturated alkyl group having 1 to 10 carbon atoms, preferably 1 to 4 carbon atoms (= C₁-C₄-alkylsulfonyl), preferably 1 to 3 carbon atoms, which is bonded via the sulfur atom of the sulfonyl group at any position in the alkyl group.

The term "haloalkylsulfonyl" as used herein refers to an alkylsulfonyl group as mentioned above wherein the hydrogen atoms are partially or fully substituted by fluorine, chlorine, bromine and/or iodine.

The term "alkylcarbonyl" refers to an alkyl group as defined above, which is bonded via the carbon atom of a carbonyl group (C=O) to the remainder of the molecule.

The term "haloalkylcarbonyl" refers to an alkylcarbonyl group as mentioned above, wherein the hydrogen atoms are partially or fully substituted by fluorine, chlorine, bromine and/or iodine.

The term "alkoxycarbonyl" refers to an alkylcarbonyl group as defined above, which is bonded via an oxygen atom to the remainder of the molecule.

The term "haloalkoxycarbonyl" refers to an alkoxycarbonyl group as mentioned above, wherein the hydrogen atoms are partially or fully substituted by fluorine, chlorine, bromine and/or iodine.

The term "alkenyl" as used herein denotes in each case a singly unsaturated hydrocarbon radical having usually 2 to 10, frequently 2 to 6, preferably 2 to 4 carbon atoms, e.g. vinyl, allyl (2-propen-1-yl), 1-propen-1-yl, 2-propen-2-yl, methallyl (2-methylprop-2-en-1-yl), 2-buten-1-yl, 3-buten-1-yl, 2-penten-1-yl, 3-penten-1-yl, 4-penten-1-yl, 1-methylbut-2-en-1-yl, 2-ethylprop-2-en-1-yl and the like.

The term "haloalkenyl" as used herein refers to an alkenyl group as defined above, wherein the hydrogen atoms are partially or totally replaced with halogen atoms.

The term "alkynyl" as used herein denotes in each case a singly unsaturated hydrocarbon radical having usually 2 to 10, frequently 2 to 6, preferably 2 to 4 carbon atoms, e.g. ethynyl, propargyl (2-propyn-1-yl), 1-propyn-1-yl, 1-methylprop-2-yn-1-yl), 2-butyn-1-yl, 3-butyn-1-yl, 1-pentyn-1-yl, 3-pentyn-1-yl, 4-pentyn-1-yl, 1-methylbut-2-yn-1-yl, 1-ethylprop-2-yn-1-yl and the like.

The term "haloalkynyl" as used herein refers to an alkynyl group as defined above, wherein the hydrogen atoms are partially or totally replaced with halogen atoms.

The term "cycloalkyl" as used herein and in the cycloalkyl moieties of cycloalkoxy and cycloal-kylthio denotes in each case a monocyclic cycloaliphatic radical having usually from 3 to 10 or from 3 to 6 carbon atoms, such as cyclopropyl (cC₃H₅ or cC₃H₅), cyclobutyl (c-C₄H₇ or cC₄H₇), cyclopentyl (c-C₅H₉ or cC₅H₉), cyclohexyl (c-C₆H₁₁ or cC₆H₁₁), cycloheptyl, cyclooctyl, cyclononyl and cyclodecyl or cyclopropyl, cyclobutyl, cyclopentyl, and cyclohexyl.

The term "halocycloalkyl" as used herein and in the halocycloalkyl moieties of halocycloalkoxy and halocycloalkylthio denotes in each case a monocyclic cycloaliphatic radical having usually from 3 to 10 C atoms or 3 to 6 C atoms, wherein at least one, e.g. 1, 2, 3, 4 or 5 of the hydrogen atoms, are replaced by halogen, in particular by fluorine or chlorine. Examples are 1- and 2-fluorocyclopropyl, 1,2-, 2,2- and 2,3-difluorocyclopropyl, 1,2,2-trifluorocyclopropyl, 2,2,3,3-tetrafluorocyclpropyl, 1- and 2-chlorocyclopropyl, 1,2-, 2,2- and 2,3-dichlorocyclopropyl, 1,2,2-trichlorocyclopropyl, 2,2,3,3-tetrachlorocyclpropyl, 1-,2- and 3-fluorocyclopentyl, 1,2-, 2,2-, 2,3-, 3,3-, 3,4-, 2,5-difluorocyclopentyl, 1-,2- and 3-chlorocyclopentyl, 1,2-, 2,2-, 2,3-, 3,3-, 3,4-, 2,5-dichlorocyclopentyl and the like.

The term "halocycloalkenyl" as used herein and in the halocycloalkenyl moieties of halocycloalkenyloxy and halocycloalkenylthio denotes in each case a monocyclic singly unsaturated non-aromatic radical having usually from 3 to 10, e.g. 3 or 4 or from 5 to 10 carbon atoms, preferably from 3- to 8 carbon atoms, wherein at least one, e.g. 1, 2, 3, 4 or 5 of the hydrogen atoms, are replaced by halogen, in particular by fluorine or chlorine. Examples are 3,3-difluorocyclopropen-1-yl and 3,3-dichlorocyclopropen-1-yl.

The term "cycloalkenylalkyl" refers to a cycloalkenyl group as defined above which is bonded via an alkyl group, such as a C₁-C₃-alkyl group or a C₁-C₄-alkyl group, in particular a methyl group (= cycloalkenylmethyl), to the remainder of the molecule.

The term "carbocycle" or "carbocyclyl" includes in general a 3- to 12-membered, preferably a 3- to 8-membered or a 5- to 8-membered, more preferably a 5- or 6-membered mono-cyclic, non-aromatic ring comprising 3 to 12, preferably 3 to 8 or 5 to 8, more preferably 5 or 6 carbon atoms. Preferably, the term "carbocycle" covers cycloalkyl and cycloalkenyl groups as defined above.

The term "heterocycle" or "heterocyclyl" includes in general 3- to 12-membered, preferably 3-to 6-membered, in particular 6-membered monocyclic heterocyclic non-aromatic radicals. The heterocyclic non-aromatic radicals usually comprise 1, 2, 3, 4 or 5, preferably 1, 2 or 3 heteroatoms selected from N, O, and S as ring members, wherein S-atoms as ring members may be present as S, SO, or SO₂. Examples of 5- or 6-membered heterocyclic radicals comprise saturated or unsaturated, non-aromatic heterocyclic rings, such as oxiranyl, oxetanyl, thietanyl, thietanyl-S-oxide (S-oxothietanyl), thietanyl-S-dioxide (S-dioxothiethanyl), pyrrolidinyl, pyrrolinyl, pyrazolinyl, tetrahydrofuranyl, dihydrofuranyl, 1,3-dioxolanyl, thiolanyl, S-oxothiolanyl, S-dioxothiolanyl, dihydrothienyl, S-oxodihydrothienyl, S-dioxodihydrothienyl, oxazolidinyl, oxazolinyl, thiazolinyl, oxathiolanyl, piperidinyl, piperazinyl, pyranyl, dihydropyranyl, tetrahydropyranyl, 1,3- and 1,4-dioxanyl, thiopyranyl, S-oxothiopyranyl, S-dioxothiopyranyl, dihydrothiopyranyl, S-oxodihydrothiopyranyl, S-dioxodihydrothiopyranyl, tetrahydrothiopyranyl, S-oxotetrahydrothiopyranyl, S-dioxotetrahydrothiopyranyl, morpholinyl, thiomorpholinyl, S-oxothiomorpholinyl, S-dioxothiomorpholinyl, thiazinyl and the like. Examples for heterocyclic rings that contain 1 or 2 carbonyl groups as ring members comprise pyrrolidin-2-onyl, pyrrolidin-2,5-dionyl, imidazolidin-2-onyl, oxazolidin-2-onyl, thiazolidin-2-only, and the like.

The term "hetaryl" includes monocyclic 5- or 6-membered heteroaromatic radicals comprising as ring members 1, 2, 3 or 4 heteroatoms selected from N, O, and S. Examples of 5- or 6-membered heteroaromatic radicals include pyridyl, i.e. 2-, 3-, or 4-pyridyl, pyrimidinyl, i.e. 2-, 4- or 5-pyrimidinyl, pyrazinyl, pyridazinyl, i.e. 3- or 4-pyridazinyl, thienyl, i.e. 2- or 3-thienyl, furyl, i.e. 2- or 3-furyl, pyrrolyl, i.e. 2- or 3-pyrrolyl, oxazolyl, i.e. 2-, 3- or 5-oxazolyl, isoxazolyl, i.e. 3-, 4- or 5-isoxazolyl, thiazolyl, i.e. 2-, 3- or 5-thiazolyl, isothiazolyl, i.e. 3-, 4- or 5-isothiazolyl, pyrazolyl, i.e. 1-, 3-, 4- or 5-pyrazolyl, i.e. 1-, 2-, 4- or 5-imidazolyl, oxadiazolyl, e.g. 2- or 5-[1,3,4]oxadiazolyl, 4- or 5-(1,2,3-oxadiazol)yl, 3- or 5-(1,2,4-oxadiazol)yl, 2- or 5-(1,3,4-thiadiazol)yl, thiadiazolyl, e.g. 2- or 5-(1,3,4-thiadiazol)yl, 4- or 5-(1,2,3-thiadiazol)yl, 3- or 5-(1,2,4-thiadiazol)yl, triazolyl, e.g. 1H-, 2H- or 3H-1,2,3-triazol-4-yl, 2H-triazol-3-yl, 1H-, 2H-, or 4H-1,2,4-triazolyl and tetrazolyl, i.e. 1H- or 2H-tetrazolyl. The term "hetaryl" also includes bicyclic 8 to 10-membered heteroaromatic radicals comprising as ring members 1, 2 or 3 heteroatoms selected from N, O, and S, wherein a 5- or 6-membered heteroaromatic ring is fused to a phenyl ring or to a 5- or 6-membered heteroaromatic radical. Examples of a 5- or 6-membered heteroaromatic ring fused to a phenyl ring or to a 5- or 6-membered heteroaromatic radical include benzofuranyl, benzothienyl, indolyl, indazolyl, benzimidazolyl, benzoxathiazolyl, benzoxadiazolyl, benzothiadiazolyl, benzoxazinyl, chinolinyl, isochinolinyl, purinyl, 1,8-naphthyridyl, pteridyl, pyrido[3,2-d]pyrimidyl or pyridoimidazolyl and the like. These fused hetaryl radicals may be bonded to the remainder of the molecule via any ring atom of 5- or 6-membered heteroaromatic ring or via a carbon atom of the fused phenyl moiety.

The terms "heterocyclylalkyl" and "hetarylalkyl" refer to heterocyclyl or hetaryl, respectively, as defined above which are bonded via a C₁-C₃-alkyl group or a C₁-C₄-alkyl group, in particular a methyl group (= heterocyclylmethyl or hetarylmethyl, respectively), to the remainder of the molecule.

The term "arylalkyl" and "phenylalkyl" refer to aryl as defined above and phenyl, respectively, which are bonded via C₁-C₅-alkyl group or a C₁-C₄-alkyl group, in particular a methyl group (= arylmethyl or phenylmethyl), to the remainder of the molecule, examples including benzyl, 1-phenylethyl, 2-phenylethyl, 2-phenoxyethyl etc.

The terms "alkylene", "cycloalkylene", "heterocycloalkylene", "alkenylene", "cycloalkenylene", "heterocycloalkenylene" and "alkynylene" refer to alkyl, cycloalkyl, heterocycloalkyl, alkenyl, cycloalkenyl, heterocycloalkenyl and alkynyl as defined above, respectively, which are bonded to the remainder of the molecule, via two atoms, preferably via two carbon atoms, of the respective group, so that they represent a linker between two moieties of the molecule.

In a particular embodiment, the variables of the compounds of the formula I have the following meanings, these meanings, both on their own and in combination with one another, being particular embodiments of the compounds of the formula I.

Embodiments and preferred compounds of the invention for use in pesticidal methods and for insecticidal application purposes are outlined in the following paragraphs.

With respect to the variables, the particularly preferred embodiments of the intermediates correspond to those of the compounds of the formula I.

In a preferred embodiment, the compounds I are present in form of a mixture of compounds I.A and I.B, wherein compound I.A with S-configuration of the carbon atom neighboring the nitrogen is present in an amount of more than 50% by weight, in particular of at least 70% by weight, more particularly of at least 85% by weight, specifically of at least 90% by weight, based on the total weight of compounds I.A and I.B.

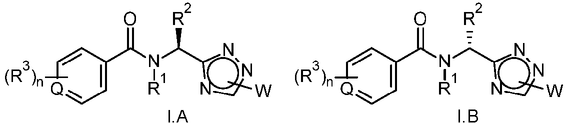

In one particularly preferred embodiment of the invention, the method comprises the step of contacting the plant, parts of it, its propagation material, the pests, their food supply, habitat or breeding grounds with a pesticidally effective amount of a compound of formula I.A.

Preferably R¹ is H, C₁-C₆-alkyl, C₃-C₆-cycloalkyl, or C₁-C₄-alkyl-C₃-C₆-cycloalkyl.

Preferably R² is CH₃.

R³ is preferably halogen, CN, C₁-C₄-haloalkyl, C₁-C₄-haloalkoxy, C₃-C₄-cycloalkyl, C₃-C₄-halocycloalkyl, S(O)ₘ-C₁-C₄-alkyl, S(O)ₘ-C₁-C₄-haloalkyl, S(O)ₘ-C₃-C₄-cyclo¬alkyl, S(O)ₘ-C₃-C₄-halocyclo-alkyl. Index m in R³ is preferably 2. Index n is preferably 2.

R³ groups stand preferably in positions 3 and 5. R³ groups stand preferably in meta positions.

In another embodiment R³ is preferably halogen, CN, C₁-C₄-haloalkyl, C₁-C₄-haloalkoxy, C₃-C₄-cycloalkyl, C₃-C₄-halocycloalkyl, S(O)ₘ-C₁-C₄-alkyl, S(O)ₘ₋C₁-C₄-haloalkyl, S(O)ₘ-C₃-C₄-cycloalkyl, S(O)ₘ-C₃-C₄-halocycloalkyl, or
S(O)ₘ-R¹⁴, wherein R¹⁴ is phenyl, which is partially substituted with R^{3a}.

In another embodiment R³ is S(O)ₘ-R¹⁴, wherein R¹⁴ is phenyl or hetaryl, such as pyridine, which rings are partially substituted with R^{3a}.

In another embodiment of formula I compounds R³ is halogen, CN, NO₂, C₁-C₄-alkyl, C₃-C₆-cycloalkyl, C₁-C₆-haloalkyl, C₁-C₆-halocycloalkyl, OR¹⁴, S(O)ₘ-R¹⁴; wherein rings are unsubstituted or substituted with R¹¹.

R⁴ is preferably H, C₁₋C₃-alkyl, or C₁-C₃-haloalkyl.

In another embodiment R⁴ is preferably H, C₁₋C₃-alkyl, or C₁-C₃-haloalkyl, or NR¹²R¹³, wherein R¹² and R¹³ are preferably selected from H and CH₃.

X is preferably NR⁵.

X is preferably NR⁵, with R⁵ being OH, C₁-C₃-alkoxy, or C₁-C₃-haloalkoxy.

In another embodiment X is O.

In a preferred embodiment, the compounds I.1 wherein X is NR⁵ are present in form of a mixture of compounds E-I.1 and Z-I.1, wherein compound E-I.1 with E-geometry of the C=N double bond is present in an amount of at least 50% by weight, in particular of at least 70% by weight, more particularly of at least 85% by weight, specifically of at least 90% by weight, based on the total weight of compounds E-I.1 and Z-I.1.

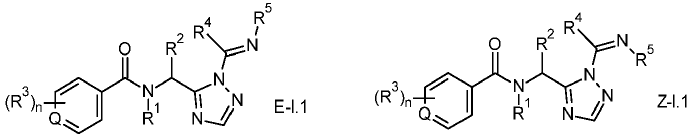

In a preferred embodiment of formula I compounds Q is CH or CR³.
in another embodiment of formula I compounds Q is N.

In preferred embodiment the triazole is a 1,5-disubstituted 1,2,4-triazole. Such compounds correspond to formula I.1.

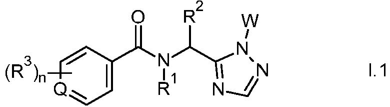

In another embodiment the triazole is a 1,3-disubstituted 1,2,4-triazole. Such compounds correspond to formula I.2.

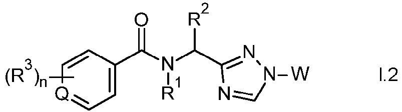

In another embodiment the triazole is a 1,2-disubstituted 1,3,4-triazole. Such compounds correspond to formula I.3.

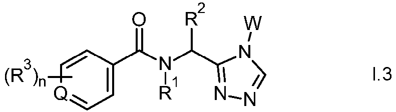

In formulae I.1, I.2, and I.3, resp., the variables are as defined and preferred for formula I.

Compounds of formula I.1 with the preferences above and below are particularly preferred.

In particular with a view to their use, preference is given to the compounds of formula I compiled in the tables below, which compounds correspond to formulae I.1, I.2A, and I.3A, resp.. Each of the groups mentioned for a substituent in the tables is furthermore per se, independently of the combination in which it is mentioned, a particularly preferred aspect of the substituent in question.

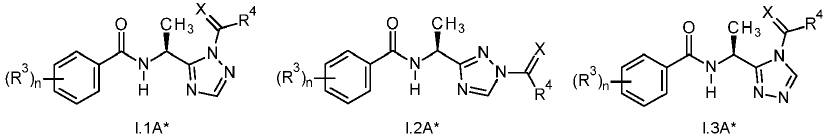

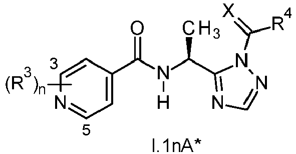

### Table 1

Compounds of formula I.1A* in which R⁴ is H, and the combination of X and (R³)ₙ for a compound corresponds in each case to one row of Table A

### Table 2

Compounds of formula I.1A* in which R⁴ is CN, and the combination of X and (R³)ₙ for a compound corresponds in each case to one row of Table A

### Table 3

Compounds of formula I.1A* in which R⁴ is CH₃, and the combination of X and (R³)ₙ for a compound corresponds in each case to one row of Table A

### Table 4

Compounds of formula I.1A* in which R⁴ is C₂H₅, and the combination of X and (R³)ₙ for a compound corresponds in each case to one row of Table A

### Table 5

Compounds of formula I.1A* in which R⁴ is CH₂CH₂CH₃, and the combination of X and (R³)ₙ for a compound corresponds in each case to one row of Table A

### Table 6

Compounds of formula I.1A* in which R⁴ is CH(CH₃)₂, and the combination of X and (R³)ₙ for a compound corresponds in each case to one row of Table A

### Table 7

Compounds of formula I.1A* in which R⁴ is c-C₃H₅, and the combination of X and (R³)ₙ for a compound corresponds in each case to one row of Table A

### Table 8

Compounds of formula I.1A* in which R⁴ is NH₂, and the combination of X and (R³)ₙ for a compound corresponds in each case to one row of Table A

### Table 9

Compounds of formula I.1A* in which R⁴ is C(=O)NH₂, and the combination of X and (R³)ₙ for a compound corresponds in each case to one row of Table A

### Table 10

Compounds of formula I.1A* in which R⁴ is C(=O)NHCH₃, and the combination of X and (R³)ₙ for a compound corresponds in each case to one row of Table A

### Table 11

Compounds of formula I.1A* in which R⁴ is C(=O)NHC₂H₃, and the combination of X and (R³)ₙ for a compound corresponds in each case to one row of Table A

### Table 12

Compounds of formula I.1A* in which R⁴ is C(=O)N(CH₃)₂, and the combination of X and (R³)ₙ for a compound corresponds in each case to one row of Table A

### Table 13

Compounds of formula I.1A* in which R⁴ is C(=O)N(CH₃)C₂H₅, and the combination of X and (R³)ₙ for a compound corresponds in each case to one row of Table A

### Table 14

Compounds of formula I.1A* in which R⁴ is C(=O)OCH₃, and the combination of X and (R³)ₙ for a compound corresponds in each case to one row of Table A

### Table 15

Compounds of formula I.1A* in which R⁴ is C(=O)OC₂H₅, and the combination of X and (R³)ₙ for a compound corresponds in each case to one row of Table A

### Table 16

Compounds of formula I.1A* in which R⁴ is 4,5-dihydrooxazol-2-yl, and the combination of X and (R³)ₙ for a compound corresponds in each case to one row of Table A

### Table 17

Compounds of formula I.2A* in which R⁴ is H, and the combination of X and (R³)ₙ for a compound corresponds in each case to one row of Table A

### Table 18

Compounds of formula I.2A* in which R⁴ is CN, and the combination of X and (R³)ₙ for a compound corresponds in each case to one row of Table A

### Table 19

Compounds of formula I.2A* in which R⁴ is CH₃, and the combination of X and (R³)ₙ for a compound corresponds in each case to one row of Table A

### Table 20

Compounds of formula I.2A* in which R⁴ is C₂H₅, and the combination of X and (R³)ₙ for a compound corresponds in each case to one row of Table A

### Table 21

Compounds of formula I.2A* in which R⁴ is CH₂CH₂CH₃, and the combination of X and (R³)ₙ for a compound corresponds in each case to one row of Table A

### Table 22

Compounds of formula I.2A* in which R⁴ is CH(CH₃)₂, and the combination of X and (R³)ₙ for a compound corresponds in each case to one row of Table A

### Table 23

Compounds of formula I.2A* in which R⁴ is c-C₃H₅, and the combination of X and (R³)ₙ for a compound corresponds in each case to one row of Table A

### Table 24

Compounds of formula I.2A* in which R⁴ is NH₂, and the combination of X and (R³)ₙ for a compound corresponds in each case to one row of Table A

### Table 25

Compounds of formula I.2A* in which R⁴ is C(=O)NH₂, and the combination of X and (R³)ₙ for a compound corresponds in each case to one row of Table A

### Table 26

Compounds of formula I.2A* in which R⁴ is C(=O)NHCH₃, and the combination of X and (R³)ₙ for a compound corresponds in each case to one row of Table A

### Table 27

Compounds of formula I.2A* in which R⁴ is C(=O)NHC₂H₃, and the combination of X and (R³)ₙ for a compound corresponds in each case to one row of Table A

### Table 28

Compounds of formula I.2A* in which R⁴ is C(=O)N(CH₃)₂, and the combination of X and (R³)ₙ for a compound corresponds in each case to one row of Table A

### Table 29

Compounds of formula I.2A* in which R⁴ is C(=O)N(CH₃)C₂H₅, and the combination of X and (R³)ₙ for a compound corresponds in each case to one row of Table A

### Table 30

Compounds of formula I.2A* in which R⁴ is C(=O)OCH₃, and the combination of X and (R³)ₙ for a compound corresponds in each case to one row of Table A

### Table 31

Compounds of formula I.2A* in which R⁴ is C(=O)OC₂H₅, and the combination of X and (R³)ₙ for a compound corresponds in each case to one row of Table A

### Table 32

Compounds of formula I.2A* in which R⁴ is 4,5-dihydrooxazol-2-yl, and the combination of X and (R³)ₙ for a compound corresponds in each case to one row of Table A

### Table 33

Compounds of formula I.3A* in which R⁴ is H, and the combination of X and (R³)ₙ for a compound corresponds in each case to one row of Table A

### Table 34

Compounds of formula I.3A* in which R⁴ is CN, and the combination of X and (R³)ₙ for a compound corresponds in each case to one row of Table A

### Table 35

Compounds of formula I.3A* in which R⁴ is CH₃, and the combination of X and (R³)ₙ for a compound corresponds in each case to one row of Table A

### Table 36

Compounds of formula I.3A* in which R⁴ is C₂H₅, and the combination of X and (R³)ₙ for a compound corresponds in each case to one row of Table A

### Table 37

Compounds of formula I.3A* in which R⁴ is CH₂CH₂CH₃, and the combination of X and (R³)ₙ for a compound corresponds in each case to one row of Table A

### Table 38

Compounds of formula I.3A* in which R⁴ is CH(CH₃)₂, and the combination of X and (R³)ₙ for a compound corresponds in each case to one row of Table A

### Table 39

Compounds of formula I.3A* in which R⁴ is c-C₃H₅, and the combination of X and (R³)ₙ for a compound corresponds in each case to one row of Table A

### Table 40

Compounds of formula I.3A* in which R⁴ is NH₂, and the combination of X and (R³)ₙ for a compound corresponds in each case to one row of Table A

### Table 41

Compounds of formula I.3A* in which R⁴ is C(=O)NH₂, and the combination of X and (R³)ₙ for a compound corresponds in each case to one row of Table A

### Table 42

Compounds of formula I.3A* in which R⁴ is C(=O)NHCH₃, and the combination of X and (R³)ₙ for a compound corresponds in each case to one row of Table A

### Table 43

Compounds of formula I.3A* in which R⁴ is C(=O)NHC₂H₅, and the combination of X and (R³)ₙ for a compound corresponds in each case to one row of Table A

### Table 44

Compounds of formula I.3A* in which R⁴ is C(=O)N(CH₃)₂, and the combination of X and (R³)ₙ for a compound corresponds in each case to one row of Table A

### Table 45

Compounds of formula I.3A* in which R⁴ is C(=O)N(CH₃)C₂H₅, and the combination of X and (R³)ₙ for a compound corresponds in each case to one row of Table A

### Table 46

Compounds of formula I.3A* in which R⁴ is C(=O)OCH₃, and the combination of X and (R³)ₙ for a compound corresponds in each case to one row of Table A

### Table 47

Compounds of formula I.3A* in which R⁴ is C(=O)OC₂H₅, and the combination of X and (R³)ₙ for a compound corresponds in each case to one row of Table A

### Table 48

Compounds of formula I.3A* in which R⁴ is 4,5-dihydrooxazol-2-yl, and the combination of X and (R³)ₙ for a compound corresponds in each case to one row of Table A

### Table 49

Compounds of formula I.1nA* in which R⁴ is H, and the combination of X and (R³)ₙ for a compound corresponds in each case to one row of Table A

### Table 50

Compounds of formula I.1nA* in which R⁴ is CN, and the combination of X and (R³)ₙ for a compound corresponds in each case to one row of Table A

### Table 51

Compounds of formula I.1nA* in which R⁴ is CH₃, and the combination of X and (R³)ₙ for a compound corresponds in each case to one row of Table A

### Table 52

Compounds of formula I.1nA* in which R⁴ is C₂H₅, and the combination of X and (R³)ₙ for a compound corresponds in each case to one row of Table A

### Table 53

Compounds of formula I.1nA* in which R⁴ is CH₂CH₂CH₃, and the combination of X and (R³)ₙ for a compound corresponds in each case to one row of Table A

### Table 54

Compounds of formula I.1nA* in which R⁴ is CH(CH₃)₂, and the combination of X and (R³)ₙ for a compound corresponds in each case to one row of Table A

### Table 55

Compounds of formula I.1nA* in which R⁴ is c-C₃H₅, and the combination of X and (R³)ₙ for a compound corresponds in each case to one row of Table A

### Table 56

Compounds of formula I.1nA* in which R⁴ is NH₂, and the combination of X and (R³)ₙ for a compound corresponds in each case to one row of Table A

### Table 57

Compounds of formula I.1nA* in which R⁴ is C(=O)NH₂, and the combination of X and (R³)ₙ for a compound corresponds in each case to one row of Table A

### Table 58

Compounds of formula I.1nA* in which R⁴ is C(=O)NHCH₃, and the combination of X and (R³)ₙ for a compound corresponds in each case to one row of Table A

### Table 59

Compounds of formula I.1nA* in which R⁴ is C(=O)NHC₂H₃, and the combination of X and (R³)ₙ for a compound corresponds in each case to one row of Table A

### Table 60

Compounds of formula I.1nA* in which R⁴ is C(=O)N(CH₃)₂, and the combination of X and (R³)ₙ for a compound corresponds in each case to one row of Table A

### Table 61

Compounds of formula I.1nA* in which R⁴ is C(=O)N(CH₃)C₂H₅, and the combination of X and (R³)ₙ for a compound corresponds in each case to one row of Table A

### Table 62

Compounds of formula I.1nA* in which R⁴ is C(=O)OCH₃, and the combination of X and (R³)ₙ for a compound corresponds in each case to one row of Table A

### Table 63

Compounds of formula I.1nA* in which R⁴ is C(=O)OC₂H₅, and the combination of X and (R³)ₙ for a compound corresponds in each case to one row of Table A

### Table 64

Compounds of formula I.1nA* in which R⁴ is 4,5-dihydrooxazol-2-yl, and the combination of X and (R³)ₙ for a compound corresponds in each case to one row of Table A

**Table A**

| No. | X | (R³)ₙ |
|---|---|---|
| A-1 | O | 3,5-F₂ |
| A-2 | NOH | 3,5-F₂ |
| A-3 | (Z)-NOH | 3,5-F₂ |
| A-4 | (E)-NOH | 3,5-F₂ |
| A-5 | (Z)-N-OCH₃ | 3,5-F₂ |
| A-6 | (E)-N-OCH₃ | 3,5-F₂ |
| A-7 | (Z)-N-OC₂H₅ | 3,5-F₂ |
| A-8 | (E)-N-OC₂H₅ | 3,5-F₂ |
| A-9 | (Z)-N-OCH₂CF₃ | 3,5-F₂ |
| A-10 | (E)-N-OCH₂CF₃ | 3,5-F₂ |
| A-11 | (Z)-N-OCH₂C₆H₅ | 3,5-F₂ |
| A-12 | (E)-N-OCH₂C₆H₅ | 3,5-F₂ |
| A-13 | O | 3,5-Cl₂ |
| A-14 | NOH | 3,5-Cl₂ |
| A-15 | (Z)-NOH | 3,5-Cl₂ |
| A-16 | (E)-NOH | 3,5-Cl₂ |
| A-17 | (Z)-N-OCH₃ | 3,5-Cl₂ |
| A-18 | (E)-N-OCH₃ | 3,5-Cl₂ |
| A-19 | (Z)-N-OC₂H₅ | 3,5-Cl₂ |
| A-20 | (E)-N-OC₂H₅ | 3,5-Cl₂ |
| A-21 | (Z)-N-OCH₂CF₃ | 3,5-Cl₂ |
| A-22 | (E)-N-OCH₂CF₃ | 3,5-Cl₂ |
| A-23 | (Z)-N-OCH₂C₆H₅ | 3,5-Cl₂ |
| A-24 | (E)-N-OCH₂C₆H₅ | 3,5-Cl₂ |
| A-25 | O | 3,5-Br₂ |
| A-26 | NOH | 3,5-Br₂ |
| A-27 | (Z)-NOH | 3,5-Br₂ |
| A-28 | (E)-NOH | 3,5-Br₂ |
| A-29 | (Z)-N-OCH₃ | 3,5-Br₂ |
| A-30 | (E)-N-OCH₃ | 3,5-Br₂ |
| A-31 | (Z)-N-OC₂H₅ | 3,5-Br₂ |
| A-32 | (E)-N-OC₂H₅ | 3,5-Br₂ |
| A-33 | (Z)-N-OCH₂CF₃ | 3,5-Br₂ |
| A-34 | (E)-N-OCH₂CF₃ | 3,5-Br₂ |
| A-35 | (Z)-N-OCH₂C₆H₅ | 3,5-Br₂ |
| A-36 | (E)-N-OCH₂C₆H₅ | 3,5-Br₂ |
| A-37 | O | 3,5-I₂ |
| A-38 | NOH | 3,5-I₂ |
| A-39 | (Z)-N-OCH₃ | 3,5-I₂ |
| A-40 | (E)-N-OCH₃ | 3,5-I₂ |
| A-41 | (Z)-N-OC₂H₅ | 3,5-I₂ |
| A-42 | (E)-N-OC₂H₅ | 3,5-I₂ |
| A-43 | (Z)-N-OCH₂CF₃ | 3,5-I₂ |
| A-44 | (E)-N-OCH₂CF₃ | 3,5-I₂ |
| A-45 | (Z)-N-OCH₂C₆H₅ | 3,5-I₂ |
| A-46 | (E)-N-OCH₂C₆H₅ | 3,5-I₂ |
| A-47 | O | 3,5-(CF₃)₂ |
| A-48 | NOH | 3,5-(CF₃)₂ |
| A-49 | (Z)-NOH | 3,5-(CF₃)₂ |
| A-50 | (E)-NOH | 3,5-(CF₃)₂ |
| A-51 | (Z)-N-OCH₃ | 3,5-(CF₃)₂ |
| A-52 | (E)-N-OCH₃ | 3,5-(CF₃)₂ |
| A-53 | (Z)-N-OC₂H₅ | 3,5-(CF₃)₂ |
| A-54 | (E)-N-OC₂H₅ | 3,5-(CF₃)₂ |
| A-55 | (Z)-N-OCH₂CF₃ | 3,5-(CF₃)₂ |
| A-56 | (E)-N-OCH₂CF₃ | 3,5-(CF₃)₂ |
| A-57 | (Z)-N-OCH₂C₆H₅ | 3,5-(CF₃)₂ |
| A-58 | (E)-N-OCH₂C₆H₅ | 3,5-(CF₃)₂ |
| A-59 | O | 3-Cl,5-F |
| A-60 | NOH | 3-Cl,5-F |
| A-61 | (Z)-NOH | 3-Cl,5-F |
| A-62 | (E)-NOH | 3-Cl,5-F |
| A-63 | (Z)-N-OCH₃ | 3-Cl,5-F |
| A-64 | (E)-N-OCH₃ | 3-Cl,5-F |
| A-65 | (Z)-N-OC₂H₅ | 3-Cl,5-F |
| A-66 | (E)-N-OC₂H₅ | 3-Cl,5-F |
| A-67 | (Z)-N-OCH₂CF₃ | 3-Cl,5-F |
| A-68 | (E)-N-OCH₂CF₃ | 3-Cl,5-F |
| A-69 | (Z)-N-OCH₂C₆H₅ | 3-Cl,5-F |
| A-70 | (E)-N-OCH₂C₆H₅ | 3-Cl,5-F |
| A-71 | O | 3-Cl,5-Br |
| A-72 | NOH | 3-Cl,5-Br |
| A-73 | (Z)-NOH | 3-Cl,5-Br |
| A-74 | (E)-NOH | 3-Cl,5-Br |
| A-75 | (Z)-N-OCH₃ | 3-Cl,5-Br |
| A-76 | (E)-N-OCH₃ | 3-Cl,5-Br |
| A-77 | (Z)-N-OC₂H₅ | 3-Cl,5-Br |
| A-78 | (E)-N-OC₂H₅ | 3-Cl,5-Br |
| A-79 | (Z)-N-OCH₂CF₃ | 3-Cl,5-Br |
| A-80 | (E)-N-OCH₂CF₃ | 3-Cl,5-Br |
| A-81 | (Z)-N-OCH₂C₆H₅ | 3-Cl,5-Br |
| A-82 | (E)-N-OCH₂C₆H₅ | 3-Cl,5-Br |
| A-83 | O | 3-Cl,5-I |
| A-84 | NOH | 3-Cl,5-I |
| A-85 | (Z)-NOH | 3-Cl,5-I |
| A-86 | (E)-NOH | 3-Cl,5-I |
| A-87 | (Z)-N-OCH₃ | 3-Cl,5-I |
| A-88 | (E)-N-OCH₃ | 3-Cl,5-I |
| A-89 | (Z)-N-OC₂H₅ | 3-Cl,5-I |
| A-90 | (E)-N-OC₂H₅ | 3-Cl,5-I |
| A-91 | (Z)-N-OCH₂CF₃ | 3-Cl,5-I |
| A-92 | (E)-N-OCH₂CF₃ | 3-Cl,5-I |
| A-93 | (Z)-N-OCH₂C₆H₅ | 3-Cl,5-I |
| A-94 | (E)-N-OCH₂C₆H₅ | 3-Cl,5-I |
| A-95 | O | 3-F,5-CN |
| A-96 | NOH | 3-F,5-CN |
| A-97 | (Z)-NOH | 3-F,5-CN |
| A-98 | (E)-NOH | 3-F,5-CN |
| A-99 | (Z)-N-OCH₃ | 3-F,5-CN |
| A-100 | (E)-N-OCH₃ | 3-F,5-CN |
| A-101 | (Z)-N-OC₂H₅ | 3-F,5-CN |
| A-102 | (E)-N-OC₂H₅ | 3-F,5-CN |
| A-103 | (Z)-N-OCH₂CF₃ | 3-F,5-CN |
| A-104 | (E)-N-OCH₂CF₃ | 3-F,5-CN |
| A-105 | (Z)-N-OCH₂C₆H₅ | 3-F,5-CN |
| A-106 | (E)-N-OCH₂C₆H₅ | 3-F,5-CN |
| A-107 | O | 3-Cl,5-CN |
| A-108 | NOH | 3-Cl,5-CN |
| A-109 | (Z)-NOH | 3-Cl,5-CN |
| A-110 | (E)-NOH | 3-Cl,5-CN |
| A-111 | (Z)-N-OCH₃ | 3-Cl,5-CN |
| A-112 | (E)-N-OCH₃ | 3-Cl,5-CN |
| A-113 | (Z)-N-OC₂H₅ | 3-Cl,5-CN |
| A-114 | (E)-N-OC₂H₅ | 3-Cl,5-CN |
| A-115 | (Z)-N-OCH₂CF₃ | 3-Cl,5-CN |
| A-116 | (E)-N-OCH₂CF₃ | 3-Cl,5-CN |
| A-117 | (Z)-N-OCH₂C₆H₅ | 3-Cl,5-CN |
| A-118 | (E)-N-OCH₂C₆H₅ | 3-Cl,5-CN |
| A-119 | O | 3-CF₃,5-CN |
| A-120 | NOH | 3-CF₃,5-CN |
| A-121 | (Z)-NOH | 3-CF₃,5-CN |
| A-122 | (E)-NOH | 3-CF₃,5-CN |
| A-123 | (Z)-N-OCH₃ | 3-CF₃,5-CN |
| A-124 | (E)-N-OCH₃ | 3-CF₃,5-CN |
| A-125 | (Z)-N-OC₂H₅ | 3-CF₃,5-CN |
| A-126 | (E)-N-OC₂H₅ | 3-CF₃,5-CN |
| A-127 | (Z)-N-OCH₂CF₃ | 3-CF₃,5-CN |
| A-128 | (E)-N-OCH₂CF₃ | 3-CF₃,5-CN |
| A-129 | (Z)-N-OCH₂C₆H₅ | 3-CF₃,5-CN |
| A-130 | (E)-N-OCH₂C₆H₅ | 3-CF₃,5-CN |
| A-131 | O | 3-F,5-CF₃ |
| A-132 | NOH | 3-F,5-CF₃ |
| A-133 | (Z)-NOH | 3-F,5-CF₃ |
| A-134 | (E)-NOH | 3-F,5-CF₃ |
| A-135 | (Z)-N-OCH₃ | 3-F,5-CF₃ |
| A-136 | (E)-N-OCH₃ | 3-F,5-CF₃ |
| A-137 | (Z)-N-OC₂H₅ | 3-F,5-CF₃ |
| A-138 | (E)-N-OC₂H₅ | 3-F,5-CF₃ |
| A-139 | (Z)-N-OCH₂CF₃ | 3-F,5-CF₃ |
| A-140 | (E)-N-OCH₂CF₃ | 3-F,5-CF₃ |
| A-141 | (Z)-N-OCH₂C₆H₅ | 3-F,5-CF₃ |
| A-142 | (E)-N-OCH₂C₆H₅ | 3-F,5-CF₃ |
| A-143 | O | 3-Cl,5-CF₃ |
| A-144 | NOH | 3-Cl,5-CF₃ |
| A-145 | (Z)-NOH | 3-Cl,5-CF₃ᵥ |
| A-146 | (E)-NOH | 3-Cl,5-CF₃ |
| A-147 | (Z)-N-OCH₃ | 3-Cl,5-CF₃ |
| A-148 | (E)-N-OCH₃ | 3-Cl,5-CF₃ |
| A-149 | (Z)-N-OC₂H₅ | 3-Cl,5-CF₃ |
| A-150 | (E)-N-OC₂H₅ | 3-Cl,5-CF₃ |
| A-151 | (Z)-N-OCH₂CF₃ | 3-Cl,5-CF₃ |
| A-152 | (E)-N-OCH₂CF₃ | 3-Cl,5-CF₃ |
| A-153 | (Z)-N-OCH₂C₆H₅ | 3-Cl,5-CF₃ |
| A-154 | (E)-N-OCH₂C₆H₅ | 3-Cl,5-CF₃ |
| A-155 | O | 3-Br,5-CF₃ |
| A-156 | NOH | 3-Br,5-CF₃ |
| A-157 | (Z)-NOH | 3-Br,5-CF₃ |
| A-158 | (E)-NOH | 3-Br,5-CF₃ |
| A-159 | (Z)-N-OCH₃ | 3-Br,5-CF₃ |
| A-160 | (E)-N-OCH₃ | 3-Br,5-CF₃ |
| A-161 | (Z)-N-OC₂H₅ | 3-Br,5-CF₃ |
| A-162 | (E)-N-OC₂H₅ | 3-Br,5-CF₃ |
| A-163 | (Z)-N-OCH₂CF₃ | 3-Br,5-CF₃ |
| A-164 | (E)-N-OCH₂CF₃ | 3-Br,5-CF₃ |
| A-165 | (Z)-N-OCH₂C₆H₅ | 3-Br,5-CF₃ |
| A-166 | (E)-N-OCH₂C₆H₅ | 3-Br,5-CF₃ |
| A-167 | O | 3-I,5-CF₃ |
| A-168 | NOH | 3-I,5-CF₃ |
| A-169 | (Z)-NOH | 3-I,5-CF₃ |
| A-170 | (E)-NOH | 3-I,5-CF₃ |
| A-171 | (Z)-N-OCH₃ | 3-I,5-CF₃ |
| A-172 | (E)-N-OCH₃ | 3-I,5-CF₃ |
| A-173 | (Z)-N-OC₂H₅ | 3-I,5-CF₃ |
| A-174 | (E)-N-OC₂H₅ | 3-I,5-CF₃ |
| A-175 | (Z)-N-OCH₂CF₃ | 3-I,5-CF₃ |
| A-176 | (E)-N-OCH₂CF₃ | 3-I,5-CF₃ |
| A-177 | (Z)-N-OCH₂C₆H₅ | 3-I,5-CF₃ |
| A-178 | (E)-N-OCH₂C₆H₅ | 3-I,5-CF₃ |
| A-179 | O | 3-Cl,5-SO₂CH₃ |
| A-180 | NOH | 3-Cl,5-SO₂CH₃ |
| A-181 | (Z)-NOH | 3-Cl,5-SO₂CH₃ |
| A-182 | (E)-NOH | 3-Cl,5-SO₂CH₃ |
| A-183 | (Z)-N-OCH₃ | 3-Cl,5-SO₂CH₃ |
| A-184 | (E)-N-OCH₃ | 3-Cl,5-SO₂CH₃ |
| A-185 | (Z)-N-OC₂H₅ | 3-Cl,5-SO₂CH₃ |
| A-186 | (E)-N-OC₂H₅ | 3-Cl,5-SO₂CH₃ |
| A-187 | (Z)-N-OCH₂CF₃ | 3-Cl,5-SO₂CH₃ |
| A-188 | (E)-N-OCH₂CF₃ | 3-Cl,5-SO₂CH₃ |
| A-189 | (Z)-N-OCH₂C₆H₅ | 3-Cl,5-SO₂CH₃ |
| A-190 | (E)-N-OCH₂C₆H₅ | 3-Cl,5-SO₂CH₃ |
| A-191 | O | 3-Cl,5-SO₂CF₃ |
| A-192 | NOH | 3-Cl,5-SO₂CF₃ |
| A-193 | (Z)-NOH | 3-Cl,5-SO₂CF₃ |
| A-194 | (E)-NOH | 3-Cl,5-SO₂CF₃ |
| A-195 | (Z)-N-OCH₃ | 3-Cl,5-SO₂CF₃ |
| A-196 | (E)-N-OCH₃ | 3-Cl,5-SO₂CF₃ |
| A-197 | (Z)-N-OC₂H₅ | 3-Cl,5-SO₂CF₃ |
| A-198 | (E)-N-OC₂H₅ | 3-Cl,5-SO₂CF₃ |
| A-199 | (Z)-N-OCH₂CF₃ | 3-Cl,5-SO₂CF₃ |
| A-200 | (E)-N-OCH₂CF₃ | 3-Cl,5-SO₂CF₃ |
| A-201 | (Z)-N-OCH₂C₆H₅ | 3-Cl,5-SO₂CF₃ |
| A-202 | (E)-N-OCH₂C₆H₅ | 3-Cl,5-SO₂CF₃ |
| A-203 | O | 3-Cl,5-c-C₃H₅ |
| A-204 | NOH | 3-Cl,5-c-C₃H₅ |
| A-205 | (Z)-NOH | 3-Cl,5-c-C₃H₅ |
| A-206 | (E)-NOH | 3-Cl,5-c-C₃H₅ |
| A-207 | (Z)-N-OCH₃ | 3-Cl,5-c-C₃H₅ |
| A-208 | (E)-N-OCH₃ | 3-Cl,5-c-C₃H₅ |
| A-209 | (Z)-N-OC₂H₅ | 3-Cl,5-c-C₃H₅ |
| A-210 | (E)-N-OC₂H₅ | 3-Cl,5-c-C₃H₅ |
| A-211 | (Z)-N-OCH₂CF₃ | 3-Cl,5-c-C₃H₅ |
| A-212 | (E)-N-OCH₂CF₃ | 3-Cl,5-c-C₃H₅ |
| A-213 | (Z)-N-OCH₂C₆H₅ | 3-Cl,5-c-C₃H₅ |
| A-214 | (E)-N-OCH₂C₆H₅ | 3-Cl,5-c-C₃H₅ |
| A-215 | O | 3-Cl, 5-[2,2-Cl₂-C-C₃H₃] |
| A-216 | NOH | 3-Cl, 5-[2,2-Cl₂-C-C₃H₃] |
| A-217 | (Z)-NOH | 3-Cl, 5-[2,2-Cl₂-C-C₃H₃] |
| A-218 | (E)-NOH | 3-Cl, 5-[2,2-Cl₂-C-C₃H₃] |
| A-219 | (Z)-N-OCH₃ | 3-Cl, 5-[2,2-Cl₂-C-C₃H₃] |
| A-220 | (E)-N-OCH₃ | 3-Cl, 5-[2,2-Cl₂-C-C₃H₃] |
| A-221 | (Z)-N-OC₂H₅ | 3-Cl, 5-[2,2-Cl₂-C-C₃H₃] |
| A-222 | (E)-N-OC₂H₅ | 3-Cl, 5-[2,2-Cl₂-C-C₃H₃] |
| A-223 | (Z)-N-OCH₂CF₃ | 3-Cl, 5-[2,2-Cl₂-C-C₃H₃] |
| A-224 | (E)-N-OCH₂CF₃ | 3-Cl, 5-[2,2-Cl₂-C-C₃H₃] |
| A-225 | (Z)-N-OCH₂C₆H₅ | 3-Cl, 5-[2,2-Cl₂-C-C₃H₃] |
| A-226 | (E)-N-OCH₂C₆H₅ | 3-Cl, 5-[2,2-Cl₂-C-C₃H₃] |
| A-227 | O | 3-Cl,5-OCF₃ |
| A-228 | NOH | 3-Cl,5-OCF₃ |
| A-229 | (Z)-NOH | 3-Cl,5-OCF₃ |
| A-230 | (E)-NOH | 3-Cl,5-OCF₃ |
| A-231 | (Z)-N-OCH₃ | 3-Cl,5-OCF₃ |
| A-232 | (E)-N-OCH₃ | 3-Cl,5-OCF₃ |
| A-233 | (Z)-N-OC₂H₅ | 3-Cl,5-OCF₃ |
| A-234 | (E)-N-OC₂H₅ | 3-Cl,5-OCF₃ |
| A-235 | (Z)-N-OCH₂CF₃ | 3-Cl,5-OCF₃ |
| A-236 | (E)-N-OCH₂CF₃ | 3-Cl,5-OCF₃ |
| A-237 | (Z)-N-OCH₂C₆H₅ | 3-Cl,5-OCF₃ |
| A-238 | (E)-N-OCH₂C₆H₅ | 3-Cl,5-OCF₃ |
| A-239 | O | 3-Br,5-OCF₃ |
| A-240 | NOH | 3-Br,5-OCF₃ |
| A-241 | (Z)-NOH | 3-Br,5-OCF₃ |
| A-242 | (E)-NOH | 3-Br,5-OCF₃ |
| A-243 | (Z)-N-OCH₃ | 3-Br,5-OCF₃ |
| A-244 | (E)-N-OCH₃ | 3-Br,5-OCF₃ |
| A-245 | (Z)-N-OC₂H₅ | 3-Br,5-OCF₃ |
| A-246 | (E)-N-OC₂H₅ | 3-Br,5-OCF₃ |
| A-247 | (Z)-N-OCH₂CF₃ | 3-Br,5-OCF₃ |
| A-248 | (E)-N-OCH₂CF₃ | 3-Br,5-OCF₃ |
| A-249 | (Z)-N-OCH₂C₆H₅ | 3-Br,5-OCF₃ |
| A-250 | (E)-N-OCH₂C₆H₅ | 3-Br,5-OCF₃ |
| A-251 | O | 3-F,5-c-C₃H₅ |
| A-252 | NOH | 3-F,5-c-C₃H₅ |
| A-253 | (Z)-NOH | 3-F,5-c-C₃H₅ |
| A-254 | (E)-NOH | 3-F,5-c-C₃H₅ |
| A-255 | (Z)-N-OCH₃ | 3-F,5-c-C₃H₅ |
| A-256 | (E)-N-OCH₃ | 3-F,5-c-C₃H₅ |
| A-257 | (Z)-N-OC₂H₅ | 3-F,5-c-C₃H₅ |
| A-258 | (E)-N-OC₂H₅ | 3-F,5-c-C₃H₅ |
| A-259 | (Z)-N-OCH₂CF₃ | 3-F,5-c-C₃H₅ |
| A-260 | (E)-N-OCH₂CF₃ | 3-F,5-c-C₃H₅ |
| A-261 | (Z)-N-OCH₂C₆H₅ | 3-F,5-c-C₃H₅ |
| A-262 | (E)-N-OCH₂C₆H₅ | 3-F,5-c-C₃H₅ |
| A-263 | O | 3-Cl,5-CH₂CN |
| A-264 | NOH | 3-Cl,5-CH₂CN |
| A-265 | (Z)-NOH | 3-Cl,5-CH₂CN |
| A-266 | (E)-NOH | 3-Cl,5-CH₂CN |
| A-267 | (Z)-N-OCH₃ | 3-Cl,5-CH₂CN |
| A-268 | (E)-N-OCH₃ | 3-Cl,5-CH₂CN |
| A-269 | (Z)-N-OC₂H₅ | 3-Cl,5-CH₂CN |
| A-270 | (E)-N-OC₂H₅ | 3-Cl,5-CH₂CN |
| A-271 | (Z)-N-OCH₂CF₃ | 3-Cl,5-CH₂CN |
| A-272 | (E)-N-OCH₂CF₃ | 3-Cl,5-CH₂CN |
| A-273 | (Z)-N-OCH₂C₆H₅ | 3-Cl,5-CH₂CN |
| A-274 | (E)-N-OCH₂C₆H₅ | 3-Cl,5-CH₂CN |
| A-275 | O | 3-Cl,5-C(CH₃)₂CN |
| A-276 | NOH | 3-Cl,5-C(CH₃)₂CN |
| A-277 | (Z)-NOH | 3-Cl,5-C(CH₃)₂CN |
| A-278 | (E)-NOH | 3-Cl,5-C(CH₃)₂CN |
| A-279 | (Z)-N-OCH₃ | 3-Cl,5-C(CH₃)₂CN |
| A-280 | (E)-N-OCH₃ | 3-Cl,5-C(CH₃)₂CN |
| A-281 | (Z)-N-OC₂H₅ | 3-Cl,5-C(CH₃)₂CN |
| A-282 | (E)-N-OC₂H₅ | 3-Cl,5-C(CH₃)₂CN |
| A-283 | (Z)-N-OCH₂CF₃ | 3-Cl,5-C(CH₃)₂CN |
| A-284 | (E)-N-OCH₂CF₃ | 3-Cl,5-C(CH₃)₂CN |
| A-285 | (Z)-N-OCH₂C₆H₅ | 3-Cl,5-C(CH₃)₂CN |
| A-286 | (E)-N-OCH₂C₆H₅ | 3-Cl,5-C(CH₃)₂CN |
| A-287 | O | 3-CF₃,5-CH₂CN |
| A-288 | NOH | 3-CF₃,5-CH₂CN |
| A-289 | (Z)-NOH | 3-CF₃,5-CH₂CN |
| A-290 | (E)-NOH | 3-CF₃,5-CH₂CN |
| A-291 | (Z)-N-OCH₃ | 3-CF₃,5-CH₂CN |
| A-292 | (E)-N-OCH₃ | 3-CF₃,5-CH₂CN |
| A-293 | (Z)-N-OC₂H₅ | 3-CF₃,5-CH₂CN |
| A-294 | (E)-N-OC₂H₅ | 3-CF₃,5-CH₂CN |
| A-295 | (Z)-N-OCH₂CF₃ | 3-CF₃,5-CH₂CN |
| A-296 | (E)-N-OCH₂CF₃ | 3-CF₃,5-CH₂CN |
| A-297 | (Z)-N-OCH₂C₆H₅ | 3-CF₃,5-CH₂CN |
| A-298 | (E)-N-OCH₂C₆H₅ | 3-CF₃,5-CH₂CN |
| A-299 | O | 3-CF₃, 5-C(CH₃)₂CN |
| A-300 | NOH | 3-CF₃, 5-C(CH₃)₂CN |
| A-301 | (Z)-NOH | 3-CF₃, 5-C(CH₃)₂CN |
| A-302 | (E)-NOH | 3-CF₃, 5-C(CH₃)₂CN |
| A-303 | (Z)-N-OCH₃ | 3-CF₃, 5-C(CH₃)₂CN |
| A-304 | (E)-N-OCH₃ | 3-CF₃, 5-C(CH₃)₂CN |
| A-305 | (Z)-N-OC₂H₅ | 3-CF₃, 5-C(CH₃)₂CN |
| A-306 | (E)-N-OC₂H₅ | 3-CF₃, 5-C(CH₃)₂CN |
| A-307 | (Z)-N-OCH₂CF₃ | 3-CF₃, 5-C(CH₃)₂CN |
| A-308 | (E)-N-OCH₂CF₃ | 3-CF₃, 5-C(CH₃)₂CN |
| A-309 | (Z)-N-OCH₂C₆H₅ | 3-CF₃, 5-C(CH₃)₂CN |
| A-310 | (E)-N-OCH₂C₆H₅ | 3-CF₃, 5-C(CH₃)₂CN |
| A-311 | O | 3-CF₃,5-SO₂CH₃ |
| A-312 | NOH | 3-CF₃,5-SO₂CH₃ |
| A-313 | (Z)-NOH | 3-CF₃,5-SO₂CH₃ |
| A-314 | (E)-NOH | 3-CF₃,5-SO₂CH₃ |
| A-315 | (Z)-N-OCH₃ | 3-CF₃,5-SO₂CH₃ |
| A-316 | (E)-N-OCH₃ | 3-CF₃,5-SO₂CH₃ |
| A-317 | (Z)-N-OC₂H₅ | 3-CF₃,5-SO₂CH₃ |
| A-318 | (E)-N-OC₂H₅ | 3-CF₃,5-SO₂CH₃ |
| A-319 | (Z)-N-OCH₂CF₃ | 3-CF₃,5-SO₂CH₃ |
| A-320 | (E)-N-OCH₂CF₃ | 3-CF₃,5-SO₂CH₃ |
| A-321 | (Z)-N-OCH₂C₆H₅ | 3-CF₃,5-SO₂CH₃ |
| A-322 | (E)-N-OCH₂C₆H₅ | 3-CF₃,5-SO₂CH₃ |
| A-323 | O | 3-CF₃,5-SO₂CF₃ |
| A-324 | NOH | 3-CF₃,5-SO₂CF₃ |
| A-325 | (Z)-NOH | 3-CF₃,5-SO₂CF₃ |
| A-326 | (E)-NOH | 3-CF₃,5-SO₂CF₃ |
| A-327 | (Z)-N-OCH₃ | 3-CF₃,5-SO₂CF₃ |
| A-328 | (E)-N-OCH₃ | 3-CF₃,5-SO₂CF₃ |
| A-329 | (Z)-N-OC₂H₅ | 3-CF₃,5-SO₂CF₃ |
| A-330 | (E)-N-OC₂H₅ | 3-CF₃,5-SO₂CF₃ |
| A-331 | (Z)-N-OCH₂CF₃ | 3-CF₃,5-SO₂CF₃ |
| A-332 | (E)-N-OCH₂CF₃ | 3-CF₃,5-SO₂CF₃ |
| A-333 | (Z)-N-OCH₂C₆H₅ | 3-CF₃,5-SO₂CF₃ |
| A-334 | (E)-N-OCH₂C₆H₅ | 3-CF₃,5-SO₂CF₃ |
| A-335 | O | 3-CF₃,5-OCF₃ |
| A-336 | NOH | 3-CF₃,5-OCF₃ |
| A-337 | (Z)-NOH | 3-CF₃,5-OCF₃ |
| A-338 | (E)-NOH | 3-CF₃,5-OCF₃ |
| A-339 | (Z)-N-OCH₃ | 3-CF₃,5-OCF₃ |
| A-340 | (E)-N-OCH₃ | 3-CF₃,5-OCF₃ |
| A-341 | (Z)-N-OC₂H₅ | 3-CF₃,5-OCF₃ |
| A-342 | (E)-N-OC₂H₅ | 3-CF₃,5-OCF₃ |
| A-343 | (Z)-N-OCH₂CF₃ | 3-CF₃,5-OCF₃ |
| A-344 | (E)-N-OCH₂CF₃ | 3-CF₃,5-OCF₃ |
| A-345 | (Z)-N-OCH₂C₆H₅ | 3-CF₃,5-OCF₃ |
| A-346 | (E)-N-OCH₂C₆H₅ | 3-CF₃,5-OCF₃ |
| A-347 | O | 3-CF₃,5-c-C₃H₅ |
| A-348 | NOH | 3-CF₃,5-c-C₃H₅ |
| A-349 | (Z)-NOH | 3-CF₃,5-c-C₃H₅ |
| A-350 | (E)-NOH | 3-CF₃,5-c-C₃H₅ |
| A-351 | (Z)-N-OCH₃ | 3-CF₃,5-c-C₃H₅ |
| A-352 | (E)-N-OCH₃ | 3-CF₃,5-c-C₃H₅ |
| A-353 | (Z)-N-OC₂H₅ | 3-CF₃,5-c-C₃H₅ |
| A-354 | (E)-N-OC₂H₅ | 3-CF₃,5-c-C₃H₅ |
| A-355 | (Z)-N-OCH₂CF₃ | 3-CF₃,5-c-C₃H₅ |
| A-356 | (E)-N-OCH₂CF₃ | 3-CF₃,5-c-C₃H₅ |
| A-357 | (Z)-N-OCH₂C₆H₅ | 3-CF₃,5-c-C₃H₅ |
| A-358 | (E)-N-OCH₂C₆H₅ | 3-CF₃,5-c-C₃H₅ |
| A-359 | O | 3-Cl, 5-[(1-CN)-c-C₃H₄] |
| A-360 | NOH | 3-Cl, 5-[(1-CN)-c-C₃H₄] |
| A-361 | (Z)-NOH | 3-Cl, 5-[(1-CN)-c-C₃H₄] |
| A-362 | (E)-NOH | 3-Cl, 5-[(1-CN)-c-C₃H₄] |
| A-363 | (Z)-N-OCH₃ | 3-Cl, 5-[(1-CN)-c-C₃H₄] |
| A-364 | (E)-N-OCH₃ | 3-Cl, 5-[(1-CN)-c-C₃H₄] |
| A-365 | (Z)-N-OC₂H₅ | 3-Cl, 5-[(1-CN)-c-C₃H₄] |
| A-366 | (E)-N-OC₂H₅ | 3-Cl, 5-[(1-CN)-c-C₃H₄] |
| A-367 | (Z)-N-OCH₂CF₃ | 3-Cl, 5-[(1-CN)-c-C₃H₄] |
| A-368 | (E)-N-OCH₂CF₃ | 3-Cl, 5-[(1-CN)-c-C₃H₄] |
| A-369 | (Z)-N-OCH₂C₆H₅ | 3-Cl, 5-[(1-CN)-c-C₃H₄] |
| A-370 | (E)-N-OCH₂C₆H₅ | 3-Cl, 5-[(1-CN)-c-C₃H₄] |
| A-371 | O | 3-CF₃, 5-[(1-CN)-c-C₃H₄] |
| A-372 | NOH | 3-CF₃, 5-[(1-CN)-c-C₃H₄] |
| A-373 | (Z)-NOH | 3-CF₃, 5-[(1-CN)-c-C₃H₄] |
| A-374 | (E)-NOH | 3-CF₃, 5-[(1-CN)-c-C₃H₄] |
| A-375 | (Z)-N-OCH₃ | 3-CF₃, 5-[(1-CN)-c-C₃H₄] |
| A-376 | (E)-N-OCH₃ | 3-CF₃, 5-[(1-CN)-c-C₃H₄] |
| A-377 | (Z)-N-OC₂H₅ | 3-CF₃, 5-[(1-CN)-c-C₃H₄] |
| A-378 | (E)-N-OC₂H₅ | 3-CF₃, 5-[(1-CN)-c-C₃H₄] |
| A-379 | (Z)-N-OCH₂CF₃ | 3-CF₃, 5-[(1-CN)-c-C₃H₄] |
| A-380 | (E)-N-OCH₂CF₃ | 3-CF₃, 5-[(1-CN)-c-C₃H₄] |
| A-381 | (Z)-N-OCH₂C₆H₅ | 3-CF₃, 5-[(1-CN)-c-C₃H₄] |
| A-382 | (E)-N-OCH₂C₆H₅ | 3-CF₃, 5-[(1-CN)-c-C₃H₄] |
| A-383 | O | 3-CF₃, 5-[(2,2-Cl₂)-c-C₃H₃] |
| A-384 | NOH | 3-CF₃, 5-[(2,2-Cl₂)-c-C₃H₃] |
| A-385 | (Z)-NOH | 3-CF₃, 5-[(2,2-Cl₂)-c-C₃H₃] |
| A-386 | (E)-NOH | 3-CF₃, 5-[(2,2-Cl₂)-c-C₃H₃] |
| A-387 | (Z)-N-OCH₃ | 3-CF₃, 5-[(2,2-Cl₂)-c-C₃H₃] |
| A-388 | (E)-N-OCH₃ | 3-CF₃, 5-[(2,2-Cl₂)-c-C₃H₃] |
| A-389 | (Z)-N-OC₂H₅ | 3-CF₃, 5-[(2,2-Cl₂)-c-C₃H₃] |
| A-390 | (E)-N-OC₂H₅ | 3-CF₃, 5-[(2,2-Cl₂)-c-C₃H₃] |
| A-391 | (Z)-N-OCH₂CF₃ | 3-CF₃, 5-[(2,2-Cl₂)-c-C₃H₃] |
| A-392 | (E)-N-OCH₂CF₃ | 3-CF₃, 5-[(2,2-Cl₂)-c-C₃H₃] |
| A-393 | (Z)-N-OCH₂C₆H₅ | 3-CF₃, 5-[(2,2-Cl₂)-c-C₃H₃] |
| A-394 | (E)-N-OCH₂C₆H₅ | 3-CF₃, 5-[(2,2-Cl₂)-c-C₃H₃] |
| A-395 | O | 3-OCF₃,5-c-C₃H₅ |
| A-396 | NOH | 3-OCF₃,5-c-C₃H₅ |
| A-397 | (Z)-NOH | 3-OCF₃,5-c-C₃H₅ |
| A-398 | (E)-NOH | 3-OCF₃,5-c-C₃H₅ |
| A-399 | (Z)-N-OCH₃ | 3-OCF₃,5-c-C₃H₅ |
| A-400 | (E)-N-OCH₃ | 3-OCF₃,5-c-C₃H₅ |
| A-401 | (Z)-N-OC₂H₅ | 3-OCF₃,5-c-C₃H₅ |
| A-402 | (E)-N-OC₂H₅ | 3-OCF₃,5-c-C₃H₅ |
| A-403 | (Z)-N-OCH₂CF₃ | 3-OCF₃,5-c-C₃H₃ |
| A-404 | (E)-N-OCH₂CF₃ | 3-OCF₃,5-C-C₃H₅ |
| A-405 | (Z)-N-OCH₂C₆H₅ | 3-OCF₃,5-c-C₃H₅ |
| A-406 | (E)-N-OCH₂C₆H₅ | 3-OCF₃,5-C-C₃H₅ |
| A-407 | O | 3,5-SO₂CH₃ |
| A-408 | NOH | 3,5-SO₂CH₃ |
| A-409 | (Z)-NOH | 3,5-SO₂CH₃ |
| A-410 | (E)-NOH | 3,5-SO₂CH₃ |
| A-411 | (Z)-N-OCH₃ | 3,5-SO₂CH₃ |
| A-412 | (E)-N-OCH₃ | 3,5-SO₂CH₃ |
| A-413 | (Z)-N-OC₂H₅ | 3,5-SO₂CH₃ |
| A-414 | (E)-N-OC₂H₅ | 3,5-SO₂CH₃ |
| A-415 | (Z)-N-OCH₂CF₃ | 3,5-SO₂CH₃ |
| A-416 | (E)-N-OCH₂CF₃ | 3,5-SO₂CH₃ |
| A-417 | (Z)-N-OCH₂C₆H₅ | 3,5-SO₂CH₃ |
| A-418 | (E)-N-OCH₂C₆H₅ | 3,5-SO₂CH₃ |
| A-419 | O | 3,5-SO₂CF₃ |
| A-420 | NOH | 3,5-SO₂CF₃ |
| A-421 | (Z)-NOH | 3,5-SO₂CF₃ |
| A-422 | (E)-NOH | 3,5-SO₂CF₃ |
| A-423 | (Z)-N-OCH₃ | 3,5-SO₂CF₃ |
| A-424 | (E)-N-OCH₃ | 3,5-SO₂CF₃ |
| A-425 | (Z)-N-OC₂H₅ | 3,5-SO₂CF₃ |
| A-426 | (E)-N-OC₂H₅ | 3,5-SO₂CF₃ |
| A-427 | (Z)-N-OCH₂CF₃ | 3,5-SO₂CF₃ |
| A-428 | (E)-N-OCH₂CF₃ | 3,5-SO₂CF₃ |
| A-429 | (Z)-N-OCH₂C₆H₅ | 3,5-SO₂CF₃ |
| A-430 | (E)-N-OCH₂C₆H₅ | 3,5-SO₂CF₃ |
| A-431 | O | 3-CI, 5-[SO₂-(4-F-C₆H₄)] |
| A-432 | NOH | 3-Cl, 5-[SO₂-(4-F-C₆H₄)] |
| A-433 | (Z)-NOH | 3-Cl, 5-[SO₂-(4-F-C₆H₄)] |
| A-434 | (E)-NOH | 3-Cl, 5-[SO₂-(4-F-C₆H₄)] |
| A-435 | (Z)-N-OCH₃ | 3-CI, 5-[SO₂-(4-F-C₆H₄)] |
| A-436 | (E)-N-OCH₃ | 3-Cl, 5-[SO₂-(4-F-C₆H₄)] |
| A-437 | (Z)-N-OC₂H₅ | 3-Cl, 5-[SO₂-(4-F-C₆H₄)] |
| A-438 | (E)-N-OC₂H₅ | 3-Cl, 5-[SO₂-(4-F-C₆H₄)] |
| A-439 | (Z)-N-OCH₂CF₃ | 3-Cl, 5-[SO₂-(4-F-C₆H₄)] |
| A-440 | (E)-N-OCH₂CF₃ | 3-Cl, 5-[SO₂-(4-F-C₆H₄)] |
| A-441 | (Z)-N-OCH₂C₆H₅ | 3-Cl, 5-[SO₂-(4-F-C₆H₄)] |
| A-442 | (E)-N-OCH₂C₆H₅ | 3-Cl, 5-[SO₂-(4-F-C₆H₄)] |
| A-443 | O | 3-Br, 5-[SO₂-(4-F-C₆H₄)] |
| A-444 | NOH | 3-Br, 5-[SO₂-(4-F-C₆H₄)] |
| A-445 | (Z)-NOH | 3-Br, 5-[SO₂-(4-F-C₆H₄)] |
| A-446 | (E)-NOH | 3-Br, 5-[SO₂-(4-F-C₆H₄)] |
| A-447 | (Z)-N-OCH₃ | 3-Br, 5-[SO₂-(4-F-C₆H₄)] |
| A-448 | (E)-N-OCH₃ | 3-Br, 5-[SO₂-(4-F-C₆H₄)] |
| A-449 | (Z)-N-OC₂H₅ | 3-Br, 5-[SO₂-(4-F-C₆H₄)] |
| A-450 | (E)-N-OC₂H₅ | 3-Br, 5-[SO₂-(4-F-C₆H₄)] |
| A-451 | (Z)-N-OCH₂CF₃ | 3-Br, 5-[SO₂-(4-F-C₆H₄)] |
| A-452 | (E)-N-OCH₂CF₃ | 3-Br, 5-[SO₂-(4-F-C₆H₄)] |
| A-453 | (Z)-N-OCH₂C₆H₅ | 3-Br, 5-[SO₂-(4-F-C₆H₄)] |
| A-454 | (E)-N-OCH₂C₆H₅ | 3-Br, 5-[SO₂-(4-F-C₆H₄)] |
| A-455 | O | 3-CF₃, 5-[SO₂-(4-F-C₆H₄)] |
| A-456 | NOH | 3-CF₃, 5-[SO₂-(4-F-C₆H₄)] |
| A-457 | (Z)-NOH | 3-CF₃, 5-[SO₂-(4-F-C₆H₄)] |
| A-458 | (E)-NOH | 3-CF₃, 5-[SO₂-(4-F-C₆H₄)] |
| A-459 | (Z)-N-OCH₃ | 3-CF₃, 5-[SO₂-(4-F-C₆H₄)] |
| A-460 | (E)-N-OCH₃ | 3-CF₃, 5-[SO₂-(4-F-C₆H₄)] |
| A-461 | (Z)-N-OC₂H₅ | 3-CF₃, 5-[SO₂-(4-F-C₆H₄)] |
| A-462 | (E)-N-OC₂H₅ | 3-CF₃, 5-[SO₂-(4-F-C₆H₄)] |
| A-463 | (Z)-N-OCH₂CF₃ | 3-CF₃, 5-[SO₂-(4-F-C₆H₄)] |
| A-464 | (E)-N-OCH₂CF₃ | 3-CF₃, 5-[SO₂-(4-F-C₆H₄)] |
| A-465 | (Z)-N-OCH₂C₆H₅ | 3-CF₃, 5-[SO₂-(4-F-C₆H₄)] |
| A-466 | (E)-N-OCH₂C₆H₅ | 3-CF₃, 5-[SO₂-(4-F-C₆H₄)] |
| A-467 | O | 3-Cl,5-OC₆H₅ |
| A-468 | NOH | 3-Cl,5-OC₆H₅ |
| A-469 | (Z)-NOH | 3-Cl,5-OC₆H₅ |
| A-470 | (E)-NOH | 3-Cl,5-OC₆H₅ |
| A-471 | (Z)-N-OCH₃ | 3-Cl,5-OC₆H₅ |
| A-472 | (E)-N-OCH₃ | 3-Cl,5-OC₆H₅ |
| A-473 | (Z)-N-OC₂H₅ | 3-Cl,5-OC₆H₅ |
| A-474 | (E)-N-OC₂H₅ | 3-Cl,5-OC₆H₅ |
| A-475 | (Z)-N-OCH₂CF₃ | 3-Cl,5-OC₆H₅ |
| A-476 | (E)-N-OCH₂CF₃ | 3-Cl,5-OC₆H₅ |
| A-477 | (Z)-N-OCH₂C₆H₅ | 3-Cl,5-OC₆H₅ |
| A-478 | (E)-N-OCH₂C₆H₅ | 3-Cl,5-OC₆H₅ |
| A-479 | O | 3-CF₃,5-OC₆H₅ |
| A-480 | NOH | 3-CF₃,5-OC₆H₅ |
| A-481 | (Z)-NOH | 3-CF₃,5-OC₆H₅ |
| A-482 | (E)-NOH | 3-CF₃,5-OC₆H₅ |
| A-483 | (Z)-N-OCH₃ | 3-CF₃,5-OC₆H₅ |
| A-484 | (E)-N-OCH₃ | 3-CF₃,5-OC₆H₅ |
| A-485 | (Z)-N-OC₂H₅ | 3-CF₃,5-OC₆H₅ |
| A-486 | (E)-N-OC₂H₅ | 3-CF₃,5-OC₆H₅ |
| A-487 | (Z)-N-OCH₂CF₃ | 3-CF₃,5-OC₆H₅ |
| A-488 | (E)-N-OCH₂CF₃ | 3-CF₃,5-OC₆H₅ |
| A-489 | (Z)-N-OCH₂C₆H₅ | 3-CF₃,5-OC₆H₅ |
| A-490 | (E)-N-OCH₂C₆H₅ | 3-CF₃,5-OC₆H₅ |
| A-491 | O | 3-CF₃, 5-[O-(4-Cl-C₆H₄)] |
| A-492 | NOH | 3-CF₃, 5-[O-(4-Cl-C₆H₄)] |
| A-493 | (Z)-NOH | 3-CF₃, 5-[O-(4-Cl-C₆H₄)] |
| A-494 | (E)-NOH | 3-CF₃, 5-[O-(4-Cl-C₆H₄)] |
| A-495 | (Z)-N-OCH₃ | 3-CF₃, 5-[O-(4-Cl-C₆H₄)] |
| A-496 | (E)-N-OCH₃ | 3-CF₃, 5-[O-(4-Cl-C₆H₄)] |
| A-497 | (Z)-N-OC₂H₅ | 3-CF₃, 5-[O-(4-Cl-C₆H₄)] |
| A-498 | (E)-N-OC₂H₅ | 3-CF₃, 5-[O-(4-Cl-C₆H₄)] |
| A-499 | (Z)-N-OCH₂CF₃ | 3-CF₃, 5-[O-(4-Cl-C₆H₄)] |
| A-500 | (E)-N-OCH₂CF₃ | 3-CF₃, 5-[O-(4-Cl-C₆H₄)] |
| A-501 | (Z)-N-OCH₂C₆H₅ | 3-CF₃, 5-[O-(4-Cl-C₆H₄)] |
| A-502 | (E)-N-OCH₂C₆H₅ | 3-CF₃, 5-[O-(4-Cl-C₆H₄)] |

The term "compound(s) of the invention" refers to compound(s) of formula I, or "compound(s) I", and includes their salts, tautomers, stereoisomers, and N-oxides.

The invention also relates to agrochemical compositions comprising an auxiliary and at least one compound I.

An agrochemical composition comprises a pesticidally effective amount of a compound I.

An agrochemical composition comprises a pesticidally effective amount of a compound I.

The compounds I can be converted into customary types of agro-chemical compositions, e.g. solutions, emulsions, suspensions, dusts, powders, pastes, granules, pressings, capsules, and mixtures thereof. Examples for composition types are suspensions (e.g. SC, OD, FS), emulsifiable concentrates (e.g. EC), emulsions (e.g. EW, EO, ES, ME), capsules (e.g. CS, ZC), pastes, pastilles, wettable powders or dusts (e.g. WP, SP, WS, DP, DS), pressings (e.g. BR, TB, DT), granules (e.g. WG, SG, GR, FG, GG, MG), insecticidal articles (e.g. LN), as well as gel formulations for the treatment of plant propagation materials e.g. seeds (e.g. GF). These and further compositions types are defined in the "Catalogue of pesticide formulation types and international coding system", Technical Monograph No. 2, 6th Ed. May 2008, CropLife International.

Suitable auxiliaries are solvents, liquid carriers, solid carriers or fillers, surfactants, dispersants, emulsifiers, wetters, adjuvants, solubilizers, penetration enhancers, protective colloids, adhesion agents, thickeners, humectants, repellents, attractants, feeding stimulants, compatibilizers, bactericides, anti-freezing agents, anti-foaming agents, colorants, tackifiers and binders.

Suitable solvents and liquid carriers are water and organic solvents. Suitable solid carriers or fillers are mineral earths.

Suitable surfactants are surface-active compounds, e.g. anionic, cationic, nonionic, and amphoteric surfactants, block polymers, polyelectrolytes. Such surfactants can be used as emulsifier, dispersant, solubilizer, wetter, penetration enhancer, protective colloid, or adjuvant. Surfactants are listed in McCutcheon's, Vol.1: Emulsifiers & Detergents, McCutcheon's Directories, Glen Rock, USA, 2008 (International or North American Ed.). Suitable anionic surfactants are alkali, alkaline earth, or ammonium salts of sulfonates, sulfates, phosphates, carboxylates. Suitable nonionic surfactants are alkoxylates, N-subsituted fatty acid amides, amine oxides, esters, sugar-based surfactants, polymeric surfactants. Suitable cationic surfactants are quaternary surfactants.

The agrochemical compositions generally comprise between 0.01 and 95%, preferably between 0.1 and 90%, and most preferably between 0.5 and 75%, by weight of active substance. The active substances are employed in a purity of from 90% to 100%, preferably from 95% to 100%.

Various types of oils, wetters, adjuvants, or fertilizer may be added to the active substances or the compositions comprising them as premix or, if appropriate not until immediately prior to use (tank mix). These agents can be admixed with the compositions according to the invention in a weight ratio of 1:100 to 100:1.

The user applies the composition according to the invention usually from a predosage device, a knapsack sprayer, a spray tank, a spray plane, or an irrigation system. Usually, the agrochemical composition is made up with water, buffer, and/or further auxiliaries to the desired application concentration and the ready-to-use spray liquor or the agrochemical composition according to the invention is thus obtained. Usually, 20 to 2000 liters of the ready-to-use spray liquor are applied per hectare of agricultural useful area.

The compounds I are suitable for use in protecting crops, plants, plant propagation materials, e.g. seeds, or soil or water, in which the plants are growing, from attack or infestation by animal pests. Therefore, the invention also relates to a plant protection method, which comprises contacting crops, plants, plant propagation materials, e.g. seeds, or soil or water, in which the plants are growing, to be protected from attack or infestation by animal pests, with a pesticidally effective amount of a compound I.

The compounds I are also suitable for use in combating or controlling animal pests. Therefore, disclosed is a method of combating or controlling animal pests, which comprises contacting the animal pests, their habitat, breeding ground, or food supply, or the crops, plants, plant propagation materials, e.g. seeds, or soil, or the area, material or environment in which the animal pests are growing or may grow, with a pesticidally effective amount of a compound I.

The compounds I are effective through both contact and ingestion to any and all developmental stages, such as egg, larva, pupa, and adult.

The compounds I can be applied as such or in form of compositions comprising them.

The application can be carried out both before and after the infestation of the crops, plants, plant propagation materials by the pests.

The term "contacting" includes both direct contact (applying the compounds/compositions directly on the animal pest or plant) and indirect contact (applying the compounds/compositions to the locus).

The term "animal pest" includes arthropods, gastropods, and nematodes. Preferred animal pests according to the invention are arthropods, preferably insects and arachnids, in particular insects.

The term "plant" includes cereals, e.g. durum and other wheat, rye, barley, triticale, oats, rice, or maize (fodder maize and sugar maize / sweet and field corn); beet, e.g. sugar beet, or fodder beet; fruits, e.g. pomes, stone fruits, or soft fruits, e.g. apples, pears, plums, peaches, nectarines, almonds, cherries, papayas, strawberries, raspberries, blackberries or gooseberries; leguminous plants, e.g. beans, lentils, peas, alfalfa, or soybeans; oil plants, e.g. rapeseed (oilseed rape), turnip rape, mustard, olives, sunflowers, coconut, cocoa beans, castor oil plants, oil palms, ground nuts, or soybeans; cucurbits, e.g. squashes, pumpkins, cucumber or melons; fiber plants, e.g. cotton, flax, hemp, or jute; citrus fruit, e.g. oranges, lemons, grapefruits or mandarins; vegetables, e.g. eggplant, spinach, lettuce (e.g. iceberg lettuce), chicory, cabbage, asparagus, cabbages, carrots, onions, garlic, leeks, tomatoes, potatoes, cucurbits or sweet peppers; lauraceous plants, e.g. avocados, cinnamon, or camphor; energy and raw material plants, e.g. corn, soybean, rapeseed, sugar cane or oil palm; tobacco; nuts, e.g. walnuts; pistachios; coffee; tea; bananas; vines; hop; sweet leaf (Stevia); natural rubber plants or ornamental and forestry plants, shrubs, broad-leaved trees or evergreens, eucalyptus; turf; lawn; grass. Preferred plants include potatoes, sugar beets, tobacco, wheat, rye, barley, oats, rice, corn, cotton, soybeans, rapeseed, legumes, sunflowers, coffee, or sugar cane; fruits; vines; ornamentals; or vegetables, e.g. cucumbers, tomatoes, beans or squashes.

The term "seed" embraces seeds and plant propagules including true seeds, seed pieces, suckers, corms, bulbs, fruit, tubers, grains, cuttings, cut shoots, and means preferably true seeds.

"Pesticidally effective amount" means the amount of active ingredient needed to achieve an observable effect on growth, including the effects of necrosis, death, retardation, prevention, and removal, destruction, or otherwise diminishing the occurrence and activity of the target organism. The pesticidally effective amount can vary for the various compounds/compositions used in the invention. A pesticidally effective amount of the compositions will also vary according to the prevailing conditions e.g. desired pesticidal effect and duration, weather, target species, locus, mode of application.

For use in treating crop plants, e.g. by foliar application, the rate of application of the active ingredients of this invention may be in the range of 0.0001 g to 4000 g per hectare, e.g. from 1 g to 2 kg per hectare or from 1 g to 750 g per hectare, desirably from 1 g to 100 g per hectare.

The compounds I are also suitable for use against non-crop insect pests. For use against said non-crop pests, compounds I can be used as bait composition, gel, general insect spray, aerosol, as ultra-low volume application and bed net (impregnated or surface applied).

The term "non-crop insect pest" refers to pests, which are particularly relevant for non-crop targets, e.g. ants, termites, wasps, flies, fleas, ticks, mosquitoes, bed bugs, crickets, or cockroaches, such as: *Aedes aegypti, Musca domestica, Tribolium spp.;* termites such as *Reticulitermes flavipes, Coptotermes formosanus;* roaches such as *Blatella germanica, Periplaneta Americana;* ants such as *Solenopsis invicta, Linepithema humile,* and *Camponotus pennsylvanicus;* stored product pests such as *Tribolium confusum, Plodia interpunctella;* turf pests such as *Popillia japonica, Blissus leucopterus, Neocapteriscus vicinus.*

The bait can be a liquid, a solid or a semisolid preparation (e.g. a gel). For use in bait compositions, the typical content of active ingredient is from 0.001 wt% to 15 wt%, desirably from 0.001 wt% to 5 wt% of active compound.

The compounds I and its compositions can be used for protecting wooden materials such as trees, board fences, sleepers, frames, artistic artifacts, etc. and buildings, but also construction materials, furniture, leathers, fibers, vinyl articles, electric wires and cables etc. from ants, termites and/or wood or textile destroying beetles, and for controlling ants and termites from doing harm to crops or human beings (e.g. when the pests invade into houses and public facilities or nest in yards, orchards or parks).

Customary application rates in the protection of materials are, e.g., from 0.001 g to 2000 g or from 0.01 g to 1000 g of active compound per m² treated material, desirably from 0.1 g to 50 g per m².

Insecticidal compositions for use in the impregnation of materials typically contain from 0.001 to 95 wt%, preferably from 0.1 to 45 wt%, and more preferably from 1 to 25 wt% of at least one repellent and/or insecticide.

The compounds of the invention are especially suitable for efficiently combating animal pests e.g. arthropods, and nematodes including:
insects from the sub-order of Auchenorrhyncha, e.g. *Amrasca biguttula, Empoasca* spp., *Ne-photettix virescens, Sogatella furcifera, Mahanarva* spp., *Laodelphax striatellus, Nilaparvata lugens, Diaphorina citri;*
Lepidoptera, e.g. *Helicoverpa* spp., *Heliothis virescens, Lobesia botrana, Ostrinia nubilalis, Plu-tella xylostella, Pseudoplusia includens, Scirpophaga incertulas, Spodoptera* spp., *Trichoplusia ni, Tuta absoluta, Cnaphalocrocis medialis, Cydia pomonella, Chilo suppressalis, Anticarsia gemmatalis, Agrotis ipsilon, Chrysodeixis includens;*
True bugs, e.g. Lygus spp., Stink bugs such as *Euschistus* spp., *Halyomorpha halys, Nezara viridula, Piezodorus guildinii, Dichelops furcatus;*
Thrips, e.g. *Frankliniella* spp., *Thrips* spp., *Dichromothrips corbettii;*
Aphids, e.g. *Acyrthosiphon pisum, Aphis* spp., *Myzus persicae, Rhopalosiphum* spp., *Schizaphis graminum, Megoura viciae;*
Whiteflies, e.g. *Trialeurodes vaporariorum, Bemisia* spp.;
Coleoptera, e.g. *Phyllotreta* spp., *Melanotus* spp., *Meligethes aeneus, Leptinotarsa decimlineata, Ceutorhynchus* spp., *Diabrotica* spp., *Anthonomus grandis, Atomaria linearia, Agriotes* spp., *Epilachna* spp.;
Flies, e.g. Delia spp., *Ceratitis capitate, Bactrocera* spp., *Liriomyza* spp., *Musca domestica;*
Mosquitoes (Diptera), e.g. *Aedes aegypti, A. albopictus, A. vexans, Anastrepha ludens, Anopheles maculipennis, A. crucians, A. albimanus, A. gambiae, A. freeborni, A. leucosphyrus, A. minimus, A. quadrimaculatus;*
Coccoidea, e.g. *Aonidiella aurantia, Ferrisia virgate;*
Anthropods of class Arachnida (Mites), e.g. *Penthaleus major, Tetranychus* spp.;

Nematodes, e.g. *Heterodera glycines, Meloidogyne sp., Pratylenchus spp., Caenorhabditis elegans.*

The compounds I are suitable for use in treating or protecting animals against infestation or infection by parasites. Therefore, the invention relates to a compound I for use in treating or protecting animals against infestation and infection by parasites, the use comprising orally, topically or parenterally administering or applying to the animals a parasiticidally effective amount of a compound I.

The invention also relates to the non-therapeutic use of compounds of the invention for treating or protecting animals against infestation and infection by parasites. Moreover, the invention relates to a non-therapeutic method of treating or protecting animals against infestation and infection by parasites, which comprises applying to a locus a parasiticidally effective amount of a compound I.

The compounds of the invention are further suitable for use in combating or controlling parasites in and on animals. Furthermore, described is a method of combating or controlling parasites in and on animals, which comprises contacting the parasites with a parasitically effective amount of a compound I.

The invention also relates to the non-therapeutic use of compounds I for controlling or combating parasites. Moreover, the invention relates to a non-therapeutic method of combating or controlling parasites, which comprises applying to a locus a parasiticidally effective amount of a compound I.

The compounds I can be effective through both contact (via soil, glass, wall, bed net, carpet, blankets, or animal parts) and ingestion (e.g. baits). Furthermore, the compounds I can be applied to any and all developmental stages.

The compounds I can be applied as such or in form of compositions comprising them.

The term "locus" means the habitat, food supply, breeding ground, area, material or environment in which a parasite is growing or may grow outside of the animal.

As used herein, the term "parasites" includes endo- and ectoparasites. In some embodiments of the invention, endoparasites can be preferred. In other embodiments, ectoparasites can be preferred. Infestations in warm-blooded animals and fish include lice, biting lice, ticks, nasal bots, keds, biting flies, muscoid flies, flies, myiasitic fly larvae, chiggers, gnats, mosquitoes and fleas.

The compounds of the invention are especially useful for combating the following parasites: *Cimex lectularius, Rhipicephalus sanguineus,* and *Ctenocephalides felis.*

As used herein, the term "animal" includes warm-blooded animals (including humans) and fish. Preferred are mammals, such as cattle, sheep, swine, camels, deer, horses, pigs, poultry, rabbits, goats, dogs and cats, water buffalo, donkeys, fallow deer and reindeer, and also in furbearing animals such as mink, chinchilla and raccoon, birds such as hens, geese, turkeys and ducks and fish such as fresh- and salt-water fish such as trout, carp and eels. Particularly preferred are domestic animals, such as dogs or cats.

The compounds I may be applied in total amounts of 0.5 mg/kg to 100 mg/kg per day, preferably 1 mg/kg to 50 mg/kg per day.

For oral administration to warm-blooded animals, the compounds I may be formulated as animal feeds, animal feed premixes, animal feed concentrates, pills, solutions, pastes, suspensions, drenches, gels, tablets, boluses and capsules. For oral administration, the dosage form chosen should provide the animal with 0.01 mg/kg to 100 mg/kg of animal body weight per day of the compounds I, preferably with 0.5 mg/kg to 100 mg/kg of animal body weight per day. Alternatively, the compounds I may be administered to animals parenterally, e.g., by intraruminal, intramuscular, intravenous or subcutaneous injection. The compounds I may be dispersed or dissolved in a physiologically acceptable carrier for subcutaneous injection. Alternatively, the compounds I may be formulated into an implant for subcutaneous administration. In addition, the compounds I may be transdermally administered to animals. For parenteral administration, the dosage form chosen should provide the animal with 0.01 mg/kg to 100 mg/kg of animal body weight per day of the compounds I.

The compounds I may also be applied topically to the animals in the form of dips, dusts, powders, collars, medallions, sprays, shampoos, spot-on and pour-on formulations and in ointments or oil-in-water or water-in-oil emulsions. For topical application, dips and sprays usually contain 0.5 ppm to 5,000 ppm and preferably 1 ppm to 3,000 ppm of the compounds I. In addition, the compounds I may be formulated as ear tags for animals, particularly quadrupeds e.g. cattle and sheep.

Oral solutions are administered directly.

Solutions for use on the skin are trickled on, spread on, rubbed in, sprinkled on or sprayed on.

Gels are applied to or spread on the skin or introduced into body cavities.

Pour-on formulations are poured or sprayed onto limited areas of the skin, the active compound penetrating the skin and acting systemically. Pour-on formulations are prepared by dissolving, suspending, or emulsifying the active compound in suitable skin-compatible solvents or solvent mixtures.

Emulsions can be administered orally, dermally or as injections.

Suspensions can be administered orally or topically/dermally.

Semi-solid preparations can be administered orally or topically/dermally.

For the production of solid preparations, the active compound is mixed with suitable excipients, if appropriate with addition of auxiliaries, and brought into the desired form.

The compositions which can be used in the invention can comprise generally from about 0.001 to 95% of the compound I.

Ready-to-use preparations contain the compounds acting against parasites, preferably ectoparasites, in concentrations of 10 ppm to 80% by weight, preferably from 0.1 to 65% by weight, more preferably from 1 to 50% by weight, most preferably from 5 to 40% by weight.

Preparations which are diluted before use contain the compounds acting against ectoparasites in concentrations of 0.5 to 90% by weight, preferably of 1 to 50% by weight.

Furthermore, the preparations comprise the compounds of formula I against endoparasites in concentrations of 10 ppm to 2% by weight, preferably of 0.05 to 0.9% by weight, very particularly preferably of 0.005 to 0.25% by weight.

Solid formulations which release compounds of the invention may be applied in total amounts of 10 mg/kg to 300 mg/kg, preferably 20 mg/kg to 200 mg/kg, most preferably 25 mg/kg to 160 mg/kg body weight of the treated animal in the course of three weeks.

### A. Preparation examples

The compounds were characterized by melting point determination, by NMR spectroscopy or by the mass-to-charge ratio ([m/z]) and retention time (RT; [min.]), as determined by mass spectrometry (MS) coupled with HPLC analysis (HPLC-MS = high performance liquid chromatography-coupled mass spectrometry) or LC analysis (LC-MS = liquid chromatography-coupled mass spectrometry).

Method A: HPLC: Shimadzu Nexera UHPLC + Shimadzu LCMS-2020, ESI; Column: Phenomenex Kinetex 1.7µm XB-C18 100A, 2.1x50mm; Mobile phase: A: water + 0.1% TFA; B: ACN; Temperature: 60°C; Gradient: 5% B to 100% B in 1.5 min; 100% B 0.25 min; Flow: 0.8 mL/min to 1.0 mL/min in 1.51 min; MS: ESI positive; Mass range (m/z): 100-700.

Method B: LC: Shimadzu LC-30AD, ESI; Column: Kinetex EVO C18.5µm 2.1x30mm; Mobile phase: A: water + 0.04% TFA; B: ACN + 0.02% TFA; Temperature: 40°C; Gradient: 5% B to 100% B in 2.5 min; 100% B to 5% B in 0.02min; 5% B for 0.5min; Flow: 0.8mL/min; MS: ESI positive; Mass range: 100-2000.

### Example 1 - Preparation of N-[1-[2-[N-hydroxy-C-methyl-carbonimidoyl]-1,2,4-triazol-3-yl]ethyl]-3,5-bis(trifluoromethyl)benzamide (I.1-1 and I.1-76)

### Step 1: Preparation of N-(2-amino-1-methyl-2-oxo-ethyl)-3,5-bis(trifluoromethyl)benzamide

To a solution of 3,5-bis(trifluoromethyl)benzoic acid (5g, 19.37mmol) in MeCN (50mL) was added Chloro-*N*,*N*,*N'*,*N'*-tetramethylformamidinium-hexafluorophosphate (8.15g, 29.05mmol), *N-*methylimidazole (6.36g, 77.48mmol), and 2-aminopropanamide (2.41g, 19.37mmol) at 20°C and the mixture stirred at 20°C for 16h until completion was determined by thin layer chromatographs (TLC; PE: EtOAc=1:1, Rf=0.5). The mixture was poured into H₂O (30mL) and extracted with ethyl acetate (EtOAc; 3x30mL). The organic layer was washed with brine (30mL), dried over Na₂SO₄, filtered, and concentrated. The crude product was triturated with H₂O (50mL) and filtered to give *N*-(2-amino-1-methyl-2-oxo-ethyl)-3,5-bis(trifluoromethyl)benzamide (5.3g, 79% yield) as a white solid.

¹H-NMR (400MHz, DMSO-d6): *δ* = 9.06 (d, J=7.4Hz, 1H), 8.56 (s, 2H), 8.32 (s, 1H), 7.49 (br s, 1H), 7.04 (br s, 1H), 4.44 (dq, J=7.2 Hz, J=7.4Hz, 1H), 1.36 (d, J=7.3Hz, 3H).

### Step 2: Preparation of N-[2-[dimethylaminomethyleneamino]-1-methyl-2-oxo-ethyl]-3,5-bis(trifluoromethyl)benzamide

To a solution of *N*-(2-amino-1-methyl-2-oxo-ethyl)-3,5-bis(trifluoromethyl)benzamide (1g, 3.1mmol) in DCM (10mL) was added DMF-DMA (730mg, 6.2mmol) at 30°C. The mixture was stirred at 50°C for 2h until completion was determined by TLC (PE:EtOAc=1:1, Rf=0.5) and LCMS. The mixture was concentrated to give *N*-[2-[dimethylaminomethyleneamino]-1-methyl-2-oxo-ethyl]-3,5-bis(trifluoromethyl)benzamide (1.2g, crude) as yellow oil. The crude product was directly employed in the next step.

### Step 3: Preparation of N-[1-(1H-1,2,4-triazol-5-yl)ethyl]-3,5-bis(trifluoromethyl)benzamide

To a solution of *N*-[2-[dimethylaminomethyleneamino]-1-methyl-2-oxo-ethyl]-3,5-bis(trifluoromethyl)benzamide (19.4g, 50.6mmol) in AcOH (10mL) was added N₂H₄xH₂O (5.1g, 101.2mmol) and the resulting mixture stirred at 20°C for 5min, then at 90°C for 2h until completion was determined by TLC (PE:EtOAc=1:1, Rf=0.6) and LCMS. The reaction mixture was concentrated. The residue was dissolved with EtOAc (5mL), poured into aq. sat. NaHCO₃ (30mL) and the mixture extracted with EtOAc (3x10mL). The organic layer was washed with brine (10mL), dried over Na₂SO₄, filtered and concentrated to give the crude product, which was triturated with DCM (15mL) and filtered to deliver *N*-[1-(1*H*-1,2,4-triazol-5-yl)ethyl]-3,5-bis(trifluoromethyl)benzamide (14g, 78% yield) as yellow solid.

¹H-NMR (400MHz, DMSO-d6): *δ* = 13.92-13.82 (m, 1H), 9.50-9.35 (m, 1H), 8.56 (br s, 2H), 8.33 (br d, J = 9.9Hz, 1H), 7.89 (s, 1H), 5.33 (dq, J = 6.9Hz, 1H), 1.57 (dd, J = 7.1, 13.3Hz, 3H).

### Step 4: Preparation of N-hydroxyacetimidoyl chloride

To a solution of acetaldehyde oxime (200 mg, 3.39 mmol) in DMF (2mL) was added *N-*chlorosuccinimide (497.3mg, 3.72mmol) at 0°C under N₂ atmosphere and the resulting mixture allowed to warm up to 20°C within 10min, at which time reaction completion was determined by TLC (PE:EtOAc=1:1, Rf=0.5). This DMF solution of *N*-hydroxyacetimidoyl chloride (3mL) was directly employed in the next step.

### Step 5: Preparation of N-[1-[2-[N-hydroxy-C-methyl-carbonimidoyl]-1,2,4-triazol-3-yl]ethyl]-3,5-bis(trifluoromethyl)benzamide (I.1-1 and I.1-76)

To a mixture of *N*-[1-(1*H*-1,2,4-triazol-5-yl)ethyl]-3,5-bis(trifluoromethyl)benzamide (715mg, 2.03mmol) and Cs₂CO₃ (1.7g, 5.08mmol) in DMF (7mL) was added *N*-hydroxyacetimidoyl chloride (3mL DMF solution) at 0°C under N₂ atmosphere. The reaction mixture was stirred at 20°C for 16h, until completion was determined by LCMS. The mixture was filtered, the filtrate was concentrated, and the residue was purified by preparative HPLC (TFA) to deliver *N*-[1-[2-[(*Z*)-*N-*hydroxy-*C*-methyl-carbonimidoyl]-1,2,4-triazol-3-yl]ethyl]-3,5-bis(trifluoromethyl)benzamide (I.1-1, 80 mg, 9.8% yield) and *N*-[1-[2-[(*E*)-*N*-hydroxy-C-methyl-carbonimidoyl]-1,2,4-triazol-3-yl]ethyl]-3,5-bis(trifluoromethyl)benzamide (I.1-76, 150mg, 18% yield) as white solids.

I.1-1: ¹H-NMR (400MHz, DMSO-d6): *δ* = 11.65 (s, 1H), 9.50 (d, J = 6.9Hz, 1H), 8.51 (s, 2H), 8.33 (s, 1H), 8.09 (s, 1H), 5.18 (dq, J = 6.9Hz, J = 6.9Hz, 1H), 2.27 (s, 3H), 1.61 (d, J = 6.9Hz, 3H).

I.1-76: ¹H-NMR (400MHz, DMSO-d6): *δ* = 11.60 (s, 1H), 9.57 (d, J = 7.2Hz, 1H), 8.53 (s, 2H), 8.33 (s, 1H), 8.06 (s, 1H), 5.63 - 5.55 (m, 1H), 2.33 (s, 3H), 1.59 (d, J = 6.9Hz, 3H).

### Example 2 - Preparation of N-[1-[2-[(Z)-N-methoxy-C-methyl-carbonimidoyl]-1,2,4-triazol-3-yl]ethyl]-3,5-bis(trifluoromethyl)benzamide (I.1-2)

To a solution of *N*-[1-[2-[*N*-hydroxy-*C*-methyl-carbonimidoyl]-1,2,4-triazol-3-yl]ethyl]-3,5-bis(trifluoromethyl)benzamide (140mg, 0.34mmol) in DMF (2mL) were added and K₂CO₃ (94.55mg, 0.68mmol) and CH₃I (97.1mg, 0.68mmol) at 20°C. The reaction mixture was stirred at 20°C for 16h, until completion was determined by LCMS. The mixture was filtered, the filtrate was concentrated, and the residue was purified by preparative HPLC (TFA) to deliver *N*-[1-[2-[(*Z*)-*N-*methoxy-*C*-methyl-carbonimidoyl]-1,2,4-triazol-3-yl]ethyl]-3,5-bis(trifluoromethyl)benzamide (I.1-2, 163 mg, 56% yield) as a yellow solid.

¹H-NMR (400MHz, DMSO-d6): *δ* = 9.54 (d, J = 6.8Hz, 1H), 8.51 (s, 2H), 8.34 (s, 1H), 8.13 (s, 1H), 5.13 (dq, J = 6.9Hz, J = 6.8Hz, 1H), 3.81 (s, 3H), 2.29 (s, 3H), 1.61 (d, J = 7.0Hz, 3H).

¹³C-NMR (125.7MHz, CDCl₃): *δ* = 163.62, 157.56, 151.42, 141.97, 135.37, 132.17, 132.17, 127.36, 127.36, 125.29, 122.74, 122.74, 62.65, 42.87, 19.65, 18.90.

### Example 3 - Preparation of N-[1-[2-[(E)-N-methoxy-C-methyl-carbonimidoyl]-1,2,4-triazol-3-yl]ethyl]-3,5-bis(trifluoromethyl)benzamide (I.1-3)

To a solution of *N*-[1-[2-[*N*-hydroxy-*C*-methyl-carbonimidoyl]-1,2,4-triazol-3-yl]ethyl]-3,5-bis(trifluoromethyl)benzamide (160mg, 0.39mmol) in DMF (2mL) were added K₂CO₃ (108mg, 0.78mmol) and CH₃I (111 mg, 0.78mmol) at 20°C. The reaction mixture was stirred at 20°C for 16h, until completion was determined by LCMS. The mixture was filtered, the filtrate was concentrated, and the residue was purified by preparative HPLC (TFA) to deliver *N*-[1-[2-[(*E*)-*N-*methoxy-*C*-methyl-carbonimidoyl]-1,2,4-triazol-3-yl]ethyl]-3,5-bis(trifluoromethyl)benzamide (I.1-3, 90mg, 42% yield) as a yellow solid.

¹H-NMR (400MHz, DMSO-d6): *δ* = 9.56 (d, J = 7.0Hz, 1H), 8.52 (s, 2H), 8.34 (s, 1H), 8.12 (s, 1H), 5.62 (dq, J = 7.0Hz, J = 6.9Hz, 1H), 3.86 (s, 3H), 2.34 (s, 3H), 1.61 (d, J = 6.9Hz, 3H).

¹³C-NMR (125.7 MHz, CDCl₃): *δ* = 163.43, 157.18, 150.32, 149.62, 135.77, 132.12, 132.12, 127.39, 127.39, 125.17, 122.80, 122.80, 63.04, 43.92, 20.30, 13.29.

### Example 4 - Preparation of 3-chloro-N-[1-[2-[C-methyl-N-phenoxy-carbonimidoyl]-1,2,4-triazol-3-yl]ethyl]-5-(trifluoromethyl)benzamide (I.1-13)

To a solution of 3-chloro-*N*-[1-[2-[*N*-hydroxy-*C*-methyl-carbonimidoyl]-1,2,4-triazol-3-yl]ethyl]-5-(trifluoromethyl)benzamide (200mg, 0.53mmol) in DCE (10ml) was added phenylboronic acid (130mg, 1.06mmol), pyridine (83mg, 1.06mmol) and Cu(OAc)₂ (211mg, 1.06mmol) at 20°C. The reaction mixture was stirred at 50°C for 16h under O₂ atmosphere, until completion was determined by LC-MS. The reaction mixture was filtered. The filtrate was diluted with water (10ml), extracted with EtOAc (3x20ml) and washed with brine (10ml). The organic layer was dried over Na₂SO₄, filtered and concentrated under reduced pressure. The crude product was purified by preparative HPLC (NH₄HCO₃, ACN-water) to give 3-chloro-N-[1-[2-[C-methyl-N-phenoxy-carbonimidoyl]-1,2,4-triazol-3-yl]ethyl]-5-(trifluoromethyl)benzamide (I.1-13, 50mg, 21% yield) as a white solid.

¹H-NMR (400MHz, CDCl₃) *δ* = 8.03 (s, 1H), 7.88 (d, *J*=14.4Hz, 2H), 7.74 (s, 1H), 7.37-7.30 (m, 2H), 7.13-7.06 (m, 1H), 6.78-6.67 (m, 1H), 5.44-5.28 (m, 1H), 2.62 (s, 3H), 1.73 (d, *J*=6.9Hz, 3H).

### Example 5 - Preparation of 3-chloro-N-[1-[1-(C-methyl-N-phenoxy-carbonimidoyl)-1,2,4-triazol-3-yl]ethyl]-5-(trifluoromethyl)benzamide (I.2-6)

### Step 1: Preparation of N-phenoxyacetamide

To a solution of phenoxyammonium chloride (2g, 18mmol) and NaHCO₃ (4.62g, 55mmol) in THF (20ml) was added dropwise acetyl chloride (2.5M, 1.43g, 18mmol) at 0°C under N₂ atmosphere. The resulting mixture was stirred at 20°C for 16h. TCL (PE:EtOAc=3:1) showed the reaction was completed. The reaction mixture was quenched with NH₄Cl (aqueous, 30ml) and extracted with EtOAc (2x30 ml). The organic layer was washed with brine (30ml), dried over Na₂SO₄, filtered and concentrated under reduced pressure. The crude product was purified by column (PE:EtOAc=3:1) to give *N*-phenoxyacetamide (1.5g, 56% yield) as a yellow oil.

¹H-NMR (400MHz, CDCl₃) *δ* = 7.28-7.34 (m, 2H), 7.16-7.22 (m, 2H), 7.05 (t, *J*=7.25Hz, 1H), 2.39 (s, 3H).

### Step 2: Preparation of N-phenoxyacetimidoyl chloride

To a solution of *N*-phenoxyacetamide (1g, 6.6mmol) in toluene (15ml) was added phosphoroxy chloride (5g, 33mmol) at 20°C. The mixture was stirred at 80°C for 16h, TLC (PE:EtOAc=10:1) showed that the reaction was completed. The reaction mixture was concentrated under reduced pressure and purified by column (PE~PE:EtOAc=80:1) to give *N*-phenoxyacetimidoyl chloride (800mg, 73% yield) as a grey solid.

¹H-NMR (400MHz, CDCl₃) *δ* = 7.31-7.36 (m, 2H), 7.20-7.24 (m, 2H), 7.08 (br d, *J*=7.25Hz, 1H), 2.41 (s, 3H)

### Step 3: Preparation of 3-chloro-N-[1-[1-(C-methyl-N-phenoxy-carbonimidoyl)-1,2,4-triazol-3-yl]ethyl]-5-(trifluoromethyl)benzamide (I.2-6)

To a solution of *N*-phenoxyacetimidoyl chloride (500mg, 3mmol) and 3-chloro-*N*-[1-1*H*-1,2,4-triazol-3-yl)ethyl]-5-(trifluoromethyl)benzamide (1.24g, 3.9mmol) in THF (10ml) was added CsF (762mg, 3.77mmol) at 20°C. The mixture was stirred at 20°C for 16h until TLC (PE:EtOAc=3:1) showed the reaction was completed. The reaction mixture was quenched with NH₄Cl (aqueous, 20ml) and extracted with EtOAc (2x30 ml). The organic layer was washed with brine (20ml), dried over Na₂SO₄, filtered and concentrated under reduced pressure. The crude product was purified by column (PE~PE:EtOAc=5:1) to give 3-chloro-*N*-[1-[1-(*C*-methyl-*N*-phenoxy-carbon-imidoyl)-1,2,4-triazol-3-yl]ethyl]-5-(trifluoromethyl)benzamide (I.2-6, 300mg, 23% yield) as a yellow solid.

¹H-NMR (400MHz, CDCl₃) *δ* = 9.38 (s, 1H), 7.99 (d, *J*=13.55Hz, 2H), 7.76 (s, 1H), 7.35-7.42 (m, 2H), 7.29 (br d, *J*=1.00Hz, 2H), 7.10-7.15 (m, 1H), 6.98 (br d, *J*=7.53Hz, 1H), 5.54 (quin, *J*=7.12Hz, 1H), 2.60 (s, 3H), 1.70 (d, *J*=6.90Hz, 3H).

### Example 6 - Preparation of 3-chloro-N-[1-[2-(N-ethoxy-C-methyl-carbonimidoyl)-1,2,4-triazol-3-yl]ethyl]-5-(trifluoromethyl)benzamide (I.1-12)

To a solution of 3-chloro-*N*-[1-[2-(*N*-hydroxy-*C*-methyl-carbonimidoyl)-1,2,4-triazol-3-yl]-5-(trifluoromethyl)benzamide (400mg, 1.06mmol) in DMF (5ml) was added iodoethane (250mg, 1.60mmol) and Cs₂CO₃ (691mg, 2.12mmol) at 20°C. The mixture was stirred at 20°C for 16h until TLC showed the reaction was completed. The reaction mixture was diluted with water (10ml) and extracted with EtOAc (3x10ml). The organic layer was washed with brine (3x10 ml), dried over Na₂SO₄, filtered and concentrated under reduced pressure. The crude product was purified by preparative HPLC to give 3-chloro-N-[1-[2-(N-ethoxy-C-methyl-carbonimidoyl)-1,2,4-triazol-3-yl]ethyl]-5-(trifluoromethyl)benzamide (I.1-12, 60mg, 14% yield) as a white solid.

¹H-NMR (400MHz, CDCl₃) *δ* = 7.97 (s, 1H), 7.93 (s, 1H), 7.90 (s, 1H), 7.75 (s, 1H), 6.83 (br d, *J*=7.1Hz, 1H),5.35 (t, *J*=7.1Hz, 1H), 4.23-4.15 (m, 2H), 4.43 (s, 3H), 1.67 (d, *J*=6.9Hz, 3H), 1.28 (t, *J*=7.1Hz, 3H).

The geometry of the C=N double bond (E/Z-isomerism) was assigned by NMR as described in G. E. Hawkes et al, The Journal of Organic Chemistry 1974, 39, 1017-1028.

With appropriate modification of the starting materials, the procedures given in the synthesis descriptions were used to obtain further compounds I. The compounds obtained in this manner are listed in the table that follows, together with physical data.

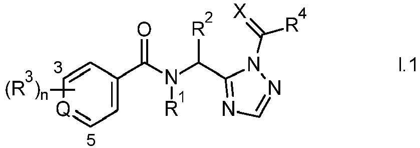

**Table I.1 - Compounds of formula I.1**

| No. | R¹ | R² | Q | R³ | X | R⁴ | m.p. [°C] | phys. data | | |
|---|---|---|---|---|---|---|---|---|---|---|
| | | | | | | | | HPLC/MS method | RT [min.] | [m/z] |
| I.1-1 | H | CH₃ | CH | 3,5-(CF₃)₂ | (Z)-N-OH | CH₃ | 173 | A | 1.050 | 409.9 |
| I.1-2 | H | CH₃ | CH | 3,5-(CF₃)₂ | (Z)-N-OCH₃ | CH₃ | 108.4 | A | 1.212 | 423.9 |
| I.1-3 | H | CH₃ | CH | 3,5-(CF₃)₂ | (E)-N-OCH₃ | CH₃ | 105.4 | A | 1.240 | 424.0 |
| I.1-4 | H | CH₃ | CH | 3,5-(CF₃)₂ | (E)-N-OCH₂CF₃ | CH₃ | | A | 1.303 | 491.9 |
| I.1-5 | H | CH₃ | CH | 3,5-(CF₃)₂ | (Z)-N-OCH₂CF₃ | CH₃ | | A | 1.296 | 491.9 |
| I.1-6 | H | CH₃ | CH | 3,5-(CF₃)₂ | N-OCH₃ | C(O)OC₂H₅ | | A | 1.197 | 482.0 |
| I.1-7 | H | CH₃ | CH | 3,5-(CF₃)₂ | N-OCH₃ | CH₃ | | A | 1.066 | 467.0 |
| I.1-8 | H | CH₃ | CH | 3,5-(CF₃)₂ | N-OCH₃ | C(O)N(CH₃)C₂H₅ | | B | 1.622 | 495.1 |
| I.1-9 | H | CH₃ | CH | 3,5-(CF₃)₂ | N-OCH₃ | CN | | A | 1.143 | 453.3 |
| I.1-10 | H | CH₃ | CH | 3,5-(CF₃)₂ | N-OCH₃ | 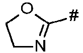 | | A | 1.077 | 479.3 |
| I.1-11 | H | CH₃ | CH | 3,5-(CF₃)₂ | N-OCH₃ | C(O)NH₂ | | A | 1.004 | 453.3 |
| I.1-12 | H | CH₃ | CH | 3-Cl-5-CF₃ | N-OC₂H₅ | CH₃ | | B | 1.608 | 404.2 |
| I.1-13 | H | CH₃ | CH | 3-Cl-5-CF₃ | N-OC₆H₅ | CH₃ | | B | 1.729 | 452.2 |
| I.1-14 | H | CH₃ | CH | 3-Cl-5-CF₃ | N-OCH₃ | CH₃ | | A | 1.196 | 390.2 |

| No. | R¹ | R² | Q | R³ | X | R⁴ | phys. data | | | |
|---|---|---|---|---|---|---|---|---|---|---|
| | | | | | | | m.p. [°C] | HPLC/MS method | RT [min.] | [m/z] |
| I.1-15 | H | CH₃ | CH | 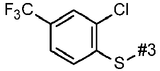 | N-OCH₃ | CH₃ | | A | 1.359 | 498.1 |
| I.1-16 | H | CH₃ | CH | 3,5-Br₂ | N-OCH₃ | CH₃ | | A | 1.193 | 445.9 |
| I.1-17 | H | CH₃ | CH | 3-O(2,2-Cl₂-cC₃H₃) | N-OCH₃ | CH₃ | | A | 1.142 | 412.0 |
| I.1-18 | H | CH₃ | CH | 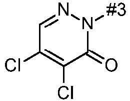 | N-OCH₃ | CH₃ | | A | 1.040 | 450.0 |
| I.1-19 | H | CH₃ | CR³ | #3-(CH₂)₃O-#4 | N-OCH₃ | CH₃ | | A | 0.997 | 344.1 |
| I.1-20 | H | CH₃ | CR³ | #3-(CH₂)₄-#4 | N-OCH₃ | CH₃ | | A | 1.135 | 342.1 |
| I.1-21 | H | CH₃ | CH | 3-OCF₃ | N-OCH₃ | CH₃ | | A | 1.113 | 372.0 |
| I.1-22 | H | CH₃ | CR³ | #3-OCH₂O-#4 | N-OCH₃ | CH₃ | | A | 0.933 | 332.0 |
| I.1-23 | H | CH₃ | CH | 3,5-Cl₂ | N-OCH₃ | CH₃ | | A | 1.157 | 356.0 |
| I.1-24 | H | CH₃ | CR³ | 4-NHC(O)CH₃-3,5-Cl₂ | N-OCH₃ | CH₃ | | A | 0.895 | 413.0 |
| I.1-25 | H | CH₃ | CH | 3-Br-5-CH₃ | N-OCH₃ | CH₃ | | A | 1.125 | 380.0 |
| I.1-26 | H | CH₃ | CH | 3-SCF₃ | N-OCH₃ | CH₃ | | A | 1.150 | 388.0 |
| I.1-27 | H | CH₃ | CR³ | 3,5-Cl₂-4-F | N-OCH₃ | CH₃ | | A | 1.175 | 374.0 |
| I.1-28 | H | CH₃ | CH | 3-C(CH₃)₃ | N-OCH₃ | CH₃ | | A | 1.166 | 344.3 |
| I.1-29 | H | CH₃ | CR³ | 4-CN-3,5-F₂ | N-OCH₃ | CH₃ | | A | 1.034 | 349.1 |
| I.1-30 | H | CH₃ | CH | 3-NHC(O)-cC₃H₅ | N-OCH₃ | CH₃ | | A | 0.919 | 371.1 |
| I.1-31 | H | CH₃ | CH | 3-CI-5-CN | N-OCH₃ | CH₃ | | A | 1.047 | 347.0 |
| I.1-32 | H | CH₃ | CH | 3-SO₂CF₃ | N-OCH₃ | CH₃ | | A | 1.101 | 420.0 |
| I.1-33 | H | CH₃ | CH | 3-Cl-5-SO₂CF₃ | N-OCH₃ | CH₃ | | A | 1.198 | 454.0 |
| I.1-34 | H | CH₃ | CR³ | 4-Cl-3-SO₂CH₃ | N-OCH₃ | CH₃ | | A | 0.923 | 399.9 |
| I.1-35 | H | CH₃ | CH | 3-Br-5-OCH(CH₃)₂ | N-OCH₃ | CH₃ | | A | 1.214 | 426.0 |
| I.1-36 | H | CH₃ | CR³ | 3-Br-4,5-(OCH₃)₂ | N-OCH₃ | CH₃ | | A | 1.076 | 426.0 |
| I.1-37 | H | CH₃ | CH | 4-Br-3-SO₂N(CH₃)₂ | N-OCH₃ | CH₃ | | A | 1.029 | 475.0 |
| I.1-38 | H | CH₃ | CH | 3-Cl-5-SO₂CH₃ | N-OCH₃ | CH₃ | | A | 0.976 | 399.9 |
| I.1-39 | H | CH₃ | CH | 3-Br-5-CH(CH₃)₂ | N-OCH₃ | CH₃ | | A | 1.241 | 408.1 |
| I.1-40 | H | CH₃ | CR³ | 3-Br, #4-(OCH₂O)-#5 | N-OCH₃ | CH₃ | | A | 1.052 | 411.9 |
| I.1-41 | H | CH₃ | CH | 3-Br-5-SO₂CH₃ | N-OCH₃ | CH₃ | | A | 0.989 | 445.9 |
| I.1-42 | H | CH₃ | CH | 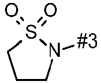 | N-OCH₃ | CH₃ | | A | 0.896 | 407.1 |
| I.1-43 | H | CH₃ | CH | 3-O(4-CN-C₆H₄) | N-OCH₃ | CH₃ | | A | 1.107 | 405.2 |
| I.1-44 | H | CH₃ | CH | 3-OCH₂CH₂CH₃ | N-OCH₃ | CH₃ | | A | 1.098 | 346.1 |
| I.1-45 | H | CH₃ | CR³ | #3-(CH₂)₃-#4 | N-OCH₃ | CH₃ | | A | 1.081 | 328.1 |
| I.1-46 | H | CH₃ | CH | 3-Br-5-OCF₃ | N-OCH₃ | CH₃ | | A | 1.223 | 450.0 |
| I.1-47 | H | CH₃ | CH | 3-OCH₂CH(CH₃)₂ | N-OCH₃ | CH₃ | | A | 1.173 | 360.1 |
| I.1-48 | H | CH₃ | CH | 3-SO₂NHCH(CH₃)₂ | N-OCH₃ | CH₃ | | A | 0.964 | 409.1 |
| I.1-49 | H | CH₃ | CH | 3-Br-5-CH₂OCH₃ | N-OCH₃ | CH₃ | | A | 1.083 | 412.0 |
| I.1-50 | H | CH₃ | CH | 3-Cl-5-SCH₃ | N-OCH₃ | CH₃ | | A | 1.144 | 368.0 |
| I.1-51 | H | CH₃ | CH | 3-SO₂NH-cC₃H₅ | N-OCH₃ | CH₃ | | A | 0.944 | 407.1 |
| I.1-52 | H | CH₃ | CH | 3-OC₂H₅-5-F | N-OCH₃ | CH₃ | | A | 1.081 | 350.1 |
| I.1-53 | H | CH₃ | CR³ | 3,5-Br₂-4-OCH₃ | N-OCH₃ | CH₃ | | A | 1.171 | 475.9 |
| I.1-54 | H | CH₃ | CH | 3,5-F₂ | N-OCH₃ | CH₃ | | A | 1.022 | 324.0 |
| I.1-55 | H | CH₃ | CH | 3,5-(OCH₃)₂ | N-OCH₃ | CH₃ | | A | 0.984 | 348.0 |
| I.1-56 | H | CH₃ | CH | 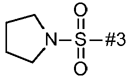 | N-OCH₃ | CH₃ | | A | 1.007 | 421.3 |
| I.1-57 | H | CH₃ | CH | 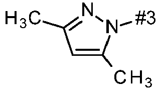 | N-OCH₃ | CH₃ | | A | 0.988 | 382.2 |
| I.1-58 | H | CH₃ | CH | 3-Cl-5-CF₃ | 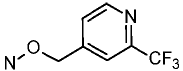 | CH₃ | | A | 1.288 | 534.8 |
| I.1-59 | H | CH₃ | CH | 3-Cl-5-CF₃ | N-OH | CH₃ | | A | 1.041 | 375.7 |
| I.1-60 | H | CH₃ | CH | 3-Cl-5-CF₃ | N-OCH₂-(4-F-C₆H₄) | CH₃ | | A | 1.347 | 483.8 |
| I.1-61 | H | CH₃ | CH | 3-Cl-5-CF₃ | 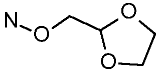 | CH₃ | | A | 1.166 | 462.2 |
| I.1-62 | H | CH₃ | CH | 3-Cl-5-CF₃ | N-OCH₂CN | CH₃ | | A | 1.128 | 414.8 |
| I.1-63 | H | CH₃ | CH | 3-Cl-5-CF₃ | N-OCH₂CH₂CH₃ | CH₃ | | A | 1.322 | 417.9 |
| I.1-64 | H | CH₃ | CH | 3-Cl-5-CF₃ | N-OCH₂CHF₂ | CH₃ | | A | 1.218 | 440.2 |
| I.1-65 | H | CH₃ | CH | 3-O-cC₅H₉ | N-OCH₃ | CH₃ | | A | 1.167 | 372.2 |
| I.1-66 | H | CH₃ | CH | 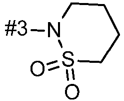 | N-OCH₃ | CH₃ | | A | 0.933 | 421.1 |
| I.1-67 | H | CH₃ | CR³ | 4-CH₃-3-SO₂CH₃ | N-OCH₃ | CH₃ | | A | 0.896 | 380.0 |
| I.1-68 | H | CH₃ | CR³ | 3-Cl-#4-OCH₂CH₂O-#5 | N-OCH₃ | CH₃ | | A | 1.022 | 3800.0 |
| I.1-69 | H | CH₃ | CH | 3-SO₂CH(CH₃)₂ | N-OCH₃ | CH₃ | | A | 0.936 | 394.1 |
| I.1-70 | H | CH₃ | CH | 3-Cl-5-CF₃ | N-OCH₂CH=CCl₂ | CH₃ | | A | 1.377 | 485.7 |
| I.1-71 | H | CH₃ | CH | 3-Cl-5-CF₃ | N-OCH₂CH₂-OC₆H₅ | CH₃ | | A | 1.342 | 495.8 |
| I.1-72 | H | CH₃ | CH | 3-Cl-5-CF₃ | 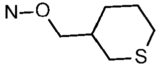 | CH₃ | | A | 1.375 | 489.9 |
| I.1-73 | H | CH₃ | CH | 3-Cl-5-CF₃ | 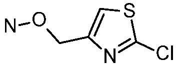 | CH₃ | | A | 1.289 | 507.1 |
| I.1-74 | H | CH₃ | CH | 3-Cl-5-CF₃ | N-OCH₂C(O)NH₂ | CH₃ | | A | 0.974 | 432.8 |
| I.1-75 | H | CH₃ | CH | 3-Cl-5-CF₃ | 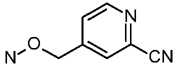 | CH₃ | | A | 1.196 | 491.9 |
| I.1-76 | H | CH₃ | CH | 3,5-(CF₃)₂ | (E) N-OH | CH₃ | 158 | A | 1.075 | 409.9 |
| I.1-77 | CH₃ | CH₃ | CH | 3-Cl-5-CF₃ | N-OCH₃ | CH₃ | | A | 1.224 | 403.8 |
| I.1-78 | H | CH₃ | CH | 3-Cl-5-CF₃ | N-O(CH₂)₂OCH₃ | CH₃ | | A | 1.18 | 434.2 |
| I.1-79 | H | CH₃ | CH | 3-SO₂CHF₂ | N-OCH₃ | CH₃ | | A | 0.986 | 402 |
| I.1-80 | H | CH₃ | CH | 3-Br-5-O-cC₃H₅ | N-OCH₃ | CH₃ | | A | 1.183 | 424 |
| I.1-81 | H | CH₃ | CH | 3-Br-5-O-cC₃H₅ | N-OCH₃ | CH₃ | | A | 1.183 | 424 |
| I.1-82 | H | CH₃ | CH | 3-CN-4,5-F₂ | N-OCH₃ | CH₃ | | A | 1.021 | 349 |
| I.1-83 | H | CH₃ | CH | 3-S(O)CH₃ | N-OCH₃ | CH₃ | | A | 0.786 | 350 |
| I.1-84 | H | CH₃ | CH | 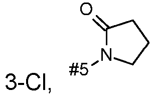 | N-OCH₃ | CH₃ | | A | 0.996 | 405.1 |
| I.1-85 | H | CH₃ | | 3-cC₃H₅-5-OCH₃ | N-OCH₃ | CH₃ | | A | 1.105 | 358.2 |
| I.1-86 | H | CH₃ | | 3-cC₅H₉ | N-OCH₃ | CH₃ | | A | 1.204 | 356.2 |
| I.1-87 | H | CH₃ | | #3-SO₂(CH₂)₃-#4 | N-OCH₃ | CH₃ | | A | 0.877 | 392.2 |
| I.1-88 | H | CH₃ | | 3-(1-CN-cC₃H₄) | N-OCH₃ | CH₃ | | A | 0.983 | 353.1 |
| I.1-89 | H | CH₃ | | 3-C(CH₃)₂CN-5-CF₃ | N-OCH₃ | CH₃ | | A | 1.141 | 423.2 |
| I.1-90 | H | CH₃ | | 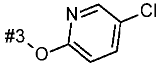 | N-OCH₃ | CH₃ | | A | 1.113 | 415.2 |
| I.1-91 | H | CH₃ | CR³ | 3-Cl-5-OCH₃-4-OCH(CH₃)₂ | N-OCH₃ | CH₃ | | A | 1.175 | 410.1 |
| I.1-92 | H | CH₃ | CR³ | #3-C(CH₃)₂CH₂O-#4 | N-OCH₃ | CH₃ | | A | 1.05 | 358.1 |
| I.1-93 | H | CH₃ | CH | 3-O(2-CN,4-Cl-C₆H₃) | N-OCH₃ | CH₃ | | A | 1.164 | 439.1 |
| I.1-94 | H | CH₃ | CH | 3-(1-OCH₃)-CC₄H₆ | N-OCH₃ | CH₃ | | A | 1.059 | 372.1 |
| I.1-95 | H | CH₃ | CR³ | 3-Cl-4-OCH₃-5-CH₃ | N-OCH₃ | CH₃ | | A | 1.087 | 366 |
| I.1-96 | H | CH₃ | CH | 3-C₂H₅-5-CF₃ | N-OCH₃ | CH₃ | | A | 1.197 | 384.2 |
| I.1-97 | H | (S)-CH₃ | CH | 3-Cl-5-CF₃ | N-OCH₃ | CH₃ | | A | 1.2 | 389.9 |
| I.1-98 | H | (R)-CH₃ | CH | 3-Cl-5-CF₃ | N-OCH₃ | CH₃ | | A | 1.99 | 389.7 |
| I.1-99 | H | CH₃ | N | 3,5-(Cl)₂ | N-OCH₃ | CH₃ | | A | 1.073 | 357.1 |
| I.1-100 | H | CH₃ | N | 3-C(CH₃)₃ | N-OCH₃ | CH₃ | | A | 0.832 | 345.2 |
| I.1-101 | H | CH₃ | N | 3-OC₆H₅ | N-OCH₃ | CH₃ | | A | 1.053 | 381.1 |
| I.1-102 | H | CH₃ | N | 3-C(CH₃)₃-5-Cl | N-OCH₃ | CH₃ | | A | 1.22 | 379.1 |
| I.1-103 | H | CH₃ | N | 3-OH-5-C₃H₇ | N-OCH₃ | CH₃ | | A | 0.846 | 347.1 |
| I.1-104 | H | CH₃ | CH | 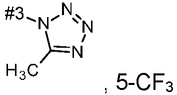 | N-OH | CH₃ | - | A | 0.906 | 424.2 |
| I.1-105 | H | CH₃ | CH | 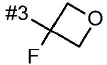 | N-OH | CH₃ | - | A | 0.803 | 348.2 |
| I.1-106 | H | CH₃ | CH | 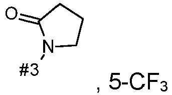 | N-OH | CH₃ | - | A | 0.928 | 425.3 |
| I.1-107 | H | CH₃ | CH | 3-NHC(O)CH₃-5-CF₃ | N-OH | CH₃ | - | A | 0.879 | 399.2 |
| I.1-108 | H | CH₃ | CH | 3-(2,2-F₂-CC₃H₃) | N-OH | CH₃ | - | A | 0.902 | 350 |
| I.1-109 | H | CH₃ | CH | 3-O-cC₄H₇ | N-OH | CH₃ | - | A | 0.949 | 344.1 |
| I.1-110 | H | CH₃ | CH | 3-(2,2,3,3-F₄-cC₄H₃) | N-OH | CH₃ | - | A | 0.997 | 400.1 |
| I.1-111 | H | CH₃ | CH | 3-Cl-5-cC₃H₅ | N-OH | CH₃ | - | A | 1.03 | 348 |
| I.1-112 | H | CH₃ | CH | 3-Cl-5-CHF₂ | N-OH | CH₃ | - | A | 0.97 | 358 |
| I.1-113 | H | CH₃ | N | 3-Cl-5-SCH₃ | N-OH | CH₃ | - | A | 0.957 | 355 |
| L.1-114 | H | CH₃ | CH | 3-CH=CF₂ | N-OH | CH₃ | - | A | 0.941 | 336 |
| l.1-115 | H | CH₃ | N | 3-O-cC₃H₅ | N-OH | CH₃ | - | A | 0.798 | 331.1 |
| I.1-116 | H | CH₃ | CH | 3-O-(3-F-C₆H₄) | N-OH | CH₃ | - | A | 1.049 | 384.1 |
| I.1-117 | H | CH₃ | CH | 3-cC₃H₅-5-F | N-OH | CH₃ | - | A | 0.976 | 332.1 |

**Table I.2 - Compounds of formula I.2**

| 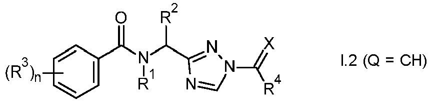 | | | | | | | | |
|---|---|---|---|---|---|---|---|---|
| No. | R¹ | R² | R³ | X | R⁴ | phys. data | | |
| | | | | | | HPLC/MS method | RT [min.] | [m/z] |
| I.2-1 | H | CH₃ | 3,5-(CF₃)₂ | N-OC₂H₅ | C(O)OC₂H₅ | A | 1.223 | 482.0 |
| I.2-2 | H | CH₃ | 3,5-(CF₃)₂ | N-OCH₃ | C(O)NHCH₃ | A | 1.075 | 466.9 |
| I.2-3 | H | CH₃ | 3,5-(CF₃)₂ | N-OCH₃ | C(O)N(CH₃)C₂H₅ | B | 1.575 | 495.1 |
| I.2-4 | H | CH₃ | 3,5-(CF₃)₂ | N-OCH₃ | CN | A | 1.114 | 435.3 |
| I.2-5 | H | CH₃ | 3,5-(CF₃)₂ | N-OCH₃ | 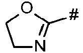 | A | 0.895 | 479.3 |
| I.2-6 | H | CH₃ | 3-Cl-5-CF₃ | N-OC₆H₅ | CH₃ | B | 1.780 | 452.1 |
| I.2-7 | H | CH₃ | 3-Cl-5-CF₃ | N-OH | CH₃ | B | 1.416 | 376.1 |

**Table I.3 - Compounds of formula I.3**

| 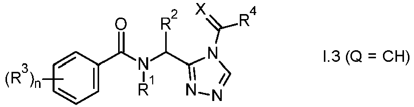 | | | | | | | | |
|---|---|---|---|---|---|---|---|---|
| No. | R¹ | R² | R³ | X | R⁴ | phys. data | | |
| | | | | | | HPLC/MS method | RT [min.] | [m/z] |
| I.3-2 | H | CH₃ | 3,5-(CF₃)₂ | N-OCH₃ | C(O)NHCH₃ | A | 1.015 | 467.1 |
| I.3-3 | H | CH₃ | 3,5-(CF₃)₂ | N-OCH₃ | C(O)N(CH₃)C₂H₅ | B | 1.551 | 495.1 |

| No. | R¹ | R² | R³ | X | R⁴ | phys. data | | |
|---|---|---|---|---|---|---|---|---|
| | | | | | | HPLC/MS method | RT [min.] | [m/z] |
| I.3-4 | H | CH₃ | 3,5-(CF₃)₂ | N-OCH₃ | 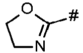 | A | 1.037 | 479.3 |
| I.3-5 | H | CH₃ | 3,5-(CF₃)₂ | N-OCH₃ | CN | A | 1.114 | 435.3 |
| I.3-6 | H | CH₃ | 3,5-(CF₃)₂ | N-OCH₂C₆H₅ | H | B | 1.713 | 486.1 |

| | | | | | | | | |
|---|---|---|---|---|---|---|---|---|
| # denotes the bond to the remainder of the molecule #3, #4 denotes the bond to position 3, and 4, resp. | | | | | | | | |

### Biological examples

If not otherwise specified, the test solutions were prepared as follow:
The active compound was dissolved at the desired concentration in a mixture of 1:1 (vol:vol) distilled water : acetone. The test solution was prepared on the day of use.

The activity of the compounds of formula I of the present invention can be demonstrated and evaluated by the following biological tests.

### B.1 Diamond back moth (Plutella xylostella)

The active compound was dissolved at the desired concentration in a mixture of 1:1 (vol:vol) distilled water : acetone. Surfactant (Kinetic HV) was added at a rate of 0.01% (vol/vol). The test solution was prepared on the day of use.

Leaves of cabbage were dipped in test solution and air-dried. Treated leaves were placed in petri dishes lined with moist filter paper and inoculated with ten 3rd instar larvae. Mortality was recorded 72 hours after treatment. Feeding damages were also recorded using a scale of 0-100%.

In this test, compounds I.1-1, I.1-2, I.1-3, I.1-4, I.1-5, I.1-8, I.1-11, and I.1-76, resp., at 300 ppm showed at least 75% mortality in comparison with untreated controls.

### B.2 Green Peach Aphid (Myzus persicae)

For evaluating control of green peach aphid (*Myzus persicae*) through systemic means, the test unit consisted of 96-well-microtiter plates containing liquid artificial diet under an artificial mem brane.

The compounds were formulated using a solution containing 75% v/v water and 25% v/v DMSO. Different concentrations of formulated compounds were pipetted into the aphid diet, using a custom built pipetter, at two replications.

After application, 5 - 8 adult aphids were placed on the artificial membrane inside the microtiter plate wells. The aphids were then allowed to suck on the treated aphid diet and incubated at about 23 ± 1°C and about 50 ± 5 % relative humidity for 3 days. Aphid mortality and fecundity was then visually assessed.

In this test, compounds I.1-1, I.1-2, I.1-3, I.1-11, I.1-14, I.1-23, I.1-32, I.1-38, I.1-41, I.1-46, I.1-50, I.1-55, I.1-62, I.1-76, and I.1-99, resp., at 2500 ppm showed at least 75% mortality in comparison with untreated controls.

### B.4 Tobacco budworm (Heliothis virescens)

For evaluating control of tobacco budworm (*Heliothis virescens*), the test unit consisted of 96-well-microtiter plates containing an insect diet and 15-25 *H. virescens* eggs.

The compounds were formulated using a solution containing 75% v/v water and 25% v/v DMSO. Different concentrations of formulated compounds were sprayed onto the insect diet at 10 µl, using a custom-built micro atomizer, at two replications.

After application, microtiter plates were incubated at about 28 ± 1°C and about 80 ± 5 % relative humidity for 5 days. Egg and larval mortality was then visually assessed.

In this test, compounds I.1-1, 1.1-2, I.1-3, I.1-4, 1.1-33, 1.1-41, 1.1-50, 1.1-59, and I.1-76, resp., at 2500 ppm showed at least 75% mortality in comparison with untreated controls.

### B.5 Boll weevil (Anthonomus grandis)

For evaluating control of boll weevil (*Anthonomus grandis*)*,* the test unit consisted of 96-well-microtiter plates containing an insect diet and 5-10 *A. grandis* eggs.

The compounds were formulated using a solution containing 75% v/v water and 25% v/v DMSO. Different concentrations of formulated compounds were sprayed onto the insect diet at 5 µl, using a custom-built micro atomizer, at two replications.

After application, microtiter plates were incubated at about 25 ± 1°C and about 75 ± 5 % relative humidity for 5 days. Egg and larval mortality was then visually assessed.

In this test, compounds I.1-1, 1.1-2, I.1-3, I.1-4, I.1-11, 1.1-12, 1.1-16, 1.1-21, I.1-23, I.1-26, I.1-31, 1.1-32, 1.1-33, 1.1-38, 1.1-40, 1.1-41, 1.1-46, 1.1-50, I.1-55, I.1-59, I.1-62, I.1-76, I.1-99, and I.2-4, resp., at 2500 ppm showed at least 75% mortality in comparison with untreated controls.

### B.7 Orchid thrips (Dichromothrips corbetti)

*Dichromothrips corbetti* adults used for bioassay were obtained from a colony maintained continuously under laboratory conditions. For testing purposes, the test compound is diluted in a 1:1 mixture of acetone:water (vol:vol), plus Kinetic HV at a rate of 0.01% v/v.

Thrips control potency of each compound was evaluated by using a floral-immersion technique. All petals of individual, intact orchid flowers were dipped into treatment solution and allowed to dry in Petri dishes. Treated petals were placed into individual re-sealable plastic along with about 20 adult thrips. All test arenas were held under continuous light and a temperature of about 28°C for duration of the assay. After 3 days, the numbers of live thrips were counted on each petal. The percent mortality was recorded 72 hours after treatment.

In this test, compounds 1.1-2, I.1-3, and 1.1-11, resp., at 300 ppm showed at least 75% mortality in comparison with untreated controls.

### B.8 Yellow fever mosquito (Aedes aegypti)

For evaluating control of yellow fever mosquito (*Aedes aegypti*) the test unit consisted of 96-well-microtiter plates containing 200µl of tap water per well and 5-15 freshly hatched *A. aegypti* larvae.

The active compounds were formulated using a solution containing 75% (v/v) water and 25% (v/v) DMSO. Different concentrations of formulated compounds or mixtures were sprayed onto the insect diet at 2.5µl, using a custom-built micro atomizer, at two replications.

After application, microtiter plates were incubated at 28 ± 1°C, 80 ± 5 % RH for 2 days. Larval mortality was then visually assessed.

In this test, compounds I.1-1, 1.1-2, I.1-3, I.1-4, I.1-5, I.1-8, I.1-11, 1.1-12, I.1-13, I.1-14, I.1-16, I.1-21, 1.1-23, 1.1-26, 1.1-27, 1.1-31, 1.1-32, 1.1-33, I.1-35, I.1-38, I.1-39, I.1-40, I.1-41, I.1-46, I.1-48, I.1-50, I.1-58, I.1-59, I.1-60, I.1-62, I.1-63, I.1-76, I.1-99, I.2-4, and I.3-5, resp., at 2500 ppm showed at least 75% mortality in comparison with untreated controls.

## Claims

1. Compounds of formula I 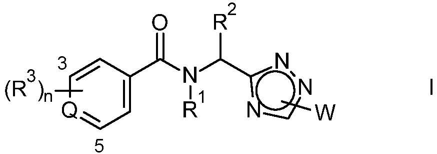 wherein
R¹ is H, OH, C₁-C₆-alkyl, C₁-C₆-haloalkyl, c₃-c₆-cycloalkyl, C₃-C₆-halocycloalkyl, C₁-C₅-alkoxy, C₁-C₄-alkyl-C₃-C₆-cycloalkyl, C₁-C₄-alkyl-C₃-C₆-halocycloalkyl, which groups are unsubstituted, or partially or fully substituted with R¹¹;
or C(=N-R¹¹)R¹², C(O)R^{11a};
R¹¹ is CN, NO₂, NR¹²R¹³, C(O)NH₂, C(S)NH₂, C(O)OH, OR¹⁴, OR¹⁵, Si(CH₃)₃; C₁-C₆-haloalkyl; C₂-C₆-alkenyl; C₂-C₆-haloalkenyl; C₂-C₆-alkynyl; C₂-C₆-haloalkynyl; C₃-C₄-cycloalkyl-C₁-C₂-alkyl, which ring is unsubstituted or substituted with 1 or 2 halogen; 3- to 6-membered heterocyclyl, 5- or 6-membered hetaryl, or phenyl, which rings are unsubstituted or substituted with halogen, C₁-C₃-haloalkyl, and/or CN;
R^{11a} is NR¹²R¹³, C(O)NH₂, C(S)NH₂, C(O)OH, OR¹⁴, OR¹⁵, Si(CH₃)₃; C₁-C₆-haloalkyl; C₂-C₆-alkenyl; C₂-C₆-haloalkenyl; C₂-C₆-alkynyl; C₂-C₆-haloalkynyl; C₃-C₄-cycloalkyl-C₁-C₂-alkyl, which ring is unsubstituted or substituted with 1 or 2 halogen; 3- to 6-membered heterocyclyl, which rings are unsubstituted or substituted with halogen, C₁-C₃-haloalkyl, and/or CN;
R¹², R¹³ are independently from each other H, C₁-C₄-alkyl, C₁-C₄-haloalkyl, C₃-C₆-cycloalkyl, C(O)-C₁-C₄-alkyl, C(O)-C₁-C₄-haloalkyl, C(O)-C₃-C₄-cycloalkyl, C(O)-C₃-C₄-halocycloalkyl, S(O)ₘ₋C₁-C₄-alkyl, S(O)ₘ-C₁-C₄-haloalkyl, S(O)ₘ-C₃-C₄-cycloalkyl, S(O)ₘ-C₃-C₄-halocycloalkyl; or
R¹² and R¹³, together with the nitrogen atom to which they are bound, form a 3-, 4-, 5-, 6- or 7-membered saturated, partially unsaturated or fully unsaturated heterocycle, which heterocycle may additionally contain 1 or 2 heteroatoms or heteroatom groups selected from N, O, and S(O)ₘ as ring members, which heterocycle is unsubstituted or substituted with one or more substituents selected from halogen, C₁-C₄-alkyl, C₁-C₄-haloalkyl, C₁-C₄-alkoxy, C₁-C₄-haloalk-oxy, and oxo;
m is 0, 1, or 2;
R¹⁴ is H, C₁-C₄-alkyl, C₁-C₄-haloalkyl, C₃-C₆-cycloalkyl, C₃-C₆-halo¬cyclo-alkyl, C₃-C₄-cycloalkyl-C₁-C₂-alkyl, C₃-C₄-halocycloalkyl-C₁-C₂-alkyl, C(O)-C₁-C₄-alkyl, C(O)-C₁-C₄-haloalkyl, C(O)-C₃-C₄-cycloalkyl, C(O)-C₃-C₄-halo¬cyclo¬alkyl, NR¹²R¹³, or 5-, or 6-membered hetaryl or phenyl, which rings are unsubstituted or partially or fully substituted with R^{3a};
R² is H, CN, C₁₋C₃-alkyl, C₁-C₃-haloalkyl, C₂-C₃-alkynyl;
Q is CH, CR³, or N;
R³ is halogen, CN, NO₂, C₁-C₄-alkyl, C₃-C₆-cycloalkyl, C₁-C₆-haloalkyl, C₁-C₆-halocyclo-alkyl, C₁-C₄-alkoxy-C₁-C₄-alkyl, NR¹²R¹³, OR¹⁴, S(O)ₘ-R¹⁴, phenyl, or 3-, 4-, 5-, or 6-membered saturated, or partially or fully unsaturated heterocycle, which heterocycle contains 1, 2, 3, or 4 heteroatoms selected from N, O, and S(O)ₘ as ring members; wherein R³ rings are bonded directly, or via C₁-C₂-alkylene, O, or S(O)ₘ spacer, and are unsubstituted or substituted with R^{3a};
R^{3a} halogen, CN, NO₂, C₁-C₄-alkyl, C₁-C₄-haloalkyl, C₁-C₄-alkoxy-C₁-C₄-alkyl, C₁-C₄-haloalkoxy, C₃-C₄-cycloalkyl, C₃-C₄-halocycloalkyl, S(O)ₘ-C₁-C₄-alkyl, S(O)ₘ-C₁-C₄-haloalkyl, S(O)ₘ-C₃-C₄-cycloalkyl, S(O)ₘ-C₃-C₄-halocycloalkyl; two R^{3a} on the same carbon atom together may form a group oxo;
two R³ on the same carbon atom together may form a group oxo;
two R³ on two adjacent carbon atoms form together with the carbon atoms they are bonded to a 5-, 6-, or 7-membered saturated, partially unsaturated or fully unsaturated ring, wherein the ring may contain 1, 2, or 3 heteroatoms or heteroatom groups selected from N, O, and S(O)ₘ as ring members, and wherein the ring is optionally substituted with one or more groups halogen, CN, C₁-C₄-alkyl, C₁-C₄-haloalkyl, C₁-C₄-alkoxy, and/or C₁-C₄-haloalkoxy;
n is 0, 1, 2, or 3;
W is C(=X)R⁴, being bonded to a nitrogen atom of the triazole ring;
X is O, or NR⁵;
R⁴ is H, OR¹⁴, CN, C₁-C₄-alkyl, C₃-C₆-cycloalkyl, C₁-C₆-haloalkyl, C₁-C₆-halocyclo-alkyl, C₂-C₄-alkenyl, C₂-C₄-halo¬alkenyl, C₂-C₄-alkynyl, C₁-C₄-alkoxy, C₁-C₄-haloalkoxy, S(O)ₘ-C₁-C₄-alkyl, S(O)ₘ-C₁-C₄-haloalkyl, S(O)ₘ-C₃-C₄-cyclo¬alkyl, S(O)ₘ-C₃-C₄-halocyclo¬alkyl, NR¹²R¹³, C(O)NR¹²R¹³, C(O)OR¹⁴, 3- to 6-membered heterocyclyl, 5- or 6-membered hetaryl, or phenyl, which rings are unsubstituted or substituted with R^{3a};
R⁵ is H, OR¹⁴, OR¹⁵, NR¹²R¹³, or C₁-C₆-alkyl which is unsubstituted, or partially or fully substituted with R¹¹;
R¹⁵ is H, C₁-C₄-alkyl, or C₁-C₄-haloalkyl, C₃-C₆-cycloalkyl, C₁-C₆-halocycloalkyl, which carbon chains are unsubstituted or partially or fully substituted with R¹¹; or 3- to 6-membered heterocyclyl, 5- or 6-membered hetaryl, or phenyl, which rings are unsubstituted or substituted with R^{3a};
and the N-oxides, stereoisomers and agriculturally or veterinarily acceptable salts thereof.

2. Compounds of formula I according to claim 1, wherein
R¹¹ is CN, NO₂, NR¹²R¹³, C(O)NH₂, C(S)NH₂, C(O)OH, OR¹⁴, Si(CH₃)₃; C₁-C₆-haloalkyl; C₂-C₆-alkenyl; C₂-C₆-haloalkenyl; C₂-C₆-alkynyl; C₂-C₆-haloalkynyl; C₃-C₄-cycloalkyl-C₁-C₂-alkyl, which ring is unsubstituted or substituted with 1 or 2 halogen; 3- to 6-membered heterocyclyl, 5- or 6-membered hetaryl, or phenyl, which rings are unsubstituted or substituted with halogen, C₁-C₃-haloalkyl, and/or CN;
R^{11a} is NR¹²R¹³, C(O)NH₂, C(S)NH₂, C(O)OH, OR¹⁴, Si(CH₃)₃; C₁-C₆-haloalkyl; C₂-C₆-alkenyl; C₂-C₆-haloalkenyl; C₂-C₆-alkynyl; C₂-C₆-haloalkynyl; C₃-C₄-cycloalkyl-C₁-C₂-alkyl, which ring is unsubstituted or substituted with 1 or 2 halogen; 3- to 6-membered heterocyclyl, which rings are unsubstituted or substituted with halogen, C₁-C₃-haloalkyl, and/or CN;
R¹², R¹³ are independently from each other H, C₁-C₄-alkyl, C₁-C₄-haloalkyl, C₃-C₆-cycloalkyl, C(O)-C₁-C₄-alkyl, C(O)-C₁-C₄-haloalkyl, C(O)-C₃-C₄-cycloalkyl, C(O)-C₃-C₄-halocycloalkyl, S(O)ₘC₁-C₄-alkyl, S(O)ₘ-C₁-C₄-haloalkyl, S(O)ₘ-C₃-C₄-cycloalkyl, S(O)ₘ-C₃-C₄-halocycloalkyl,
R¹⁴ is H, C₁-C₄-alkyl, C₁-C₄-haloalkyl, C₃-C₆-cycloalkyl, C₃-C₆-halo¬cyclo-alkyl, C₃-C₄-cycloalkyl-C₁-C₂-alkyl, C₃-C₄-halocycloalkyl-C₁-C₂-alkyl, C(O)-C₁-C₄-alkyl, C(O)-C₁-C₄-haloalkyl, C(O)-C₃-C₄-cycloalkyl, C(O)-C₃-C₄-halo¬cyclo¬alkyl, or phenyl which is unsubstituted or partially or fully substituted with R³;
R³ is halogen, CN, NO₂, C₁-C₄-alkyl, C₃-C₆-cycloalkyl, C₁-C₆-haloalkyl, C₁-C₆-halocyclo-alkyl, OR¹⁴, S(O)ₘ-R¹⁴; wherein rings are unsubstituted or substituted with R^{3a};
R^{3a} halogen, CN, NO₂, C₁-C₄-alkyl, C₁-C₄-haloalkyl, C₁-C₄-alkoxy-C₁-C₄-alkyl, C₁-C₄-haloalkoxy, C₃-C₄-cycloalkyl, C₃-C₄-halocycloalkyl, S(O)ₘ-C₁-C₄-alkyl, S(O)ₘ-C₁-C₄-haloalkyl, S(O)ₘ-C₃-C₄-cycloalkyl, S(O)ₘ-C₃-C₄-halocycloalkyl;
Q is CH, or CR³;
R⁴ is H, OH, CN, C₁-C₄-alkyl, C₃-C₆-cycloalkyl, C₁-C₆-haloalkyl, C₁-C₆-halocycloalkyl, C₂-C₄-alkenyl, C₂-C₄-halo¬alkenyl, C₂-C₄-alkynyl, C₁-C₄-alkoxy, C₁-C₄-haloalkoxy, S(O)ₘ-C₁-C₄-alkyl, S(O)ₘ-C₁-C₄-haloalkyl, S(O)ₘ-C₃-C₄-cyclo¬alkyl, S(O)ₘ-C₃-C₄-halocyclo¬alkyl, NR¹²R¹³, C(O)NR¹²R¹³, C(O)OR¹⁴, 3- to 6-membered heterocyclyl, 5- or 6-membered hetaryl, or phenyl, which rings are unsubstituted or substituted with R³;
R⁵ is H, OR¹⁵, NR¹²R¹³, or C₁-C₆-alkyl which is unsubstituted, or partially or fully substituted with R¹¹;
R¹⁵ is H, C₁-C₄-alkyl, or C₁-C₄-haloalkyl, C₃-C₆-cycloalkyl, C₁-C₆-halocycloalkyl, which carbon chains are unsubstituted or partially or fully substituted with R¹¹; or 3- to 6-membered heterocyclyl, 5- or 6-membered hetaryl, or phenyl, which rings are unsubstituted or substituted with R³.

3. Compounds of formula I according to claim 1 or 2, wherein R¹ is H and R² is CH₃.

4. Compounds of formula I according to any of claims 1 to 3, wherein R³ is halogen, CN, C₁-C₄-haloalkyl, C₁-C₄-haloalkoxy, C₃-C₄-cycloalkyl unsubstituted or substituted with one or more CN, C₃-C₄-halocycloalkyl, S(O)ₘ-C₁-C₄-alkyl, S(O)ₘ-C₁-C₄-haloalkyl, S(O)ₘ-C₃-C₄-cycloalkyl, S(O)ₘ-C₃-C₄-halocycloalkyl, or
S(O)ₘ-R¹⁴, wherein R¹⁴ is phenyl, which is partially substituted with R^{3a}.

5. Compounds of formula I according to any of claim 1 to 4, wherein n is 2 and R³ is in positions 3 and 5.

6. Compounds of formula I according to any one of claims 1 to 5, which correspond to formula I.1

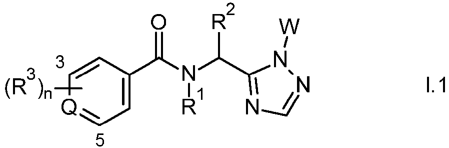

7. Compounds of formula I according to any one of claims 1 to 5, wherein R³ is selected from halogen, CN, C₁-C₄-haloalkyl, C₁-C₄-haloalkoxy, C₃-C₄-cycloalkyl, C₃-C₄-halocycloalkyl, S(O)ₘ-C₁-C₄-alkyl, S(O)ₘ-C₁-C₄-haloalkyl, S(O)ₘ-C₃-C₄-cycloalkyl, S(O)ₘ-C₃-C₄-halocyclo-alkyl, or S(O)ₘ-R¹⁴, wherein R¹⁴ is phenyl, which is partially substituted with R^{3a}.

8. Compounds of formula I according to any one of claims 1 to 7, wherein X is NOH, NOCH₃, NOCH₂CF₃, or NOCH₂-C₆H₅.

9. Compounds of formula I according to any one of the preceding claims, which consist mainly of the isomer I.A.

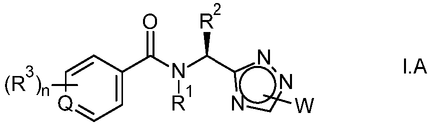

10. An agricultural or veterinary composition comprising at least one compound according to any one of claims 1 to 9 and/or at least one agriculturally or veterinarily acceptable salt thereof, and at least one inert liquid and/or solid agriculturally or veterinarily acceptable carrier.

11. An agricultural composition for use in combating animal pests comprising at least one compound as defined in any of claims 1 to 9 and at least one inert liquid and/or solid acceptable carrier and, if desired, at least one surfactant.

12. A compound as defined in any one of claims 1 to 9 for use in method for combating or controlling invertebrate pests.

13. A method for protecting growing plants from attack or infestation by invertebrate pests, which method comprises contacting a plant, or soil or water in which the plant is growing, with a pesticidally effective amount of at least one compound as defined in any of claims 1 to 9.

14. Seed comprising a compound as defined in any of claims 1 to 9, or the enantiomers, diastereomers or salts thereof, in an amount of from 0.1 g to 10 kg per 100 kg of seed.

15. A compound of the formula I as defined in any of claims 1 to 9, a stereoisomer thereof and/or a veterinarily acceptable salt thereof for use in a method for treating or protecting an animal from infestation or infection by invertebrate pests.

## Patentansprüche

1. Verbindungen der Formel I 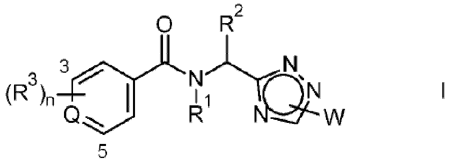 wobei
R¹ für H, OH, C₁-C₆-Alkyl, C₁-C₆-Halogenalkyl, C₃-C₆-Cycloalkyl, C₃-C₆-Halogencycloalkyl, C₁-C₅-Alkoxy, C₁-C₄-Alkyl-C₃-C₆-cycloalkyl, C₁-C₄-Alkyl-C₃-C₆-Halogengencycloalkyl, wobei diese Gruppen unsubstituiert oder teilweise oder vollständig durch R¹¹ substituiert sind;
oder C(=N-R¹¹)R¹², C(O)R^{11a} steht;
R¹¹ für CN, NO₂, NR¹²R¹³, C(O)NH₂, C(S)NH₂, C(O)OH, OR¹⁴, OR¹⁵, Si (CH₃)₃; C₁-C₆-Halogenalkyl; C₂-C₆-Alkenyl; C₂-C₆-Halogenalkenyl; C₂-C₆-Alkinyl; C₂-C₆-Halogenalkinyl; C₃-C₄-Cycloalkyl-C₁-C₂-alkyl, wobei der Ring unsubstituiert oder durch 1 oder 2 Halogen substituiert ist; 3- bis 6-gliedriges Heterocyclyl, 5- oder 6-gliedriges Hetaryl oder Phenyl, wobei die Ringe unsubstituiert oder durch Halogen, C₁-C₃-Halogenalkyl und/oder CN substituiert sind;
R^{11a} für NR¹²R¹³, C(O)NH₂, C(S)NH₂, C(O)OH, OR¹⁴, OR¹⁵, Si (CH₃)₃; C₁-C₆-Halogenalkyl; C₂-C₆-Alkenyl; C₂-C₆-Halogenalkenyl; C₂-C₆-Alkinyl; C₂-C₆-Halogenalkinyl; C₃-C₄-Cycloalkyl-C₁-C₂-alkyl, wobei der Ring unsubstituiert oder durch 1 oder 2 Halogen substituiert ist; oder 3- bis 6-gliedriges Heterocyclyl, wobei die Ringe unsubstituiert oder durch Halogen, C₁-C₃-Halogenalkyl und/oder CN substituiert sind; steht;
R¹², R¹³ unabhängig voneinander für H, C₁-C₄-Alkyl, C₁-C₄-Halogenalkyl, C₃-C₆-Cycloalkyl, C(O)-C₁-C₄-Alkyl, C(O)-C₁-C₄-Halogenalkyl, C (O) -C₃-C₄-Cycloalkyl, C(O)-C₃-C₄-Halogencycloalkyl, S(O)ₘ-C₁-C₄-Alkyl, S (O) ₘ-C₁-C₄-Halogenalkyl, S (O)ₘ-C₃-C₄-Cycloalkyl, S (O) ₘ-C₃-C₄-Halogencycloalkyl stehen oder
R¹² und R¹³ zusammen mit dem Stickstoffatom, an das sie gebunden sind, einen 3-, 4-, 5-, 6- oder 7-gliedrigen gesättigten, teilweise ungesättigten oder vollständig ungesättigten Heterocyclus bilden, wobei der Heterocyclus zusätzlich 1 oder 2 Heteroatome bzw. Heteroatomgruppen, die aus N, O und S (O) ₘ ausgewählt sind, als Ringglieder enthalten kann, wobei der Heterocyclus unsubstituiert oder durch einen oder mehrere Substituenten, die aus Halogen, C₁-C₄-Alkyl, C₁-C₄-Halogenalkyl, C₁-C₄-Alkoxy, C₁-C₄-Halogenalkoxy und Oxo ausgewählt sind, substituiert ist;
m für 0, 1 oder 2 steht;
R¹⁴ für H, C₁-C₄-Alkyl, C₁-C₄-Halogenalkyl, C₃-C₆-Cycloalkyl, C₃-C₆-Halogencycloalkyl, C₃-C₄-Cycloalkyl-C₁-C₂-alkyl, C₃-C₄-Halogencycloalkyl-C₁-C₂-alkyl, C(O)-C₁-C₄-Alkyl, C (O) -C₁-C₄-Halogenalkyl, C (O) -C₃-C₄-Cycloalkyl, C (O) -C₃-C₄-Halogencycloalkyl, NR¹²R¹³ oder 5- oder 6-gliedriges Hetaryl oder Phenyl, wobei die Ringe unsubstituiert oder teilweise oder vollständig durch R^{3a} substituiert sind, steht;
R² für H, CN, C₁-C₃-Alkyl, C₁-C₃-Halogenalkyl, C₂-C₃-Alkinyl steht;
Q für CH, CR³ oder N steht;
R³ für Halogen, CN, NO₂, C₁-C₄-Alkyl, C₃-C₆-Cycloalkyl, C₁-C₆-Halogenalkyl, C₁-C₆-Halogencycloalkyl, C₁-C₄-Alkoxy-C₁-C₄-alkyl, NR¹²R¹³, OR¹⁴, S(O)ₘ-R¹⁴, Phenyl oder einen 3-, 4-, 5- oder 6-gliedrigen gesättigten oder teilweise oder vollständig ungesättigten Heterocyclus, wobei der Heterocyclus 1, 2, 3 oder 4 Heteroatome, die aus N, O und S(O)ₘ, ausgewählt sind, als Ringglieder enthält, steht; wobei R³-Ringe direkt oder über einen C₁-C₂-Alkylen-, O- oder S(O)ₘ-Spacer gebunden sind und unsubstituiert oder durch R^{3a} substituiert sind;
R^{3a} für Halogen, CN, NO₂, C₁-C₄-Alkyl, C₁-C₄-Halogenalkyl, C₁-C₄-Alkoxy-C₁-C₄-alkyl, C₁-C₄-Halogenalkoxy, C₃-C₄-Cycloalkyl, C₃-C₄-Halogencycloalkyl, S (O) ₘ-C₁-C₄-Alkyl, S(O)ₘ-C₁-C₄-Halogenalkyl, S (O) ₘ-C₃-C₄-Cycloalkyl, S(O)ₘ-C₃-C₄-Halogencycloalkyl steht; zwei R^{3a} an demselben Kohlenstoffatom zusammen eine Oxogruppe bilden können; zwei R³ an demselben Kohlenstoffatom zusammen eine Gruppe Oxo bilden können;
zwei R³ an zwei benachbarten Kohlenstoffatomen bilden zusammen mit den Kohlenstoffatomen, an die sie gebunden sind, einen 5-, 6- oder 7-gliedrigen gesättigten, teilweise ungesättigten oder vollständig ungesättigten Ring bilden, wobei der Ring 1, 2 oder 3 Heteroatome bzw. Heteroatomgruppen, die aus N, O und S (O)ₘ ausgewählt sind, als Ringglieder enthalten kann und wobei der Ring gegebenenfalls durch eine oder mehrere Gruppen Halogen, CN, C₁-C₄-Alkyl, C₁-C₄-Halogenalkyl, C₁-C₄-Alkoxy und/oder C₁-C₄-Halogenalkoxy substituiert ist;
n für 0, 1, 2 oder 3 steht;
W für C(=X)R⁴ steht und an ein Stickstoffatom des Triazolrings gebunden ist;
Y für O oder NR⁵ steht;
R⁴ für H, OR¹⁴, CN, C₁-C₄-Alkyl, C₃-C₆-Cycloalkyl, C₁-C₆-Halogenalkyl, C₁-C₆-Halogencycloalkyl, C₂-C₄-Alkenyl, C₂-C₄-Halogenalkenyl, C₂-C₄-Alkinyl, C₁-C₄-Alkoxy, C₁-C₄-Halogenalkoxy, S (O) m-C₁-C₄-Alkyl, S (O) ₘ-C₁-C₄-Halogenalkyl, S (O) ₘ-C₃-C₄-Cycloalkyl, S (O) ₘ-C₃-C₄-Halogencycloalkyl, NR¹²R¹³, C(O)NR¹²R¹³, C(O)OR¹⁴, 3- bis 6-gliedriges Heterocyclyl, 5- oder 6-gliedriges Hetaryl oder Phenyl, wobei die Ringe unsubstituiert oder durch R^{3a} substituiert sind, steht;
R⁵ für H, OR¹⁴, OR¹⁵, NR¹²R¹³ oder C₁-C₆-Alkyl, das unsubstituiert oder teilweise oder vollständig durch R¹¹ substituiert ist, steht;
R¹⁵ für H, C₁-C₄-Alkyl oder C₁-C₄-Halogenalkyl, C₃-C₆-Cycloalkyl, C₁-C₆-Halogencycloalkyl, wobei Kohlenstoffketten unsubstituiert oder teilweise oder vollständig durch R¹¹ substituiert sind; oder 3- bis 6-gliedriges Heterocyclyl, 5- oder 6-gliedriges Hetaryl oder Phenyl, wobei die Ringe unsubstituiert oder durch R^{3a} substituiert sind, steht;
und die N-Oxide, Stereoisomere und landwirtschaftlich oder veterinärmedizinisch unbedenklichen Salze davon.

2. Verbindungen der Formel I nach Anspruch 1, wobei R¹¹ für CN, NO₂, NR¹²R¹³, C(O)NH₂, C(S)NH₂, C(O)OH, OR¹⁴, Si (CH₃)₃; C₁-C₆-Halogenalkyl; C₂-C₆-Alkenyl; C₂-C₆-Halogenalkenyl; C₂-C₆-Alkinyl; C₂-C₆-Halogenalkinyl; C₃-C₄-Cycloalkyl-C₁-C₂-alkyl, wobei der Ring unsubstituiert oder durch 1 oder 2 Halogen substituiert ist; 3- bis 6-gliedriges Heterocyclyl, 5- oder 6-gliedriges Hetaryl oder Phenyl, wobei die Ringe unsubstituiert oder durch Halogen, C₁-C₃-Halogenalkyl und/oder CN substituiert sind; steht;
R^{11a} für NR¹²R¹³, C(O)NH₂, C(S)NH₂, C(O)OH, OR¹⁴, Si(CH₃)₃; C₁-C₆-Halogenalkyl; C₂-C₆-Alkenyl; C₂-C₆-Halogenalkenyl; C₂-C₆-Alkinyl; C₂-C₆-Halogenalkinyl; C₃-C₄-Cycloalkyl-C₁-C₂-alkyl, wobei der Ring unsubstituiert oder durch 1 oder 2 Halogen substituiert ist; oder 3- bis 6-gliedriges Heterocyclyl, wobei die Ringe unsubstituiert oder durch Halogen, C₁-C₃-Halogenalkyl und/oder CN substituiert sind; steht;
R¹², R¹³ unabhängig voneinander für H, C₁-C₄-Alkyl, C₁-C₄-Halogenalkyl, C₃-C₆-Cycloalkyl, C(O)-C₁-C₄-Alkyl, C(O)-C₁-C₄-Halogenalkyl, C (O) -C₃-C₄-Cycloalkyl, C (O) -C₃-C₄-Halogencycloalkyl, S (O)ₘ-C₁-C₄-Alkyl, S (O) ₘ-C₁-C₄-Halogenalkyl, S (O)ₘ-C₃-C₄-Cycloalkyl, S (O) ₘ-C₃-C₄-Halogencycloalkyl stehen;
R¹⁴ für H, C₁-C₄-Alkyl, C₁-C₄-Halogenalkyl, C₃-C₆-Cycloalkyl, C₃-C₆-Halogencycloalkyl, C₃-C₄-Cycloalkyl-C₁-C₂-alkyl, C₃-C₄-Halogencycloalkyl-C₁-C₂-alkyl, C(O)-C₁-C₄-Alkyl, C (O) -C₁-C₄-Halogenalkyl, C (O) -C₃-C₄-Cycloalkyl, C(O)-C₃-C₄-Halogencycloalkyl, oder Phenyl, das unsubstituiert oder teilweise oder vollständig durch R³ substituiert ist, steht;
R³ für Halogen, CN, NO₂, C₁-C₄-Alkyl, C₃-C₆-Cycloalkyl, C₁-C₆-Halogenalkyl, C₁-C₆-Halogencycloalkyl, OR¹⁴, S (O)ₘ-R¹⁴ steht; wobei Ringe unsubstituiert oder durch R^{3a} substituiert sind;
R^{3a} für Halogen, CN, NO₂, C₁-C₄-Alkyl, C₁-C₄-Halogenalkyl, C₁-C₄-Alkoxy-C₁-C₄-alkyl, C₁-C₄-Halogenalkoxy, C₃-C₄-Cycloalkyl, C₃-C₄-Halogencycloalkyl, S (O)ₘ-C₁-C₄-Alkyl, S (O) ₘ-C₁-C₄-Halogenalkyl, S (O)ₘ-C₃-C₄-Cycloalkyl, S(O)ₘ-C₃-C₄-Halogencycloalkyl steht;
Q für CH oder CR³ steht;
R⁴ für H, OH, CN, C₁-C₄-Alkyl, C₃-C₆-Cycloalkyl, C₁-C₆-Halogenalkyl, C₁-C₆-Halogencycloalkyl, C₂-C₄-Alkenyl, C₂-C₄-Halogenalkenyl, C₂-C₄-Alkinyl, C₁-C₄-Alkoxy, C₁-C₄-Halogenalkoxy, S (O) m-C₁-C₄-Alkyl, S (O) ₘ-C₁-C₄-Halogenalkyl, S (O) ₘ-C₃-C₄-Cycloalkyl, S (O) ₘ-C₃-C₄-Halogencycloalkyl, NR¹²R¹³, C(O)NR¹²R¹³, C(O)OR¹⁴, 3- bis 6-gliedriges Heterocyclyl, 5- oder 6-gliedriges Hetaryl oder Phenyl, wobei die Ringe unsubstituiert oder durch R³ substituiert sind, steht;
R⁵ für H, OR¹⁵, NR¹²R¹³ oder C₁-C₆-Alkyl, das unsubstituiert oder teilweise oder vollständig durch R¹¹ substituiert ist, steht;
R¹⁵ für H, C₁-C₄-Alkyl oder C₁-C₄-Halogenalkyl, C₃-C₆-Cycloalkyl, C₁-C₆-Halogencycloalkyl, wobei Kohlenstoffketten unsubstituiert oder teilweise oder vollständig durch R¹¹ substituiert sind; oder 3- bis 6-gliedriges Heterocyclyl, 5- oder 6-gliedriges Hetaryl oder Phenyl, wobei die Ringe unsubstituiert oder durch R³ substituiert sind, steht.

3. Verbindungen der Formel I nach Anspruch 1 oder 2, wobei R¹ für H steht und R² für CH₃ steht.

4. Verbindungen der Formel I nach einem der Ansprüche 1 bis 3, wobei R³ für Halogen, CN, C₁-C₄-Halogenalkyl, C₁-C₄-Halogenalkoxy, C₃-C₄-Cycloalkyl, das unsubstituiert oder durch ein oder mehrere CN substituiert ist, C₃-C₄-Halogencycloalkyl, S(O)m-C₁-C₄-Alkyl, S(O)ₘ-C₁-C₄-Halogenalkyl, S(O)ₘ-C₃-C₄-Cycloalkyl, S(O)ₘ-C₃-C₄-Halogencycloalkyl oder
S (O)ₘ-R¹⁴ steht, wobei R¹⁴ für Phenyl, das teilweise durch R^{3a} substituiert ist, steht.

5. Verbindungen der Formel I nach einem der Ansprüche 1 bis 4, wobei n für 2 steht und R³ in den Positionen 3 und 5 steht.

6. Verbindungen der Formel I nach einem der Ansprüche 1 bis 5, die der Formel I.1 entsprechen:

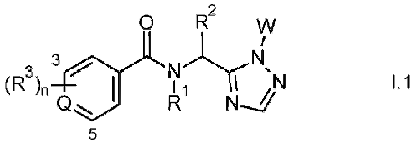

7. Verbindungen der Formel I nach einem der Ansprüche 1 bis 5, wobei R³ aus Halogen, CN, C₁-C₄-Halogenalkyl, C₁-C₄-Halogenalkoxy, C₃-C₄-Cycloalkyl, C₃-C₄-Halogencycloalkyl, S(O)ₘ-C₁-C₄-Alkyl, S(O)ₘ-C₁-C₄-Halogenalkyl, S(O)ₘ-C₃-C₄-Cycloalkyl, S(O)ₘ-C₃-C₄-Halogencycloalkyl oder S(O)ₘ-R¹⁴ steht, wobei R¹⁴ für Phenyl, das teilweise durch R^{3a} substituiert ist, steht.

8. Verbindungen der Formel I nach einem der Ansprüche 1 bis 7, wobei X für NOH, NOCH₃, NOCH₂CF₃ oder NOCH₂-C₆H₅ steht.

9. Verbindungen der Formel I nach einem der vorhergehenden Ansprüche, die hauptsächlich aus dem Isomer I.A. bestehen.

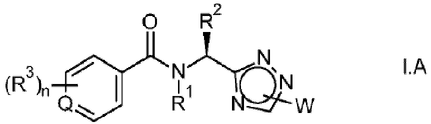

10. Landwirtschaftliche oder veterinärmedizinische Zusammensetzung, umfassend mindestens eine Verbindung nach einem der Ansprüche 1 bis 9 und/oder mindestens ein landwirtschaftlich oder veterinärmedizinisch unbedenkliches Salz davon und mindestens einen inerten flüssigen und/oder festen landwirtschaftlich oder veterinärmedizinisch unbedenklichen Träger.

11. Landwirtschaftliche Zusammensetzung zur Verwendung bei der Bekämpfung von tierischen Schädlingen, umfassend mindestens eine Verbindung gemäß einem der Ansprüche 1 bis 9 und mindestens einen inerten flüssigen und/oder festen unbedenklichen Träger und gegebenenfalls mindestens ein Tensid.

12. Verbindung nach einem der Ansprüche 1 bis 9 zur Verwendung bei einem Verfahren zum Bekämpfen oder Kontrollieren von wirbellosen Schädlingen.

13. Verfahren zum Schützen von wachsenden Pflanzen vor Angriff oder Befall durch wirbellose Schädlinge, bei dem man eine Pflanze oder den Boden oder das Wasser, in dem die Pflanze wächst, mit einer pestizid wirksamen Menge mindestens einer Verbindung gemäß einem der Ansprüche 1 bis 9 in Berührung bringt.

14. Saatgut, umfassend eine Verbindung gemäß einem der Ansprüche 1 bis 9 oder die Enantiomere, Diastereomere oder Salze davon in einer Menge von 0,1 g bis 10 kg pro 100 kg Saatgut.

15. Verbindung der Formel I gemäß einem der Ansprüche 1 bis 9, ein Stereoisomer davon und/oder ein veterinärmedizinisch unbedenkliches Salz davon zur Verwendung bei einem Verfahren zum Behandeln oder Schützen eines Tiers vor Befall oder Infektion durch wirbellose Schädlinge.

## Revendications

1. Composés de formule I 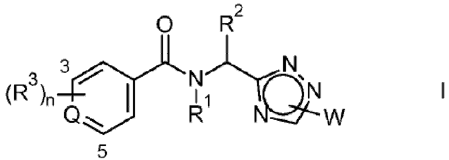 dans lesquels
R¹ représente H, un groupe OH, alkyle en C₁-C₆, halogénoalkyle en C₁-C₆, cycloalkyle en C₃-C₆, halogénocycloalkyle en C₃-C₆, alcoxy en C₁-C₅, (alkyle en C₁-C₄) - (cycloalkyle en C₃-C₆), (alkyle en C₁-C₄)-(halogénocycloalkyle en C₃-C₆), lesdits groupes étant non substitués ou partiellement ou complètement substitués par R¹¹ ;
ou C(=N-R¹¹)R¹², C(O)R^{11a} ;
R¹¹ représente un groupe CN, NO₂, NR¹²R¹³, C(O)NH₂, C(S)NH₂, C(O)OH, OR¹⁴, OR¹⁵, Si (CH₃)₃ ; halogénoalkyle en C₁-C₆ ; alcényle en C₂-C₆ ; halogénoalcényle en C₂-C₆ ; alcynyle en C₂-C₆ ; halogénoalcynyle en C₂-C₆ ; (cycloalkyle en C₃-C₄)-(alkyle en C₁-C₂), ledit cycle étant non substitué ou substitué par 1 ou 2 atomes d'halogène ; hétérocyclyle de 3 à 6 chaînons, hétéroaryle de 5 ou 6 chaînons, ou phényle, lesdits cycles étant non substitués ou substitués par un atome d'halogène, un groupe halogénoalkyle en C₁-C₃ et/ou CN ;
R^{11a} représente un groupe NR¹²R¹³, C(O)NH₂, C(S)NH₂, C(O)OH, OR¹⁴, OR¹⁵, Si (CH₃)₃ ; halogénoalkyle en C₁-C₆ ; alcényle en C₂-C₆ ; halogénoalcényle en C₂-C₆ ; alcynyle en C₂-C₆ ; halogénoalcynyle en C₂-C₆ ; (cycloalkyle en C₃-C₄)-(alkyle en C₁-C₂), ledit cycle étant non substitué ou substitué par 1 ou 2 atomes d'halogène ; hétérocyclyle de 3 à 6 chaînons, lesdits cycles étant non substitués ou substitués par un atome d'halogène, un groupe halogénoalkyle en C₁-C₃ et/ou CN ;
R¹², R¹³ représentent, indépendamment l'un de l'autre, H, un groupe alkyle en C₁-C₄, halogénoalkyle en C₁-C₄, cycloalkyle en C₃-C₆, C(O)-(alkyle en C₁-C₄), C(O)-(halogénoalkyle en C₁-C₄), C(O)-(cycloalkyle en C₃-C₄), C(O)-(halogénocycloalkyle en C₃-C₄), S(O)ₘ-(alkyle en C₁-C₄), S(O)ₘ-(halogénoalkyle en C₁-C₄), S(O)ₘ-(cycloalkyle en C₃-C₄), S(O)ₘ-(halogénocycloalkyle en C₃-C₄) ; ou
R¹² et R¹³, avec l'atome d'azote auquel ils sont liés, forment un hétérocycle de 3, 4, 5, 6 ou 7 chaînons saturé, partiellement insaturé ou complètement insaturé, ledit hétérocycle pouvant contenir en outre 1 ou 2 hétéroatomes ou groupes hétéroatomiques choisis parmi N, O et S(O)ₘ en tant que chaînons de cycle, ledit hétérocycle étant non substitué ou substitué par un ou plusieurs substituants choisis parmi un atome d'halogène, un groupe alkyle en C₁-C₄, halogénoalkyle en C₁-C₄, alcoxy en C₁-C₄, halogénoalcoxy en C₁-C₄ et oxo ;
m est 0, 1 ou 2 ;
R¹⁴ représente H, un groupe alkyle en C₁-C₄, halogénoalkyle en C₁-C₄, cycloalkyle en C₃-C₆, halogénocycloalkyle en C₃-C₆, (cycloalkyle en C₃-C₄) -(alkyle en C₁-C₂), (halogénocycloalkyle en C₃-C₄)-(alkyle en C₁-C₂), C(O)-(alkyle en C₁-C₄), C(O)-(halogénoalkyle en C₁-C₄), C(O)-(cycloalkyle en C₃-C₄), C(O)-(halogénocycloalkyle en C₃-C₄), NR¹²R¹³, ou hétéroaryle de 5 ou 6 chaînons ou phényle, lesdits cycles étant non substitués ou partiellement ou complètement substitués par R^{3a} ;
R² représente H, un groupe CN, alkyle en C₁-C₃, halogénoalkyle en C₁-C₃, alcynyle en C₂-C₃ ;
Q représente un groupe CH, CR³ ou N ;
R³ représente un atome d'halogène, un groupe CN, NO₂, alkyle en C₁-C₄, cycloalkyle en C₃-C₆, halogénoalkyle en C₁-C₆, halogénocycloalkyle en C₁-C₆, (alcoxy en C₁-C₄)-(alkyle en C₁-C₄), NR¹²R¹³, OR¹⁴, S(O)ₘ-R¹⁴, phényle, ou un hétérocycle de 3, 4, 5 ou 6 chaînons saturé, partiellement insaturé ou complètement insaturé, ledit hétérocycle pouvant contenir en outre 1, 2, 3 ou 4 hétéroatomes choisis parmi N, O et S(O)ₘ en tant que chaînons de cycle ; les cycles R³ étant liés directement ou par l'intermédiaire d'un espaceur alkylène en C₁-C₂, O ou S(O)ₘ, et étant non substitués ou substitués par R^{3a} ; R^{3a} représente un atome d'halogène, un groupe CN, NO₂, alkyle en C₁-C₄, halogénoalkyle en C₁-C₄, (alcoxy en C₁-C₄)-(alkyle en C₁-C₄), halogénoalcoxy en C₁-C₄, cycloalkyle en C₃-C₄, halogénocycloalkyle en C₃-C₄, S(O)ₘ-(alkyle en C₁-C₄), S(O)ₘ-(halogénoalkyle en C₁-C₄), S(O)ₘ-(cycloalkyle en C₃-C₄), S(O)ₘ-(halogénocycloalkyle en C₃-C₄) ; deux groupes R^{3a} sur le même atome de carbone peuvent former ensemble un groupe oxo ;
deux groupes R³ sur le même atome de carbone peuvent former ensemble un groupe oxo ;
deux groupes R³ sur deux atomes de carbone adjacents formant conjointement avec les atomes de carbone auxquels ils sont liés un cycle saturé, partiellement insaturé ou complètement insaturé de 5, 6 ou 7 chaînons, le cycle pouvant contenir 1, 2 ou 3 hétéroatomes ou groupes hétéroatomiques choisis parmi N, O, et S(O)ₘ en tant que chaînons de cycle, et le cycle étant facultativement substitué par un ou plusieurs atomes d'halogène ou groupes CN, alkyle en C₁-C₄, halogénoalkyle en C₁-C₄, alcoxy en C₁-C₄ et/ou halogénoalcoxy en C₁-C₄ ;
n est 0, 1, 2 ou 3 ;
W représente un groupe C(=X)R⁴, étant lié à un atome d'azote du cycle triazole ;
X est O ou NR⁵ ;
R⁴ représente H, un groupe OR¹⁴, CN, alkyle en C₁-C₄, cycloalkyle en C₃-C₆, halogénoalkyle en C₁-C₆, halogénocycloalkyle en C₁-C₆, alcényle en C₂-C₄, halogénoalcényle en C₂-C₄, alcynyle en C₂-C₄, alcoxy en C₁-C₄, halogénoalcoxy en C₁-C₄, S(O)ₘ-(alkyle en C₁-C₄), S(O)ₘ-(halogénoalkyle en C₁-C₄), S(O)ₘ-(cycloalkyle en C₃-C₄), S(O)ₘ-(halogénocycloalkyle en C₃-C₄), NR¹²R¹³, C(O)NR¹²R¹³, C(O)OR¹⁴, hétérocyclyle de 3 à 6 chaînons, hétéroaryle de 5 ou 6 chaînons, ou phényle, lesdits cycles étant non substitués ou substitués par R^{3a} ;
R⁵ représente H, un groupe OR¹⁴, OR¹⁵, NR¹²R¹³, ou alkyle en C₁-C₆ qui est non substitué, ou partiellement ou complètement substitué par R¹¹ ;
R¹⁵ représente H, un groupe alkyle en C₁-C₄, ou halogénoalkyle en C₁-C₄, cycloalkyle en C₃-C₆, halogénocycloalkyle en C₁-C₆, lesdites chaînes de carbone étant non substituées ou partiellement ou complètement substituées par R¹¹ ; ou hétérocyclyle de 3 à 6 chaînons, hétéroaryle de 5 ou 6 chaînons, ou phényle, lesdits cycles étant non substitués ou substitués par R^{3a} ;
et les N-oxydes, stéréoisomères et sels acceptables sur le plan agricole ou vétérinaire de ceux-ci.

2. Composés de formule I selon la revendication 1, dans lesquels
R¹¹ représente un groupe CN, NO₂, NR¹²R¹³, C(O)NH₂, C(S)NH₂, C(O)OH, OR¹⁴, Si (CH₃)₃ ; halogénoalkyle en C₁-C₆ ; alcényle en C₂-C₆ ; halogénoalcényle en C₂-C₆ ; alcynyle en C₂-C₆ ; halogénoalcynyle en C₂-C₆ ; (cycloalkyle en C₃-C₄)-(alkyle en C₁-C₂), ledit cycle étant non substitué ou substitué par 1 ou 2 atomes d'halogène ; hétérocyclyle de 3 à 6 chaînons, hétéroaryle de 5 ou 6 chaînons, ou phényle, lesdits cycles étant non substitués ou substitués par un atome d'halogène, un groupe halogénoalkyle en C₁-C₃ et/ou CN ;
R^{11a} représente un groupe NR¹²R¹³, C(O)NH₂, C(S)NH₂, C(O)OH, OR¹⁴, Si (CH₃)₃ ; halogénoalkyle en C₁-C₆ ; alcényle en C₂-C₆ ; halogénoalcényle en C₂-C₆ ; alcynyle en C₂-C₆ ; halogénoalcynyle en C₂-C₆ ; (cycloalkyle en C₃-C₄)-(alkyle en C₁-C₂), ledit cycle étant non substitué ou substitué par 1 ou 2 atomes d'halogène ; un groupe hétérocyclyle de 3 à 6 chaînons, lesdits cycles étant non substitués ou substitués par un atome d'halogène, un groupe halogénoalkyle en C₁-C₃ et/ou CN ;
R¹², R¹³ représentent, indépendamment l'un de l'autre, H, un groupe alkyle en C₁-C₄, halogénoalkyle en C₁-C₄, cycloalkyle en C₃-C₆, C(O)-(alkyle en C₁-C₄), C(O)-(halogénoalkyle en C₁-C₄), C(O)-(cycloalkyle en C₃-C₄), C(O)-(halogénocycloalkyle en C₃-C₄), S(O)ₘ-(alkyle en C₁-C₄), S(O)ₘ- (halogénoalkyle en C₁-C₄), S(O)ₘ- (cycloalkyle en C₃-C₄), S(O)ₘ-(halogénocycloalkyle en C₃-C₄) ;
R¹⁴ représente H, un groupe alkyle en C₁-C₄, halogénoalkyle en C₁-C₄, cycloalkyle en C₃-C₆, halogénocycloalkyle en C₃-C₆, (cycloalkyle en C₃-C₄) - (alkyle en C₁-C₂), (halogénocycloalkyle en C₃-C₄)-(alkyle en C₁-C₂), C(O)-(alkyle en C₁-C₄), C(O)-(halogénoalkyle en C₁-C₄), C(O)-(cycloalkyle en C₃-C₄), C(O)-(halogénocycloalkyle en C₃-C₄) ou phényle qui est non substitué ou partiellement ou complètement substitué par R³ ;
R³ représente un atome d'halogène, un groupe CN, NO₂, alkyle en C₁-C₄, cycloalkyle en C₃-C₆, halogénoalkyle en C₁-C₆, halogénocycloalkyle en C₁-C₆, OR¹⁴, S(O)ₘ-R¹⁴ ; les cycles étant non substitués ou substitués par R^{3a} ;
R^{3a} représente un atome d'halogène, un groupe CN, NO₂, alkyle en C₁-C₄, halogénoalkyle en C₁-C₄, (alcoxy en C₁-C₄) - (alkyle en C₁-C₄), halogénoalcoxy en C₁-C₄, cycloalkyle en C₃-C₄, halogénocycloalkyle en C₃-C₄, S(O)ₘ-(alkyle en C₁-C₄), S(O)ₘ-(halogénoalkyle en C₁-C₄), S(O)ₘ-(cycloalkyle en C₃-C₄), S(O)ₘ-(halogénocycloalkyle en C₃-C₄) ;
Q représente un groupe CH ou CR³ ;
R⁴ représente H, un groupe OH, CN, alkyle en C₁-C₄, cycloalkyle en C₃-C₆, halogénoalkyle en C₁-C₆, halogénocycloalkyle en C₁-C₆, alcényle en C₂-C₄ ; halogénoalcényle en C₂-C₄, alcynyle en C₂-C₄, alcoxy en C₁-C₄, halogénoalcoxy en C₁-C₄, S(O)ₘ-(alkyle en C₁-C₄), S(O)ₘ-(halogénoalkyle en C₁-C₄), S(O)ₘ-(cycloalkyle en C₃-C₄), S(O)ₘ-(halogénocycloalkyle en C₃-C₄), NR¹²R¹³, C(O)NR¹²R¹³, C(O)OR¹⁴, hétérocyclyle de 3 à 6 chaînons, hétéroaryle de 5 ou 6 chaînons ou phényle, lesdits cycles étant non substitués ou substitués par R³ ;
R⁵ représente H, un groupe OR¹⁵, NR¹²R¹³ ou alkyle en C₁-C₆ qui est non substitué, ou partiellement ou complètement substitué par R¹¹ ;
R¹⁵ représente H, un groupe alkyle en C₁-C₄ ou halogénoalkyle en C₁-C₄, cycloalkyle en C₃-C₆, halogénocycloalkyle en C₁-C₆, lesdites chaînes de carbone étant non substituées ou partiellement ou complètement substituées par R¹¹ ; ou hétérocyclyle de 3 à 6 chaînons, hétéroaryle de 5 ou 6 chaînons, ou phényle, lesdits cycles étant non substitués ou substitués par R³.

3. Composés de formule I selon la revendication 1 ou 2, dans lesquels R¹ est H et R² est CH₃.

4. Composés de formule I selon l'une quelconque des revendications 1 à 3, dans lesquels R³ est un atome d'halogène, un groupe CN, halogénoalkyle en C₁-C₄, halogénoalcoxy en C₁-C₄, cycloalkyle en C₃-C₄ non substitué ou substitué par un ou plusieurs groupes CN, halogénocycloalkyle en C₃-C₄, S(O)ₘ-(alkyle en C₁-C₄), S(O)ₘ-(halogénoalkyle en C₁-C₄), S(O)ₘ-(cycloalkyle en C₃-C₄), S(O)ₘ-(halogénocycloalkyle en C₃-C₄), ou
S(O)ₘ-R¹⁴, R¹⁴ étant phényle, qui est partiellement substitué par R^{3a}.

5. Composés de formule I selon l'une quelconque des revendications 1 à 4, dans lesquels n est 2 et R³ est aux positions 3 et 5.

6. Composés de formule I selon l'une quelconque des revendications 1 à 5, qui correspondent à la formule I.1

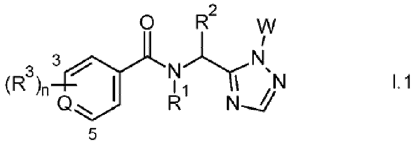

7. Composés de formule I selon l'une quelconque des revendications 1 à 5, dans lesquels R³ est choisi parmi un atome d'halogène, un groupe CN, halogénoalkyle en C₁-C₄, halogénoalcoxy en C₁-C₄, cycloalkyle en C₃-C₄, halogénocycloalkyle en C₃-C₄, S(O)ₘ-(alkyle en C₁-C₄), S(O)ₘ-(halogénoalkyle en C₁-C₄), S(O)ₘ-(cycloalkyle en C₃-C₄), S(O)ₘ- (halogénocycloalkyle en C₃-C₄) ou S(O)ₘ-R¹⁴, R¹⁴ étant phényle, qui est partiellement substitué par R^{3a}.

8. Composés de formule I selon l'une quelconque des revendications 1 à 7, dans lesquels X représente un groupe NOH, NOCH₃, NOCH₂CF₃ ou NOCH₂-C₆H₅.

9. Composés de formule I selon l'une quelconque des revendications précédentes, qui sont principalement constitués de l'isomère I.A.

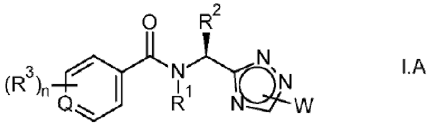

10. Composition agricole ou vétérinaire comprenant au moins un composé selon l'une quelconque des revendications 1 à 9 et/ou au moins un sel acceptable sur le plan agricole ou vétérinaire de celui-ci, et au moins un support liquide et/ou solide inerte acceptable sur le plan agricole ou vétérinaire.

11. Composition agricole destinée à être utilisée dans la lutte contre des organismes nuisibles animaux, comprenant au moins un composé tel que défini dans l'une quelconque des revendications 1 à 9 et au moins un support liquide et/ou solide inerte acceptable et, le cas échéant, au moins un tensioactif.

12. Composé tel que défini dans l'une quelconque des revendications 1 à 9 destiné à être utilisé dans un procédé de lutte contre des organismes nuisibles invertébrés.

13. Procédé de protection de végétaux en croissance contre une attaque ou une infestation par des organismes nuisibles invertébrés, le procédé comprenant la mise en contact d'un végétal, ou du sol ou de l'eau dans lesquels le végétal croît, avec une quantité efficace sur le plan pesticide d'au moins un composé tel que défini dans l'une quelconque des revendications 1 à 9.

14. Graine comprenant un composé tel que défini dans l'une quelconque des revendications 1 à 9, ou les énantiomères, diastéréoisomères ou sels de celui-ci, en une quantité de 0,1 g à 10 kg pour 100 kg de graines.

15. Composé de la formule I selon l'une quelconque des revendications 1 à 9, un stéréoisomère de celui-ci et/ou au moins un sel acceptable sur le plan vétérinaire de celui-ci destiné à être utilisé dans un procédé de traitement ou de protection d'un animal contre une infestation ou une infection par des organismes nuisibles invertébrés.
